# EUROPEAN PATENT APPLICATION

(11) **EP 3 753 575 A1**
(43) Date of publication of application: **23.12.2020**
(21) Application number: 20174278.0
(22) Date of filing: 16.07.2015
(51) Int. Cl.: A61K 39/395

(54) **METHODS FOR TREATING HIGH CARDIOVASCULAR RISK PATIENTS WITH HYPERCHOLESTEROLEMIA USING INHIBITORS OF PCSK9**

(30) Priority: 16.07.2014 US 201462025371 P; 28.08.2014 US 201462043167 P; 17.11.2014 US 201462080725 P; 13.03.2015 US 201562132709 P; 29.05.2015 EP 15305830
(62) Divisional of application: 15760317.6
(71) Applicant: Sanofi Biotechnology, 75008 Paris (FR); Regeneron Pharmaceuticals, Inc., Tarrytown, NY 10591-6707 (US)
(72) Inventor: HANOTIN, Corinne, 75008 Paris (FR); BESSAC, Laurence, 75008 Paris (FR); CHAUDHARI, Umesh, Bridgewater, NJ 08807 (US); PORDY, Robert C., Tarrytown, NY 10591 (US); SCHWEMMER GIPE, Daniel A., Tarrytown, NY 10591 (US)
(74) Representative: Zwicker, Jörk

(57) **Abstract**

The present invention provides methods for treating hypercholesterolemia. The methods of the present invention comprise administering to a high cardiovascular risk patient a pharmaceutical composition comprising a PCSK9 inhibitor. In certain embodiments, the PCSK9 inhibitor is an anti-PCSK9 antibody such as the exemplary antibody referred to herein as mAb316P. The methods of the present invention are useful for treating high cardiovascular risk patients with hypercholesterolemia and established CHD or CHD risk equivalents that are not adequately controlled by maximum tolerated dose statin therapy.

## Description

### FIELD OF THE INVENTION

The present invention relates to the field of therapeutic treatments of diseases and disorders that are associated with elevated levels of lipids and lipoproteins. More specifically, the invention relates to the use of PCSK9 inhibitors to treat high cardiovascular risk patients with hypercholesterolemia and established coronary heart disease (CHD) or CHD risk equivalents that are not adequately controlled by maximum tolerated dose statin therapy.

### BACKGROUND

Hypercholesterolemia, particularly an increase in low-density lipoprotein (LDL) cholesterol (LDL-C) levels, constitutes a major risk for the development of atherosclerosis and coronary heart disease (CHD) (Sharrett et al., 2001, Circulation 104:1108-1113). Low-density lipoprotein cholesterol is identified as the primary target of cholesterol lowering therapy and is accepted as a valid surrogate therapeutic endpoint. Numerous studies have demonstrated that reducing LDL-C levels reduces the risk of CHD with a strong direct relationship between LDL-C levels and CHD events; for each 1 mmol/L (-40 mg/dL) reduction in LDL-C, cardiovascular disease (CVD) mortality and morbidity is lowered by 22%. Greater reductions in LDL-C produce greater reduction in events, and comparative data of intensive versus standard statin treatment suggest that the lower the LDL-C level, the greater the benefit in patients at very high cardiovascular (CV) risk.

Current LDL-C lowering medications include statins, cholesterol absorption inhibitors (e.g., ezetimibe [EZE]), fibrates, niacin, and bile acid sequestrants. Statins are the most commonly prescribed, as they have shown a greater ability to lower LDL-C and reduce CHD events. However, many patients at risk of cardiovascular disease (CVD) have poorly controlled low-density lipoprotein cholesterol (LDL-C) despite statin therapy.

### BRIEF SUMMARY OF THE INVENTION

The present invention provides methods for treating hypercholesterolemia. In particular, the methods of the present invention are useful for treating high cardiovascular risk patients with hypercholesterolemia and established coronary heart disease (CHD) or CHD risk equivalents that are not adequately controlled by maximum tolerated dose statin therapy.

According to one aspect, the methods of the present invention comprise administering one or more doses of a PCSK9 inhibitor to a high cardiovascular risk patient with hypercholesterolemia and established coronary heart disease or CHD risk equivalents that are not adequately controlled by maximum tolerated dose statin therapy (*i.e.*, hypercholesterolemia that is not adequately controlled by maximum tolerated dose statin therapy in the absence of a PCSK9 inhibitor, with or without other lipid modifying therapy). According to certain embodiments of the present invention, the PCSK9 inhibitor is administered to the high cardiovascular risk patient as an add-on therapy to the patient's existing statin therapy.

According to another aspect, the methods of the present invention comprise selecting a high cardiovascular risk patient who is on a therapeutic regimen comprising a daily dose of a statin (*e.g.*, a maximum tolerated dose statin therapy), and administering to the patient one or more doses of a PCSK9 inhibitor in combination with (*i.e.*, "on top of") the statin therapy.

An aspect of the invention includes a method for treating a high cardiovascular risk patient with hypercholesterolemia and established coronary heart disease (CHD) or CHD risk equivalents that are not adequately controlled by maximum tolerated dose statin therapy with or without other lipid lowering therapy comprising administering one or more doses of a proprotein convertase subtilisin/kexin type 9 (PCSK9) inhibitor to the patient, wherein the patient exhibits inadequate control of the hypercholesterolemia despite treatment with the maximum tolerated dose statin therapy with or without other lipid lowering therapy in the absence of the PCSK9 inhibitor.

Another aspect of the invention includes a method for reducing low-density lipoprotein cholesterol (LDL-C) in a high cardiovascular risk patient with hypercholesterolemia and established coronary heart disease (CHD) or CHD risk equivalents that is not adequately controlled by maximum tolerated dose statin therapy with or without other lipid lowering therapy comprising administering one or more doses of a proprotein convertase subtilisin/kexin type 9 (PCSK9) inhibitor to the patient, wherein the patient exhibits inadequate control of LDL-C despite treatment with a maximum tolerated dose statin therapy with or without other lipid lowering therapy in the absence of the PCSK9 inhibitor.

Another aspect of the invention includes a method for treating hypercholesterolemia in a high cardiovascular risk patient with hypercholesterolemia and established coronary heart disease (CHD) or CHD risk equivalents that are not adequately controlled by maximum tolerated dose statin therapy with or without other lipid lowering therapy comprising administering one or more doses of a proprotein convertase subtilisin/kexin type 9 (PCSK9) inhibitor to the patient, wherein the patient exhibits inadequate control of the hypercholesterolemia despite treatment with the maximum tolerated dose statin therapy with or without other lipid lowering therapy in the absence of the PCSK9 inhibitor.

Another aspect of the invention includes a method for improving the serum level of one or more lipid components in a high cardiovascular risk patient with hypercholesterolemia and established coronary heart disease (CHD) or CHD risk equivalents that are not adequately controlled by maximum tolerated dose statin therapy with or without other lipid lowering therapy comprising administering one or more doses of a proprotein convertase subtilisin/kexin type 9 (PCSK9) inhibitor to the patient, wherein the patient exhibits inadequate control of the lipid component despite treatment with the maximum tolerated dose statin therapy with or without other lipid lowering therapy in the absence of the PCSK9 inhibitor. In certain aspects, the invention provides a decrease in the serum level of a lipid component selected from the group consisting of LDL-C, Apo B, non-HDL-C, total cholesterol, Lp(a), and triglycerides. In certain aspects, the invention provides an increase in the serum level of a lipid component selected from the group consisting of HDL-C and ApoA-1.

In certain aspects, established CHD is defined by a history of CHD, including one or more of the following: acute myocardial infarction (MI), silent MI, unstable angina, coronary revascularization procedure, and clinically significant CHD diagnosed by invasive or non-invasive testing.

In certain aspects, the CHD risk equivalents include one or more of the following four criteria: 1) peripheral arterial disease (PAD); 2) ischemic stroke; 3) chronic kidney disease (CKD); and/or 4) known history of diabetes mellitus and 2 or more additional risk factors, including: a) history of hypertension, b) history of ankle-brachial index ≤0.90, c) history of microalbuminuria or macroalbuminuria or dipstick urinalysis at screening visit with >2+ protein, d) history of preproliferative or proliferative retinopathy or laser treatment for retinopathy, and e) known family history of premature CHD.

In certain aspects, the PCSK9 inhibitor is an antibody or an antigen-binding fragment thereof that specifically binds PCSK9. In some aspects, the antibody or antigen-binding fragment thereof comprises the heavy and light chain complementarity determining regions (CDRs) of a heavy chain variable region/light chain variable region (HCVR/LCVR) amino acid sequence pair selected from the group consisting of SEQ ID NOs: 1/6 and 11/15. In some aspects, the antibody or antigen-binding fragment thereof comprises heavy and light chain CDR amino acid sequences having SEQ ID NOs: 12, 13, 14, 16, 17, and 18. In some aspects, the antibody or antigen-binding fragment thereof comprises an HCVR having the amino acid sequence of SEQ ID NO:11 and an LCVR having the amino acid sequence of SEQ ID NO:15. In some aspects, the antibody or antigen-binding fragment thereof comprises heavy and light chain CDR amino acid sequences having SEQ ID NOs:2, 3, 4, 7, 8, and 10. In some aspects, the antibody or antigen-binding fragment thereof comprises an HCVR having the amino acid sequence of SEQ ID NO:1 and an LCVR having the amino acid sequence of SEQ ID NO:6. In some aspects, the antibody or antigen-binding fragment thereof binds to the same epitope on PCSK9 as an antibody comprising heavy and light chain CDR amino acid sequences having SEQ ID NOs: 12, 13, 14, 16, 17, and 18; or SEQ ID NOs: 2, 3, 4, 7, 8, and 10. In some aspects, the antibody or antigen-binding fragment thereof competes for binding to PCSK9 with an antibody comprising heavy and light chain CDR amino acid sequences having SEQ ID NOs: 12, 13, 14, 16, 17, and 18; or SEQ ID NOs: 2, 3, 4, 7, 8, and 10.

In certain aspects, the antibody or antigen-binding fragment thereof that specifically binds PCSK9 is administered to the patient at a dose of about 75 mg at a frequency of once every two weeks. In some aspects, the about 75 mg dose is maintained if the patient's LDL-C measured after five or more doses is <70 mg/dL. In some aspects, the about 75 mg dose is discontinued if the patient's LDL-C measured after five or more doses remains ≥70 mg/dL, and the antibody or antigen-binding fragment thereof that specifically binds PCSK9 is subsequently administered to the patient at a dose of about 150 mg at a frequency of once every two weeks. In some aspects, the antibody or antigen-binding fragment thereof that specifically binds PCSK9 is administered to the patient at a dose of about 150 mg at a frequency of once every two weeks.

In certain aspects, the PCSK9 inhibitor is administered to the patient in combination with the maximum tolerated dose statin therapy. In some aspects, the maximum tolerated dose statin therapy comprises a daily dose of about 40 mg to about 80 mg of atorvastatin. In some aspects, the maximum tolerated dose statin therapy comprises a daily dose of about 20 mg to about 40 mg of rosuvastatin. In some aspects, the maximum tolerated dose statin therapy comprises a daily dose of about 80 mg of simvastatin. In some aspects, the PCSK9 inhibitor is administered to the patient in combination with the other lipid lowering therapy, wherein the other lipid lowering therapy comprises a therapeutic agent selected from the group consisting of ezetimibe, a fibrate, niacin, an omega-3 fatty acid, and a bile acid resin.

In certain aspects, the method improves at least one hypercholesterolemia-associated parameter selected from the group consisting of: (a) reduction of the patient's low density lipoprotein cholesterol (LDL-C) by at least 40%; (b) reduction of the patient's apolipoprotein B100 (ApoB) by at least 35%; (c) reduction of the patient's non-high density lipoproprotein cholesterol (non-HDL-C) by at least 35%; (d) reduction of the patient's total cholesterol by at least 20%; (e) increase of the patient's high density lipoprotein cholesterol (HDL-C) by at least 1%; (f) reduction of the patient's triglycerides by at least 1%; (g) reduction of the patient's lipoprotein a (Lp(a)) by at least 10%; and (h) increase of the patient's apolipoprotein A-1 by at least 1%.

Other embodiments of the present invention will become apparent from a review of the ensuing detailed description.

### BRIEF DESCRIPTION OF THE FIGURES

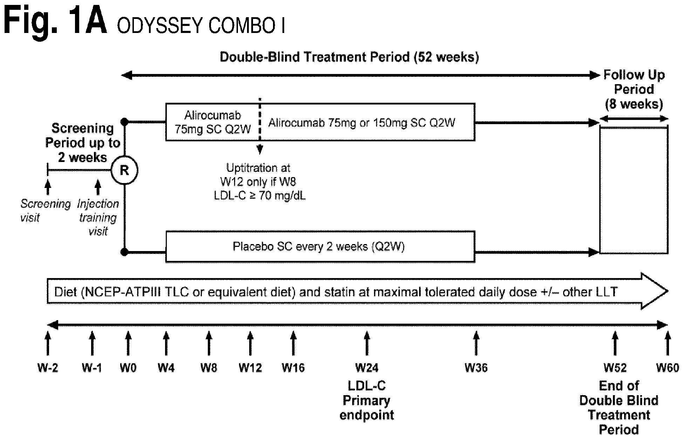

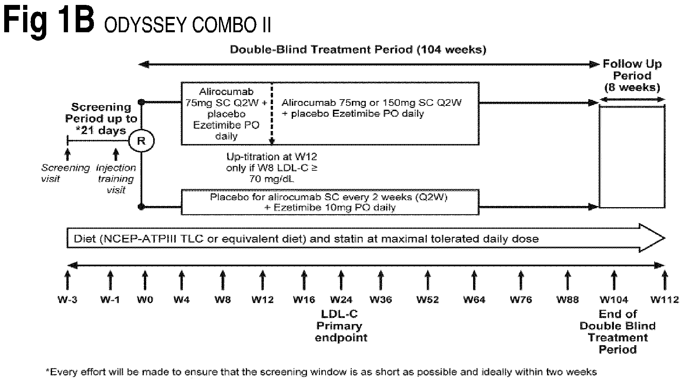

**Figure 1A** is a graphic representation of the study design for ODYSSEY COMBO I. **Figure 1B** is a graphic representation of the study design for ODYSSEY COMBO II. LDL-C, low-density lipoprotein cholesterol; LLT, lipid-lowering therapy; NCEP ATP III, National Cholesterol Education Program Adult Treatment Panel III; PO, *per os*; Q2W, every 2 weeks; R, randomization; SC, subcutaneous; TLC, therapeutic lifestyle changes.

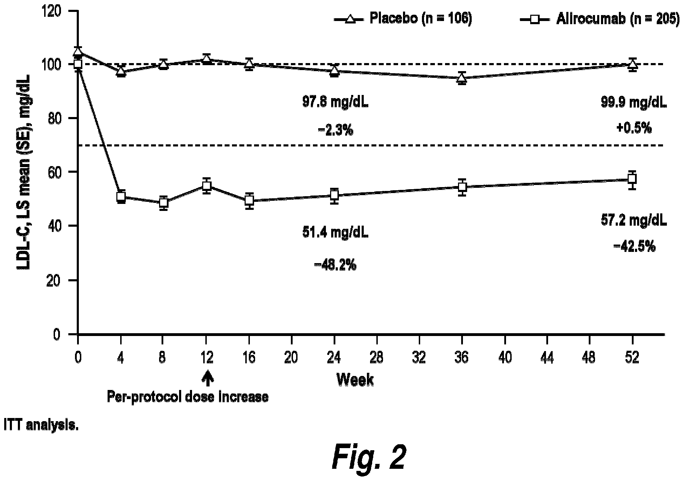

**Figure 2** is a graph that shows the calculated LDL-C LS mean (+/- SE) percent change from baseline for placebo and alirocumab over time for the ITT analysis - ITT population in the ODYSSEY COMBO I study. Note: Least-squares (LS) means and standard errors (SE) taken from MMRM (mixed-effect model with repeated measures) analysis. The model includes the fixed categorical effects of treatment group, randomization strata as per IVRS, time point, treatment-by-time point interaction, strata-by-time point interaction as well as the continuous fixed covariates of baseline LDL-C value and baseline LDL-C-by-time point interaction.

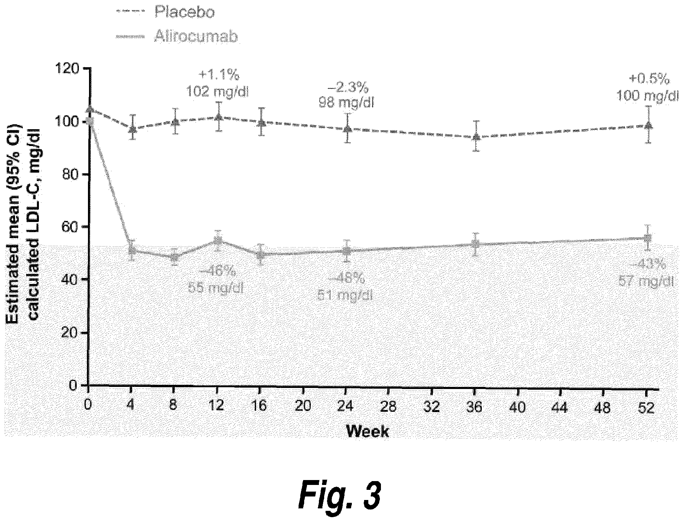

**Figure 3** is a graph that shows the calculated LDL-C LS mean (+/- SE) percent change from baseline for placebo and alirocumab over time for the on treatment analysis - mITT population in the ODYSSEY COMBO I study. The efficacy treatment period ends at last injection date + 21 days. Note: Least-squares (LS) means and standard errors (SE) taken from MMRM (mixed-effect model with repeated measures) analysis. The model includes the fixed categorical effects of treatment group, randomization strata as per IVRS, time point, treatment-by-time point interaction, strata-by-time point interaction as well as the continuous fixed covariates of baseline LDL-C value and baseline LDL-C-by-time point interaction.

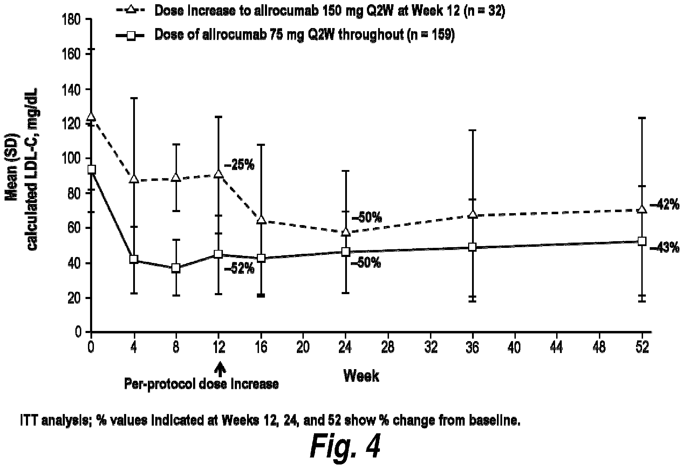

**Figure 4** is a graph of LDL-C levels over time for alirocumab-treated patients with/without dose increase at week 12 in the ODYSSEY COMBO I study.

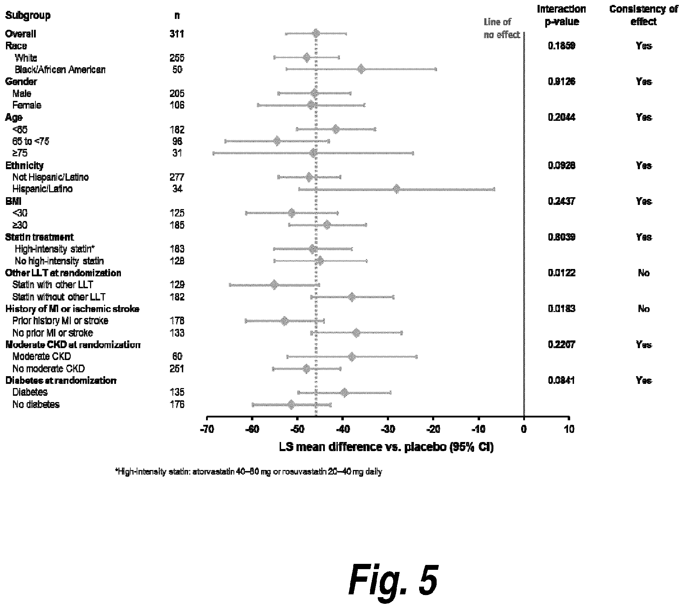

**Figure 5** is a graph of the percentage LDL-C reduction from baseline at week 24 for various alirocumab and placebo demographic subgroups in the ODYSSEY COMBO I study.

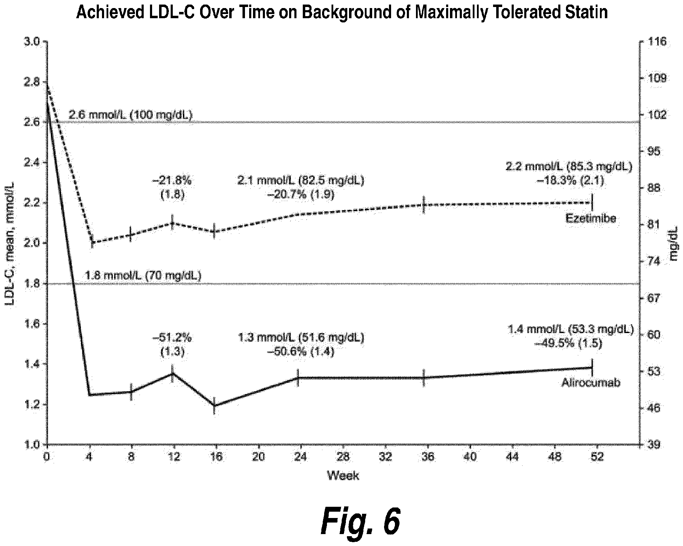

**Figure 6** is a graph that shows the calculated LDL-C LS mean (+/- SE) percent change from baseline over time for the ITT population in the ODYSSEY COMBO II study. Note: Least-squares (LS) means and standard errors (SE) taken from MMRM (mixed-effect model with repeated measures) analysis. The model includes the fixed categorical effects of treatment group, randomization strata as per IVRS, time point, treatment-by-time point interaction, strata-by-time point interaction as well as the continuous fixed covariates of baseline LDL-C value and baseline LDL-C-by-time point interaction.

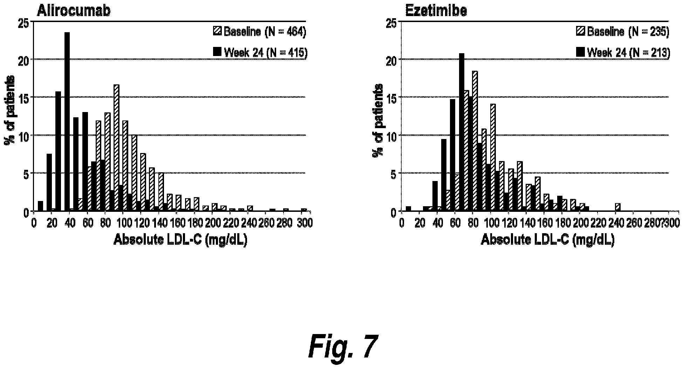

**Figure 7** is a graph of achieved LDL-C concentrations at 24 weeks by decile of baseline LDL-C in patients for the alirocumab and ezetimibe groups in the ODYSSEY COMBO II study.

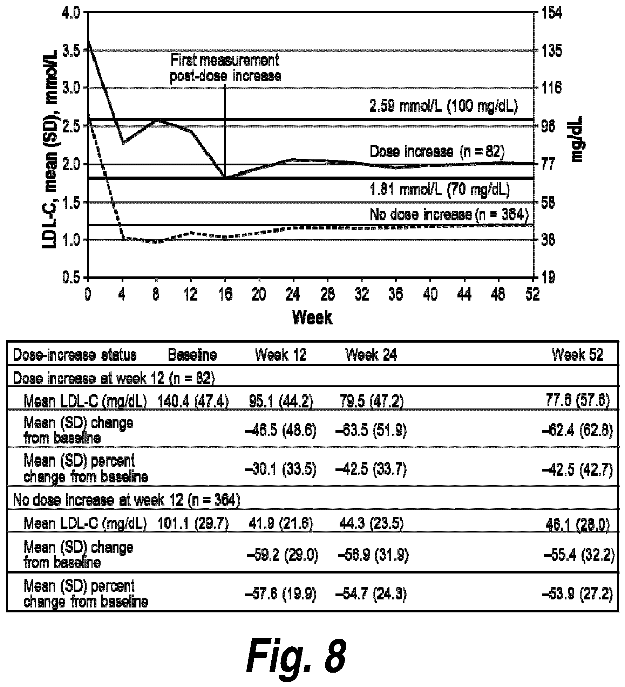

**Figure 8** is a graph of LDL-C concentrations versus study time point by dose-increase status (intention-to-treat analysis) for the alirocumab and ezetimibe groups in the ODYSSEY COMBO II study. LS = least squares; SD = standard deviation; SE = standard error.

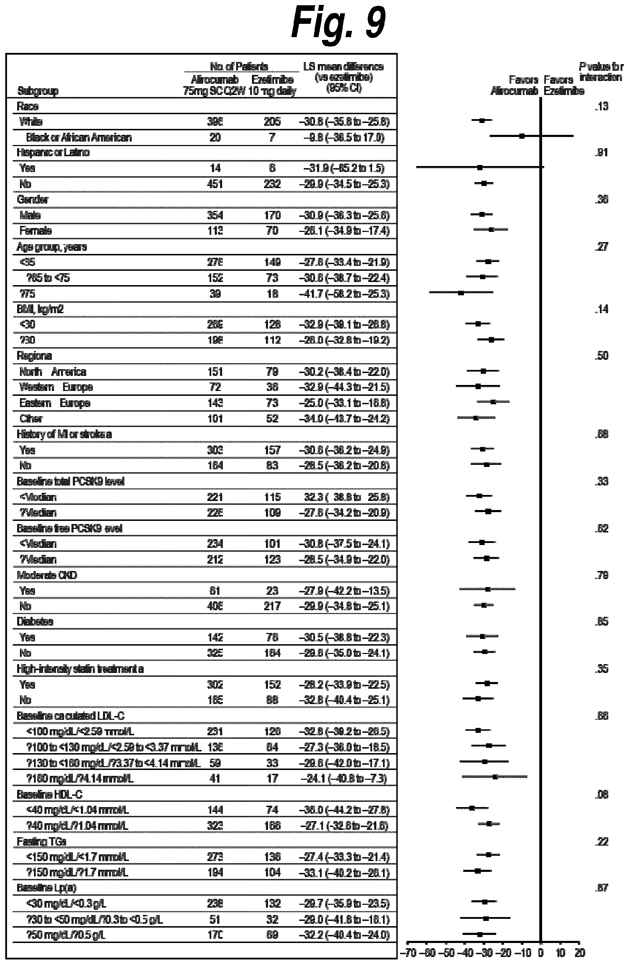

**Figure 9** is chart of the percent change from baseline to week 24 in LDL-C in various subgroups (ITT analysis) of the alirocumab and ezetimibe groups in the ODYSSEY COMBO II study. BMI = body mass index; CI = confidence interval; CKD = chronic kidney disease; LS = least squares; MI = myocardial infarction; SC = subcutaneous; TG = triglyceride.

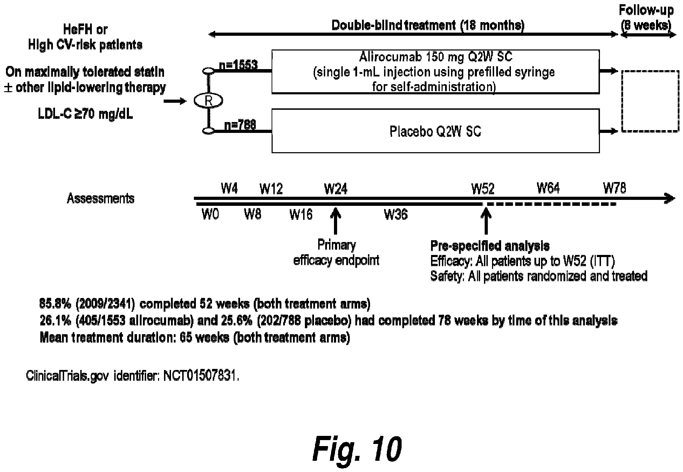

**Figure 10** is a graphic representation of the study design for ODYSSEY LONG TERM. Phone call visits are indicated in italics, and continue every 4 weeks between on-site visits until the end of the double-blind treatment period visit. Labels in the study design are defined as follows: HeFH: heterozygous familial hypercholesterolemia; LDL-C: low-density lipoprotein cholesterol; LLT: lipid-lowering therapy; Q2W: every two weeks; and SC: subcutaneous.

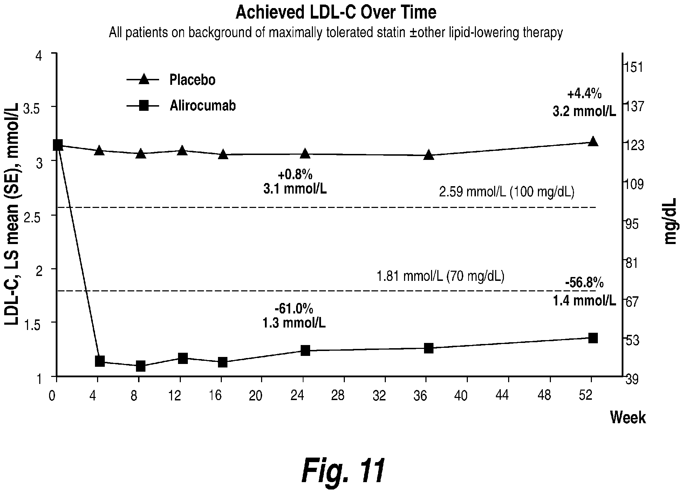

**Figure 11** is a graph that shows the calculated LDL-C LS mean (+/- SE) percent change from baseline for placebo and alirocumab over time for the ITT population in the ODYSSEY LONG TERM study at the time of the pre-specified analysis. Note: Least-squares (LS) means and standard errors (SE) taken from MMRM (mixed-effect model with repeated measures) analysis. The model includes the fixed categorical effects of treatment group, randomization strata as per IVRS, time point, treatment-by-time point interaction, strata-by-time point interaction as well as the continuous fixed covariates of baseline LDL-C value and baseline LDL-C-by-time point interaction.

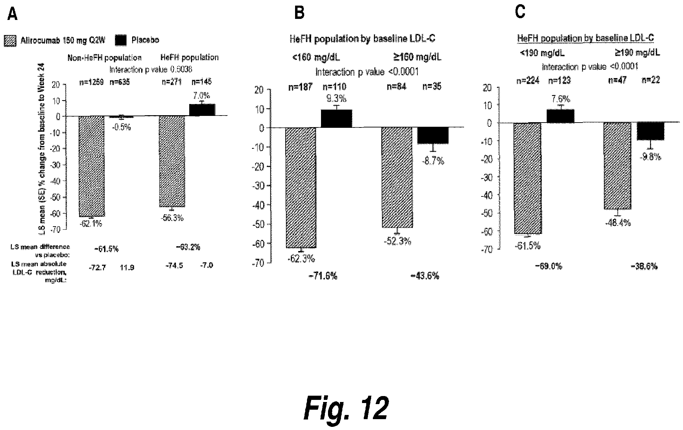

**Figure 12** is a set of graphs showing the LS mean (SE) percent change from baseline to Week 24 for LDL-C in the non-HeFH and HeFH populations of the ODYSSEY LONG TERM study (A), as well as the same population of HeFH patients separated by baseline LDL-C levels (< 160 mg/dL and ≥160 mg/dL (B); and < 190 mg/dL and ≥190 mg/dL. (C)). All patients were on a background statin (at the maximum tolerated level). A subset of patients also received a further lipid lowering therapy.

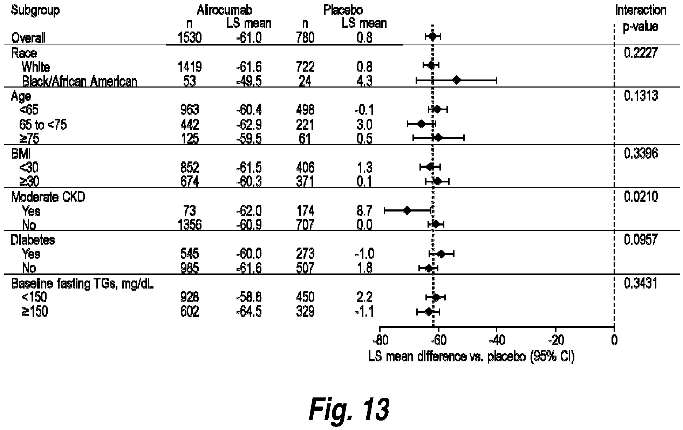

**Figure 13** is a graph demonstrating the difference in LS mean (SE) percent change from baseline to Week 24 in different subgroups of the ODYSSEY LONG TERM study. Labels in the graph are defined as follows: CKD: chronic kidney disease; TGs: triglycerides. All patients were on a background statin (at the maximum tolerated level). A subset of patients also received a further lipid lowering therapy.

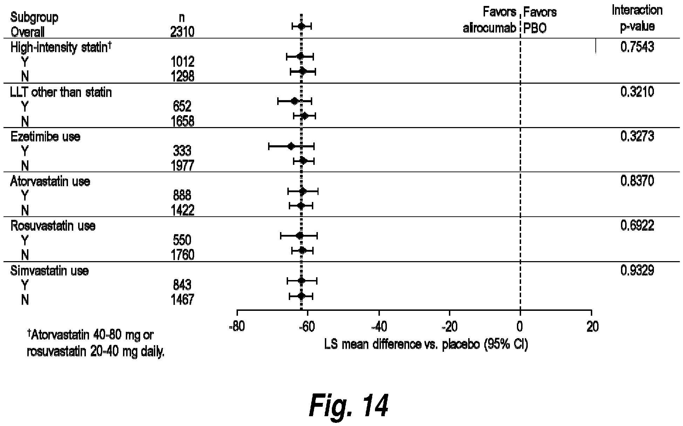

**Figure 14** is a graph demonstrating the difference in LS mean (SE) percent change from baseline to Week 24 in specific statin and LLT use subgroups of the ODYSSEY LONG TERM study. All patients were on a background statin (at the maximum tolerated level). A subset of patients also received a further lipid lowering therapy.

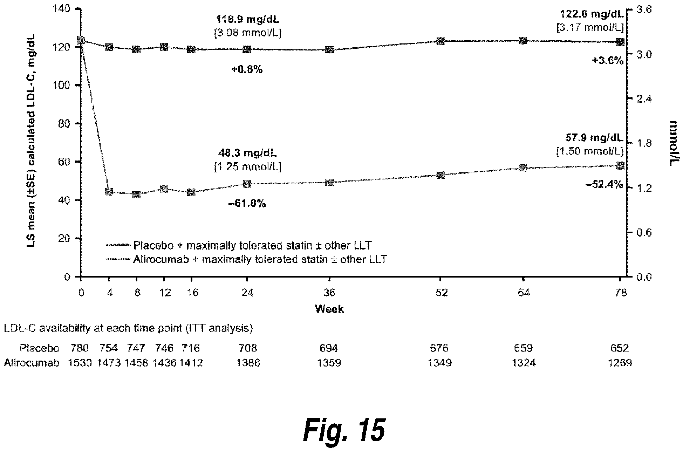

**Figure 15** is a graph that shows the calculated LDL-C LS mean (+/- SE) percent change from baseline for placebo and alirocumab over time for the ITT population in the ODYSSEY LONG TERM study. Values above data points indicate LS mean absolute LDL cholesterol levels, values below data points indicate LS mean % change from baseline. Values below chart indicate number of patients with LDL cholesterol values available for ITT analysis at each time point, i.e., LDL cholesterol measurements taken both on-treatment and post-treatment (for patients who had discontinued study treatment but had returned to the clinic for assessments). Missing data were accounted for using a mixed effects model with repeated measures.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (*e.g.*, 99.1, 99.2, 99.3, 99.4, etc.).

Although any methods and materials similar or equivalent to those described herein can be used in the practice of the present invention, the preferred methods and materials are now described. All publications mentioned herein are incorporated herein by reference to describe in their entirety.

### Hypercholesterolemia and Established Coronary Heart Disease or CHD Risk Equivalents Not Adequately Controlled by Maximum Tolerated Dose Statin Therapy

The present invention relates generally to methods and compositions for treating high cardiovascular risk patients who have hypercholesterolemia and established CHD or CHD risk equivalents that are not adequately controlled by statins, *i.e.*, hypercholesterolemia not adequately controlled by a therapeutic regimen comprising a daily maximum tolerated dose of a statin. As used herein, the expression "not adequately controlled," in reference to hypercholesterolemia, means that the patient's serum low-density lipoprotein cholesterol (LDL-C) concentration, total cholesterol concentration, and/or triglyceride concentration is not reduced to a recognized, medically-acceptable level (taking into account the patient's relative risk of coronary heart disease) after at least 4 weeks on a therapeutic regimen comprising a stable daily dose of a statin. For example, "a patient with hypercholesterolemia that is not adequately controlled by a statin" includes patients with a serum LDL-C concentration of greater than about 70 mg/dL, 100 mg/dL, 130 mg/dL, 140 mg/dL, or more (depending on the patient's underlying risk of heart disease) after the patient has been on a stable daily statin regimen for at least 4 weeks.

According to certain embodiments, the high cardiovascular risk patient who is treatable by the methods of the present invention has hypercholesterolemia (*e.g.*, a serum LDL-C concentration of greater than or equal to 70 mg/dL) despite taking a stable daily dose of a statin (with or without other lipid modifying therapy) for at least 4 weeks, 5 weeks, 6 weeks, or more. In certain embodiments, the high cardiovascular risk patient's hypercholesterolemia is inadequately controlled by a maximum tolerated dose statin therapy (also referred to herein as "a daily maximum tolerated dose therapeutic statin regimen").

As used herein, "maximum tolerated dose statin therapy" means a therapeutic regimen comprising the administration of daily dose of a statin that is the maximally tolerated dose for a particular patient. Maximally tolerated dose means the highest dose of statin that can be administered to a patient without causing unacceptable adverse side effects in the patient. Maximum tolerated dose statin therapy includes, but is not limited to, e.g., 40-80 mg of atorvastatin daily, 20-40 mg of rosuvastatin daily, or 80 mg of simvastatin (if already on this dose for >1 year). However, patients not able to tolerate the above statin doses could take a lower dose of daily atorvastatin, rosuvastatin, or simvastatin provided there was an acceptable reason for not using the higher dose. Some examples of acceptable reasons for a patient taking a lower statin dose include: adverse effects on higher doses, advanced age, low body mass index (BMI), regional practices, local prescribing information, concomitant medications, concern about cognitive adverse events, and comorbid conditions such as impaired glucose tolerance/impaired fasting glucose, increased blood glucose, glycated hemoglobin, liver disease or elevated liver enzymes, and muscle symptoms and/or raised creatine phosphokinase.

The present invention also includes methods for treating high cardiovascular risk patients with hypercholesterolemia and established CHD or CHD risk equivalents that are not adequately controlled by maximum tolerated dose statin therapy comprising daily administration of other statins such as cerivastatin, pitavastatin, fluvastatin, lovastatin, and pravastatin.

### Patient Selection

The present invention includes methods and compositions useful for treating high cardiovascular risk patients who have hypercholesterolemia and established CHD or CHD risk equivalents that are not adequately controlled by a daily maximum tolerated dose therapeutic statin regimen.

Established CHD is defined as a documented history of CHD, including one or more of the following: acute myocardial infarction (MI), silent MI, unstable angina, coronary revascularization procedure (e.g., percutaneous coronary intervention [PCI] or coronary artery bypass graft [CABG] surgery), and clinically significant CHD diagnosed by invasive or non-invasive testing (such as coronary angiography, stress test using treadmill, stress echocardiography, or nuclear imaging).

CHD risk equivalents includes one or more of the following 4 criteria: 1) peripheral arterial disease (PAD); 2) ischemic stroke; 3) chronic kidney disease; and/or 4) known history of diabetes mellitus and 2 or more additional risk factors, including: a) history of hypertension (established on antihypertensive medication), b) history of ankle-brachial index ≤0.90, c) history of microalbuminuria or macroalbuminuria or dipstick urinalysis at screening visit (Week -2) with >2+ protein, d) history of preproliferative or proliferative retinopathy or laser treatment for retinopathy, e) known family history of premature CHD (CHD in father or brother before 55 years of age; CHD in mother or sister before 65 years of age).

As used herein, in peripheral arterial disease (PAD), one of the following criteria [a, b, or c] must be satisfied): a) current intermittent claudication (muscle discomfort in the lower limb produced by exercise that is both reproducible and relieved by rest within 10 minutes) of presumed atherosclerotic origin together with ankle-brachial index equal to or less than 0.90 in either leg at rest, or b) history of intermittent claudication together with endovascular procedure or surgical intervention in one or both legs because of atherosclerotic disease, or c) history of critical limb ischemia together with thrombolysis, endovascular procedure or surgical intervention in one or both legs because of atherosclerotic disease.

As used herein, documented ischemic stroke includes ischemic stroke with a focal ischemic neurological deficit that persisted more than 24 hours, considered as being of atherothrombotic origin. Computed tomography (CT) or magnetic resonance imaging (MRI) can be performed to rule out hemorrhage and non-ischemic neurological disease.

As used herein, chronic kidney disease (CKD) is defined by an estimated glomerular filtration rate (eGFR) of ≥30-<60 ml/min/1.73 m2 for 3 months or more.

According to certain embodiments, the high cardiovascular risk patient may be selected on the basis of having one or more additional risk factors selected from the group consisting of age (*e.g.*, older than 40, 45, 50, 55, 60, 65, 70, 75, or 80 years), race, national origin, gender (male or female), exercise habits (*e.g.*, regular exerciser, non-exerciser), other preexisting medical conditions (*e.g.*, type-II diabetes, high blood pressure, etc.), and current medication status (*e.g.*, currently taking beta blockers, niacin, ezetimibe, fibrates, omega-3 fatty acids, bile acid resins, etc.).

According to the present invention, high cardiovascular risk patients may be selected on the basis of a combination of one or more of the foregoing selection criteria or therapeutic characteristics.

### Administration of a PCSK9 Inhibitor as Add-On Therapy to Maximum Tolerated Dose Statin Therapy

The present invention includes methods wherein a high cardiovascular risk patient with hypercholesterolemia and established CHD or CHD risk equivalents that are not adequately controlled by a stable daily maximum tolerated dose therapeutic statin regimen in the absence of a PCSK9 inhibitor is administered a PCSK9 inhibitor according to a particular dosing amount and frequency, and wherein the PCSK9 inhibitor is administered as an add-on to the patient's therapeutic statin regimen. For example, according to certain embodiments, if a high cardiovascular risk patient has hypercholesterolemia and established CHD or CHD risk equivalents that are not adequately controlled despite being on a stable daily maximum tolerated dose therapeutic statin regimen comprising, *e.g.*, 40-80 mg of atorvastatin, the high cardiovascular risk patient may be administered a PCSK9 inhibitor at a particular amount and dosing interval while the patient continues his or her stable daily therapeutic statin regimen.

The methods of the present invention include add-on therapeutic regimens wherein the PCSK9 inhibitor is administered as add-on therapy to the same stable daily maximum tolerated dose therapeutic statin regimen (*i.e.*, same dosing amount of statin) that the high cardiovascular risk patient was on prior to receiving the PCSK9 inhibitor. In other embodiments, the PCSK9 inhibitor is administered as add-on therapy to a daily maximum tolerated dose therapeutic statin regimen comprising a statin in an amount that is more than or less than the dose of statin the patient was on prior to receiving the PCSK9 inhibitor. For example, after starting a therapeutic regimen comprising a PCSK9 inhibitor administered at a particular dosing frequency and amount, the daily dose of statin administered or prescribed to the patient may (a) stay the same, (b) increase, or (c) decrease (*e.g.*, up-titrate or down-titrate) in comparison to the daily statin dose the high cardiovascular risk patient was taking before starting the PCSK9 inhibitor therapeutic regimen, depending on the therapeutic needs of the patient.

### Therapeutic Efficacy

The methods of the present invention will result in the improvement in the serum level of one or more lipid components selected from the group consisting of LDL-C, ApoB, non-HDL-C, total cholesterol, HDL-C, ApoA-1, triglycerides, and Lp(a). For example, according to certain embodiments of the present invention, administration of a pharmaceutical composition comprising a PCSK9 inhibitor to a high cardiovascular risk patient with hypercholesterolemia and established CHD or CHD risk equivalents that are not adequately controlled by a stable daily maximum tolerated dose therapeutic statin regimen (*e.g.*, administration of the PCSK9 inhibitor on top of the patient's maximum tolerated dose statin therapy) will result in a mean percent reduction from baseline in serum low density lipoprotein cholesterol (LDL-C) of at least about 40%, 41%, 42%, 43%, 44%, 45%, 46%, 47%, 48%, 49%, 50%, 51%, 52%, 53%, 54%, 55%, 56%, 57%, 58%, 59%, 60%, or greater; a mean percent reduction from baseline in ApoB of at least about 35%, 36%, 37%, 38%, 39%, 40%, or greater; a mean percent reduction from baseline in non-HDL-C of at least about 35%, 36%, 37%, 38%, 39%, 40%, 41%, 42%, 43%, 44%, 45%, 46%, 47%, 48%, 49%, 50%, or greater; a mean percent reduction from baseline in total cholesterol of at least about 20%, 21%, 22%, 23%, 24%, 25%, 26%, 27%, 28%, 29%, 30%, 31%, 32%, 33%, 34%, 35%, or greater; a mean percent increase from baseline in HDL-C of at least about 1%, 2%, 3%, 4%, 5%, 6%, 7%, 8%, 9%, 10%, or greater; a mean percent increase from baseline in ApoA-1 of at least about 1%, 2%, 3%, 4%, 5%, 6%, 7%, 8%, 9%, 10%, or greater; a mean percent reduction from baseline in triglycerides of at least about 1%, 2%, 3%, 4%, 5%, 6%, 7%, 8%, 9%, 10%, or greater; and/or a mean percent reduction from baseline in Lp(a) of at least about 10%, 11%, 12%, 13%, 14%, 15%, 16%, 17%, 18%, 19%, 20%, 21%, 22%, 23%, 24%, 25%, or greater.

### PCSK9 Inhibitors

The methods of the present invention comprise administering to a high cardiovascular risk patient a therapeutic composition comprising a PCSK9 inhibitor. As used herein, a "PCSK9 inhibitor" is any agent which binds to or interacts with human PCSK9 and inhibits the normal biological function of PCSK9 *in vitro* or *in vivo.* Non-limiting examples of categories of PCSK9 inhibitors include small molecule PCSK9 antagonists, peptide-based PCSK9 antagonists (*e.g.*, "peptibody" molecules), and antibodies or antigen-binding fragments of antibodies that specifically bind human PCSK9.

The term "human proprotein convertase subtilisin/kexin type 9" or "human PCSK9" or "hPCSK9", as used herein, refers to PCSK9 having the nucleic acid sequence shown in SEQ ID NO:197 and the amino acid sequence of SEQ ID NO:198, or a biologically active fragment thereof.

The term "antibody", as used herein, is intended to refer to immunoglobulin molecules comprising four polypeptide chains, two heavy (H) chains and two light (L) chains interconnected by disulfide bonds, as well as multimers thereof (*e.g.*, IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments of the invention, the FRs of the anti-PCSK9 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, *e.g.*, commercial sources, DNA libraries (including, *e.g.*, phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (*e.g.*, an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (*e.g.* monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody of the present invention include: (i) V_{H}-C_{H}1; (ii) V_{H}-C_{H}2; (iii) V_{H}-C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L}-C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (*e.g.*, 5, 10, 15, 20, 40, 60 or more) amino acids which result in a flexible or semi-flexible linkage between adjacent variable and/or constant domains in a single polypeptide molecule. Moreover, an antigen-binding fragment of an antibody of the present invention may comprise a homo-dimer or hetero-dimer (or other multimer) of any of the variable and constant domain configurations listed above in non-covalent association with one another and/or with one or more monomeric V_{H} or V_{L} domain (*e.g.*, by disulfide bond(s)).

As with full antibody molecules, antigen-binding fragments may be monospecific or multispecific (*e.g*., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody of the present invention using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglobulin sequences. The human antibodies of the invention may nonetheless include amino acid residues not encoded by human germline immunoglobulin sequences (e.g., mutations introduced by random or site-specific mutagenesis *in vitro* or by somatic mutation *in vivo*), for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant invention encompasses antibodies having one or more mutations in the hinge, C_{H}2 or C_{H}3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" PCSK9, as used in the context of the present invention, includes antibodies that bind PCSK9 or portion thereof with a K_{D} of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human PCSK9, however, have cross-reactivity to other antigens, such as PCSK9 molecules from other (non-human) species.

The anti-PCSK9 antibodies useful for the methods of the present invention may comprise one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The present invention includes methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments which comprise one or more individual germline mutations or combinations thereof. In certain embodiments, all of the framework and/or CDR residues within the V_{H} and/or V_{L} domains are mutated back to the residues found in the original germline sequence from which the antibody was derived. In other embodiments, only certain residues are mutated back to the original germline sequence, e.g., only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding residue(s) of a different germline sequence (*i.e.*, a germline sequence that is different from the germline sequence from which the antibody was originally derived). Furthermore, the antibodies of the present invention may contain any combination of two or more germline mutations within the framework and/or CDR regions, *e.g.*, wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present invention.

The present invention also includes methods involving the use of anti-PCSK9 antibodies comprising variants of any of the HCVR, LCVR, and/or CDR amino acid sequences disclosed herein having one or more conservative substitutions. For example, the present invention includes the use of anti-PCSK9 antibodies having HCVR, LCVR, and/or CDR amino acid sequences with, *e.g.*, 10 or fewer, 8 or fewer, 6 or fewer, 4 or fewer, etc. conservative amino acid substitutions relative to any of the HCVR, LCVR, and/or CDR amino acid sequences disclosed herein.

The term "surface plasmon resonance", as used herein, refers to an optical phenomenon that allows for the analysis of real-time interactions by detection of alterations in protein concentrations within a biosensor matrix, for example using the BIAcore™ system (Biacore Life Sciences division of GE Healthcare, Piscataway, NJ).

The term "K_{D}", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstances, an epitope may include moieties of saccharides, phosphoryl groups, or sulfonyl groups on the antigen.

According to certain embodiments, the anti-PCSK9 antibody used in the methods of the present invention is an antibody with pH-dependent binding characteristics. As used herein, the expression "pH-dependent binding" means that the antibody or antigen-binding fragment thereof exhibits "reduced binding to PCSK9 at acidic pH as compared to neutral pH" (for purposes of the present disclosure, both expressions may be used interchangeably). For example, antibodies "with pH-dependent binding characteristics" includes antibodies and antigen-binding fragments thereof that bind PCSK9 with higher affinity at neutral pH than at acidic pH. In certain embodiments, the antibodies and antigen-binding fragments of the present invention bind PCSK9 with at least 3, 5, 10, 15, 20, 25, 30, 35, 40, 45, 50, 55, 60, 65, 70, 75, 80, 85, 90, 95, 100, or more times higher affinity at neutral pH than at acidic pH.

According to this aspect of the invention, the anti-PCSK9 antibodies with pH-dependent binding characteristics may possess one or more amino acid variations relative to the parental anti-PCSK9 antibody. For example, an anti-PCSK9 antibody with pH-dependent binding characteristics may contain one or more histidine substitutions or insertions, e.g., in one or more CDRs of a parental anti-PCSK9 antibody. Thus, according to certain embodiments of the present invention, methods are provided comprising administering an anti-PCSK9 antibody which comprises CDR amino acid sequences (*e.g.*, heavy and light chain CDRs) which are identical to the CDR amino acid sequences of a parental anti-PCSK9 antibody, except for the substitution of one or more amino acids of one or more CDRs of the parental antibody with a histidine residue. The anti-PCSK9 antibodies with pH-dependent binding may possess, *e.g.*, 1, 2, 3, 4, 5, 6, 7, 8, 9, or more histidine substitutions, either within a single CDR of a parental antibody or distributed throughout multiple (*e.g*., 2, 3, 4, 5, or 6) CDRs of a parental anti-PCSK9 antibody. For example, the present invention includes the use of anti-PCSK9 antibodies with pH-dependent binding comprising one or more histidine substitutions in HCDR1, one or more histidine substitutions in HCDR2, one or more histidine substitutions in HCDR3, one or more histidine substitutions in LCDR1, one or more histidine substitutions in LCDR2, and/or one or more histidine substitutions in LCDR3, of a parental anti-PCSK9 antibody.

As used herein, the expression "acidic pH" means a pH of 6.0 or less (*e.g.*, less than about 6.0, less than about 5.5, less than about 5.0, etc.). The expression "acidic pH" includes pH values of about 6.0, 5.95, 5.90, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. As used herein, the expression "neutral pH" means a pH of about 7.0 to about 7.4. The expression "neutral pH" includes pH values of about 7.0, 7.05, 7.1, 7.15, 7.2, 7.25, 7.3, 7.35, and 7.4.

### Preparation of Human Antibodies

Methods for generating human antibodies in transgenic mice are known in the art. Any such known methods can be used in the context of the present invention to make human antibodies that specifically bind to human PCSK9.

Using VELOCIMMUNE® technology (see, for example, US 6,596,541, Regeneron Pharmaceuticals) or any other known method for generating monoclonal antibodies, high affinity chimeric antibodies to PCSK9 are initially isolated having a human variable region and a mouse constant region. The VELOCIMMUNE® technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE® mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc, using standard procedures known to those skilled in the art. The mouse constant regions are replaced with a desired human constant region to generate the fully human antibody of the invention, for example wild-type or modified IgG1 or IgG4. While the constant region selected may vary according to specific use, high affinity antigen-binding and target specificity characteristics reside in the variable region.

In general, the antibodies that can be used in the methods of the present invention possess high affinities, as described above, when measured by binding to antigen either immobilized on solid phase or in solution phase. The mouse constant regions are replaced with desired human constant regions to generate the fully human antibodies of the invention. While the constant region selected may vary according to specific use, high affinity antigen-binding and target specificity characteristics reside in the variable region.

Specific examples of human antibodies or antigen-binding fragments of antibodies that specifically bind PCSK9 which can be used in the context of the methods of the present invention include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs: 1 and 11, or a substantially similar sequence thereof having at least 90%, at least 95%, at least 98% or at least 99% sequence identity. Alternatively, specific examples of human antibodies or antigen-binding fragments of antibodies that specifically bind PCSK9 which can be used in the context of the methods of the present invention include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs 37, 45, 53, 61, 69, 77, 85, 93, 101, 109, 117, 125, 133, 141, 149, 157, 165, 173, 181, and 189, or a substantially similar sequence thereof having at least 90%, at least 95%, at least 98% or at least 99% sequence identity. The antibody or antigen-binding fragment may comprise the three light chain CDRs (LCVR1, LCVR2, LCVR3) contained within a light chain variable region (LCVR) having an amino acid sequence selected from the group consisting of SEQ ID NOs: 6 and 15, or a substantially similar sequence thereof having at least 90%, at least 95%, at least 98% or at least 99% sequence identity. Alternatively, the antibody or antigen-binding fragment may comprise the three light chain CDRs (LCVR1, LCVR2, LCVR3) contained within a light chain variable region (LCVR) having an amino acid sequence selected from the group consisting of SEQ ID NOs 41, 49, 57, 65, 73, 81, 89, 97, 105, 113, 121, 129, 137, 145, 153, 161, 169, 177, 185, and 193, or a substantially similar sequence thereof having at least 90%, at least 95%, at least 98% or at least 99% sequence identity.

In certain embodiments of the present invention, the antibody or antigen-binding fragment thereof comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs: 1/6 and 11/15. Alternatively, in certain embodiments of the present invention, the antibody or antigen-binding protein comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs:37/41, 45/49, 53/57, 61/65, 69/73, 77/81, 85/89, 93/97, 101/105, 109/113, 117/121, 125/129, 133/137, 141/145, 149/153, 157/161, 165/169, 173/177, 181/185, and 189/193.

In certain embodiments of the present invention, the anti-PCSK9 antibody, or antigen-binding fragment thereof, that can be used in the methods of the present invention has HCDR1/HCDR2/HCDR3/LCDR1/LCDR2/LCDR3 amino acid sequences selected from SEQ ID NOs: 2/3/4/7/8/10 (mAb316P) and 12/13/14/16/17/18 (mAb300N) (See US Patent App. Publ No. 2010/0166768).

In certain embodiments of the present invention, the antibody or antigen-binding fragment thereof comprises HCVR/LCVR amino acid sequence pairs selected from the group consisting of SEQ ID NOs: 1/6 and 11/15. Alternatively, in certain embodiments of the present invention, the antibody or antigen-binding protein comprises the heavy and light chain variable region amino acid sequence pairs (HCVR/LCVR) selected from the group consisting of SEQ ID NOs:37/41, 45/49, 53/57, 61/65, 69/73, 77/81, 85/89, 93/97, 101/105, 109/113, 117/121, 125/129, 133/137, 141/145, 149/153, 157/161, 165/169, 173/177, 181/185, and 189/193.

### Pharmaceutical Compositions and Methods of Administration

The present invention includes methods which comprise administering a PCSK9 inhibitor to a high cardiovascular risk patient, wherein the PCSK9 inhibitor is contained within a pharmaceutical composition. The pharmaceutical compositions of the invention are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Various delivery systems are known and can be used to administer the pharmaceutical composition of the invention, *e.g.*, encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition of the present invention can be delivered subcutaneously or intravenously with a standard needle and syringe. In addition, with respect to subcutaneous delivery, a pen delivery device readily has applications in delivering a pharmaceutical composition of the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the present invention. Examples include, but are not limited to AUTOPEN™ (Owen Mumford, Inc., Woodstock, UK), DlSETRONlC™ pen (Disetronic Medical Systems, Bergdorf, Switzerland), HUMALOG MIX 75/25™ pen, HUMALOG™ pen, HUMALlN 70/30™ pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™, OPTIPEN STARLET™, and OPTICLIK™ (sanofi-aventis, Frankfurt, Germany), to name only a few. Examples of disposable pen delivery devices having applications in subcutaneous delivery of a pharmaceutical composition of the present invention include, but are not limited to the SOLOSTAR™ pen (sanofi-aventis), the FLEXPEN™ (Novo Nordisk), and the KWIKPEN™ (Eli Lilly), the SURECLICK™ Autoinjector (Amgen, Thousand Oaks, CA), the PENLET™ (Haselmeier, Stuttgart, Germany), the EPIPEN (Dey, L.P.), and the HUMIRA™ Pen (Abbott Labs, Abbott Park IL), to name only a few.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, *e.g.*, Goodson, 1984, in Medical Applications of Controlled Release, supra, vol. 2, pp. 115-138). Other controlled release systems are discussed in the review by Langer, 1990, Science 249: 1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, *e.g.*, by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

### Dosage

The amount of PCSK9 inhibitor (*e.g.*, anti-PCSK9 antibody) administered to a high cardiovascular risk subject according to the methods of the present invention is, generally, a therapeutically effective amount. As used herein, the phrase "therapeutically effective amount" means a dose of PCSK9 inhibitor that results in a detectable improvement (at least about 5%, 10%, 15%, 20%, 25%, 30%, 35%, 40%, 45%, 50%, 55%, 60%, 65%, 70%, 75%, or more from baseline) in one or more parameters selected from the group consisting of LDL-C, ApoB, non-HDL-C, total cholesterol, HLDL-C, triglycerides, and Lp(a).

In the case of an anti-PCSK9 antibody, a therapeutically effective amount can be from about 0.05 mg to about 600 mg, *e.g.*, about 0.05 mg, about 0.1 mg, about 1.0 mg, about 1.5 mg, about 2.0 mg, about 10 mg, about 20 mg, about 30 mg, about 40 mg, about 50 mg, about 60 mg, about 70 mg, about 75 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-PCSK9 antibody.

The amount of anti-PCSK9 antibody contained within the individual doses may be expressed in terms of milligrams of antibody per kilogram of patient body weight (*i.e.*, mg/kg). For example, the anti-PCSK9 antibody may be administered to a patient at a dose of about 0.0001 to about 10 mg/kg of patient body weight.

### Combination Therapies

As described elsewhere herein, the methods of the present invention may comprise administering a PCSK9 inhibitor to a high cardiovascular risk patient in combination with the patient's previously prescribed stable daily maximum tolerated dose therapeutic statin regimen. According to certain embodiments of the present invention, additional therapeutic agents, besides a statin, may be administered to the patient in combination with the PCSK9 inhibitor. Examples of such additional therapeutic agents include *e.g.*, (1) an agent which inhibits cholesterol uptake and or bile acid re-absorption (*e.g.*, ezetimibe); (2) an agent which increase lipoprotein catabolism (such as niacin); and/or (3) activators of the LXR transcription factor that plays a role in cholesterol elimination such as 22-hydroxycholesterol.

### Administration Regimens

According to certain embodiments of the present invention, multiple doses of a PCSK9 inhibitor (*i.e.*, a pharmaceutical composition comprising a PCSK9 inhibitor) may be administered to a subject over a defined time course (*e.g.*, on top of a daily therapeutic statin regimen). The methods according to this aspect of the invention comprise sequentially administering to a high cardiovascular risk subject multiple doses of a PCSK9 inhibitor. As used herein, "sequentially administering" means that each dose of PCSK9 inhibitor is administered to the subject at a different point in time, *e.g.*, on different days separated by a predetermined interval (*e.g.*, hours, days, weeks or months). The present invention includes methods which comprise sequentially administering to the high cardiovascular risk patient a single initial dose of a PCSK9 inhibitor, followed by one or more secondary doses of the PCSK9 inhibitor, and optionally followed by one or more tertiary doses of the PCSK9 inhibitor.

The terms "initial dose," "secondary doses," and "tertiary doses," refer to the temporal sequence of administration of the individual doses of a pharmaceutical composition comprising a PCSK9 inhibitor. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of the PCSK9 inhibitor, but generally may differ from one another in terms of frequency of administration. In certain embodiments, however, the amount of PCSK9 inhibitor contained in the initial, secondary and/or tertiary doses varies from one another (*e.g.*, adjusted up or down as appropriate) during the course of treatment. In certain embodiments, two or more (*e.g*., 2, 3, 4, or 5) doses are administered at the beginning of the treatment regimen as "loading doses" followed by subsequent doses that are administered on a less frequent basis (*e.g.*, "maintenance doses").

According to exemplary embodiments of the present invention, each secondary and/or tertiary dose is administered 1 to 26 (*e.g.*, 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, 15, 15½, 16, 16½, 17, 17½, 18, 18½, 19, 19½, 20, 20½, 21, 21½, 22, 22½, 23, 23½, 24, 24½, 25, 25½, 26, 26½, or more) weeks after the immediately preceding dose. The phrase "the immediately preceding dose," as used herein, means, in a sequence of multiple administrations, the dose of antigen-binding molecule which is administered to a patient prior to the administration of the very next dose in the sequence with no intervening doses.

The methods according to this aspect of the invention may comprise administering to a high cardiovascular risk patient any number of secondary and/or tertiary doses of a PCSK9 inhibitor. For example, in certain embodiments, only a single secondary dose is administered to the patient. In other embodiments, two or more (*e.g.*, 2, 3, 4, 5, 6, 7, 8, or more) secondary doses are administered to the patient. Likewise, in certain embodiments, only a single tertiary dose is administered to the patient. In other embodiments, two or more (*e.g.*, 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the high cardiovascular risk patient 1 to 2, 4, 6, 8 or more weeks after the immediately preceding dose. Similarly, in embodiments involving multiple tertiary doses, each tertiary dose may be administered at the same frequency as the other tertiary doses. For example, each tertiary dose may be administered to the patient 1 to 2, 4, 6, 8 or more weeks after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

The present invention includes administration regimens comprising an up-titration option (also referred to herein as "dose modification"). As used herein, an "up-titration option" means that, after receiving a particular number of doses of a PCSK9 inhibitor, if a patient has not achieved a specified reduction in one or more defined therapeutic parameters, the dose of the PCSK9 inhibitor is thereafter increased. For example, in the case of a therapeutic regimen comprising administration of 75 mg doses of an anti-PCSK9 antibody to a high cardiovascular risk patient at a frequency of once every two weeks, if after 8 weeks (i.e., 5 doses administered at Week 0, Week 2 and Week 4, Week 6 and Week 8), the patient has not achieved a serum LDL-C concentration of less than 70 mg/dL, then the dose of anti-PCSK9 antibody is increased to *e.g.*, 150 mg administered once every two weeks thereafter (*e.g.*, starting at Week 12).

In certain embodiments, the anti-PCSK9 antibody is administered to a subject at a dose of about 150 mg every two weeks, for example for at least six doses.

In certain embodiments, the anti-PCSK9 antibody is administered to a subject at a dose of about 75 mg every two weeks, for example for at least six doses.

In some embodiments, the antibody is administered to a subject at a dose of about 75 mg every two weeks for 12 weeks, and the dose remains at 75 mg every two weeks if, at week 8, the subject's LDL-C value was less than 70 mg/dl.

In other embodiments, the antibody is administered to a subject at a dose of about 75 mg every two weeks for 12 weeks, and the dose is titrated up to about 150 mg every two weeks if, at week 8, the subject's LDL-C value was greater than or equal to 70 mg/dl.

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human PCSK9

Human anti-PCSK9 antibodies were generated as described in US Patent No. 8,062,640. The exemplary PCSK9 inhibitor used in the following Example is the human anti-PCSK9 antibody designated "mAb316P," also known as "Alirocumab." mAb316P has the following amino acid sequence characteristics: heavy chain variable region (HCVR) comprising SEQ ID NO:1; light chain variable domain (LCVR) comprising SEQ ID NO:6; heavy chain complementarity determining region 1 (HCDR1) comprising SEQ ID NO:2; HCDR2 comprising SEQ ID NO:3; HCDR3 comprising SEQ ID NO:4; light chain complementarity determining region 1 (LCDR1) comprising SEQ ID NO:7; LCDR2 comprising SEQ ID NO:8; and LCDR3 comprising SEQ ID NO:10.

### Example 2: Efficacy and Safety of Alirocumab, a Fully Human PCSK9 Monoclonal Antibody ("mAb316P"), in High Cardiovascular Risk Patients with Uncontrolled Hypercholesterolemia on Maximally Tolerated Doses of Statins

### INTRODUCTION

The objective of the COMBO I and II studies was to evaluate the efficacy and safety of alirocumab ("mAb316P") as add-on therapy to stable, maximally tolerated daily statin therapy, with or without other lipid-lowering therapy, in patients with hypercholesterolemia at high cardiovascular risk. COMBO I was placebo-controlled and patients may have received other lipid-lowering therapies in addition to statin therapy; COMBO II compared alirocumab with ezetimibe in patients receiving statin therapy (but no other lipid-lowering therapies). Each of the COMBO studies was randomized, double-blinded, and placebo-controlled. Both studies utilized a starting dose of alirocumab 75 mg every 2 weeks (Q2W; administered as a 1 ml solution via auto-injector). Patients with LDL-C levels ≥70 mg/dl after 8 weeks of treatment were up-titrated in a blinded manner at Week 12 to alirocumab 150 mg Q2W (also 1 ml auto-injector).

### STUDY OBJECTIVES

The primary efficacy endpoint for each study was the difference between treatment arms in the percent change in LDL-C from baseline to Week 24.

The key secondary endpoints for each study were: percentage change in calculated LDL-C from baseline to Week 24 in the on-treatment population, using all low-density lipoprotein cholesterol (LDL-C) values during the efficacy treatment period (ITT estimand); percentage change in calculated LDL-C from baseline to Week 12 (ITT estimand); percentage change in calculated LDL-C from baseline to Week 12 (on-treatment estimand); percentage change in apoliprotein (Apo) B from baseline to Week 24 (ITT estimand); percentage change in Apo B from baseline to Week 24 (on-treatment estimand); percentage change in non high-density lipoprotein cholesterol (non-HDL-C) from baseline to Week 24 (ITT estimand); percentage change in non-HDL-C from baseline to Week 24 (on-treatment estimand); percentage change in total cholesterol from baseline to Week 24 (ITT estimand); percentage change in Apo B from baseline to Week 12 (ITT estimand); percentage change in non-HDL-C from baseline to Week 12 (ITT estimand); percentage change in total cholesterol from baseline to Week 12 (ITT estimand); percentage change in calculated LDL-C from baseline to Week 52 (ITT estimand); proportion of patients reaching calculated LDL-C <70 mg/dl (1.81 mmol/l) at Week 24 (ITT estimand); proportion of patients reaching calculated LDL-C <70 mg/dl (1.81 mmol/l) at Week 24 (on-treatment estimand); percentage change in lipoproten (a) (Lp(a)) from baseline to Week 24 (ITT estimand); percentage change in HDL-C from baseline to Week 24 (ITT estimand); percentage change in fasting triglycerides (TG) from baseline to Week 24 (ITT estimand); percentage change in Apo A1 from baseline to Week 24 (ITT estimand); percentage change in Lp(a) from baseline to Week 12 (ITT estimand); percentage change in HDL-C from baseline to Week 12 (ITT estimand); percentage change in fasting TG from baseline to Week 12 (ITT estimand); and percentage change in Apo A1 from baseline to Week 12 (ITT estimand). Apo, apolipoprotein; HDL-C, high-density lipoprotein cholesterol; ITT, intent-to-treat; LDL-C, low-density lipoprotein cholesterol; Lp(a), lipoprotein (a); TG, triglycerides.

### STUDY DESIGN

COMBO I was a Phase 3, randomized, double-blind, placebo-controlled, parallel-group, multicenter, 52-week study. This study evaluated the efficacy and safety of alirocumab as add-on therapy to stable, maximally tolerated doses of daily statin, with or without other stable lipid lowering therapies, in high-risk patients with uncontrolled hypercholesterolemia (**Figure 1A**).

COMBO II was a Phase 3, randomized, double-blind, active-controlled, parallel-group, multinational 104-week study. Thestudy enrolled high-risk patients with uncontrolled hypercholesterolemia on stable, maximally tolerated daily statin therapy. Unlike COMBO I, with a placebo arm, COMBO II incorporated an active-treatment arm (ezetimibe) with double-dummy design (**Figure 1B**) and patients were not allowed to receive any other lipid lowering therapies besides statin and their randomized treatment.

### PATIENT SELECTION

All patients in COMBO I and II had hypercholesterolemia and established CHD or CHD risk equivalents, with LDL-C inadequately controlled with a maximally tolerated daily dose of statin. In COMBO I only, patients were also permitted to receive other lipid lowering therapies on top of statin, provided both the statin and other lipid lowering therapies were at stable dose for at least 4 weeks (6 weeks for fenofibrate) prior to the screening visit; in COMBO II, statin dose was required to be stable for at least 4 weeks prior to the screening visit and other lipid lowering therapies were not permitted. At screening, patients either had LDL-C ≥70 mg/dl (≥1.81 mmol/l) with documented CVD or LDL-C ≥100 mg/dl (≥2.59 mmol/l) with no documented history of CVD.

The 'maximum tolerated dose' of statin was defined as either rosuvastatin 20 mg or 40 mg daily, atorvastatin 40 mg or 80 mg daily, or simvastatin 80 mg daily (if already on this dose for >1 year). However, patients not able to tolerate the above statin doses remained eligible for inclusion if they were on a lower dose of daily atorvastatin, rosuvastatin, or simvastatin provided that the investigator had a documented reason for not using the higher dose.

### COMBO I

The inclusion criteria for the COMBO I study were as follows. Patients with hypercholesterolemia and established coronary heart disease (CHD) or CHD risk equivalents (see below for definitions) who are not adequately controlled with a maximally tolerated daily dose of statin with or without other lipid-lowering therapy (LLT), both at stable dose for at least 4 weeks prior to the screening visit (and 6 weeks for fenofibrate prior to the screening visit).

Definition of maximally tolerated dose (any of the following are acceptable): rosuvastatin 20 mg or 40 mg daily, atorvastatin 40 mg or 80 mg daily, or simvastatin 80 mg daily (if already on this dose for >1 year). Patients not able to be on any of the above statin doses were treated with the dose of daily atorvastatin, rosuvastatin, or simvastatin which is considered appropriate for the patient as per the investigator's judgment or concerns. Some examples of acceptable reasons for a patient taking a lower statin dose include: adverse effects on higher doses, advanced age, low body mass index (BMI), regional practices, local prescribing information, concomitant medications, and comorbid conditions such as impaired glucose tolerance/impaired fasting glucose.

Established CHD is defined as a documented history of CHD, including one or more of the following: acute myocardial infarction (MI), silent MI, unstable angina, coronary revascularization procedure (e.g., percutaneous coronary intervention [PCI] or coronary artery bypass graft [CABG] surgery), and clinically significant CHD diagnosed by invasive or non-invasive testing (such as coronary angiography, stress test using treadmill, stress echocardiography, or nuclear imaging).

CHD risk equivalents includes one or more of the following 4 criteria: 1) documented peripheral arterial disease (PAD) (one of the following criteria [a, b, or c] must be satisfied): a) current intermittent claudication (muscle discomfort in the lower limb produced by exercise that is both reproducible and relieved by rest within 10 minutes) of presumed atherosclerotic origin together with ankle-brachial index equal to or less than 0.90 in either leg at rest, or b) history of intermittent together with endovascular procedure or surgical intervention in one or both legs because of atherosclerotic disease, or c) history of critical limb ischemia together with thrombolysis, endovascular procedure or surgical intervention in one or both legs because of atherosclerotic disease; 2) documented ischemic stroke with a focal ischemic neurological deficit that persisted more than 24 hours, considered as being of atherothrombotic origin. Computed tomography (CT) or magnetic resonance imaging (MRI) must have been performed to rule out hemorrhage and non-ischemic neurological disease; 3) documented chronic kidney disease (CKD) as defined by estimated glomerular filtration rate (eGFR) of ≥30-<60 ml/min/1.73 m2 for 3 months or more, including the screening visit; and/or 4) known history of diabetes mellitus and 2 or more additional risk factors, including: a) history of hypertension (established on antihypertensive medication), b) documented history of ankle-brachial index ≤0.90, c) documented history of microalbuminuria or macroalbuminuria or dipstick urinalysis at screening visit (Week -2) with >2+ protein, d) documented history of preproliferative or proliferative retinopathy or laser treatment for retinopathy, e) known family history of premature CHD (CHD in father or brother before 55 years of age; CHD in mother or sister before 65 years of age).

Patients who met all the above inclusion criteria were screened for the following exclusion criteria.

Exclusion criteria related to study methodology were as follows: Patients without established CHD or CHD risk equivalents; LDL-C <70 mg/dl (<1.81 mmol/l) at the screening visit in patients with an history of documented cardiovascular disease [CVD]); LDL-C <100 mg/dl (<2.59 mmol/l) at the screening visit in patients without history of documented CVD); those not on a stable dose of lipid lowering therapy (including statin) for at least 4 weeks and/or fenofibrate for at least 6 weeks, as applicable, prior to the screening visit and from screening to randomization; currently taking a statin other than simvastatin, atorvastatin, or rosuvastatin; simvastatin, atorvastatin, or rosuvastatin is not taken daily or not taken at a registered dose; daily doses above atorvastatin 80 mg, rosuvastatin 40 mg, or simvastatin 40 mg (except for patients on simvastatin 80 mg for >1 year, who are eligible); use of fibrates, other than fenofibrate within 6 weeks of the screening visit or between screening and randomization visits; use of nutraceutical products or over-the-counter therapies that may affect lipids which have not been at a stable dose/amount for at least 4 weeks prior to the screening visit or between screening and randomization visits; use of red yeast rice products within 4 weeks of the screening visit or between screening and randomization visits; patients who have received plasmapheresis treatment within 2 months prior to the screening visit (Week -2), or have plans to receive it during the study; recent (within 3 months prior to the screening visit or between screening and randomization visits) MI, unstable angina leading to hospitalization, PCI, CABG, uncontrolled cardiac arrhythmia, stroke, transient ischemic attack, carotid revascularization, endovascular procedure or surgical intervention for peripheral vascular disease; planned to undergo scheduled PCI, CABG, carotid or peripheral revascularization during the study; systolic blood pressure (BP) >160 mmHg or diastolic BP >100 mmHg at screening visit or randomization visit; history of New York Heart Association (NYHA) Class III or IV heart failure within the past 12 months; known history of hemorrhagic stroke; age <18 years or legal age of majority at the screening visit, whichever is greater; patients not previously instructed on a cholesterol-lowering diet prior to the screening visit; newly diagnosed (within 3 months prior to randomization visit) or poorly controlled (glycated hemoglobin [HbA1c] >8.5% at the screening visit) diabetes; presence of any clinically significant uncontrolled endocrine disease known to influence serum lipids or lipoproteins (note: patients on thyroid replacement therapy can be included if the dosage has been stable for at least 12 weeks prior to screening and between screening and randomization visits, and thyroid-stimulating hormone (TSH) level is within the normal range of the Central Laboratory at the screening visit); history of bariatric surgery within 12 months prior to the screening visit; unstable weight defined by a variation >5 kg within 2 months prior to the screening visit; known history of homozygous or heterozygous familial hypercholesterolemia (FH); known history of loss of function of proprotein convertase subtilisin kexin 9 (PCSK9) (i.e., genetic mutation or sequence variation); use of systemic corticosteroids, unless used as replacement therapy for pituitary/adrenal disease with a stable regimen for at least 6 weeks prior to randomization visit (note: topical, intra-articular, nasal, inhaled, and ophthalmic steroid therapies are not considered as 'systemic' and are allowed); use of continuous estrogen or testosterone hormone replacement therapy unless the regimen has been stable in the past 6 weeks prior to the screening visit (Week-2) and no plans to change the regimen during the study; history of cancer within the past 5 years, except for adequately treated basal cell skin cancer, squamous cell skin cancer, or in situ cervical cancer; known history of HIV positivity; patients who have taken any investigational drugs other than the alirocumab training placebo kits within 1 month or 5 half-lives, whichever is longer; patients who have previously participated in any clinical trial of alirocumab or any other anti-PCSK9 therapy; patients who withdraw consent during the screening period (patients who are not willing to continue or fail to return).

Additional exclusion criteria were conditions/situations such as: any clinically significant abnormality identified at the time of screening that in the judgment of the investigator or any sub-investigator would preclude safe completion of the study or constrain endpoint assessment such as major systemic diseases or patients with short life expectancy; considered by the investigator or any sub-investigator as inappropriate for this study for any reason, e.g. deemed unable to meet specific protocol requirements, such as scheduled visits; deemed unable to administer or tolerate long-term injections based on the opinion of the patient or investigator; investigator or any sub-investigator, pharmacist, study coordinator, other study staff, or relative thereof directly involved in the conduct of the protocol, etc.; presence of any other conditions (e.g. geographic, social) actual or anticipated which the investigator feels would restrict or limit the patient's participation for the duration of the study.

Laboratory findings during the screening period (not including randomization assessments): positive test for hepatitis B surface antigen or hepatitis C antibody (confirmed by reflexive testing); positive serum beta-human chorionic gonadotropin (hCG) or urine pregnancy test (including Week 0) in women of childbearing potential; triglycerides >400 mg/dl (>4.52 mmol/l) (1 repeat assessment is allowed); eGFR <30 ml/min/1.73 m2; alanine aminotransferase (ALT) or aspartate aminotransferase (AST) >3 x upper limit of normal (ULN) (1 repeat assessment is allowed); creatine phosphokinase (CPK) >3 x ULN (1 repeat assessment is allowed); thyroid stimulating hormone (TSH) <lower limit of normal (LLN) or >ULN.

Exclusion criteria related to background therapy were as follows. All contraindications to the background therapies or warning/precaution of use (when appropriate) as displayed in the respective National Product labeling.

Exclusion criteria related to alirocumab were as follows. Known hypersensitivity to monoclonal antibody therapeutics; pregnant or breast-feeding women; women of childbearing potential with no effective contraceptive method of birth control and/or who are unwilling or unable to be tested for pregnancy. Women of childbearing potential must have a confirmed negative pregnancy test at screening and randomization visits. They must use an effective contraceptive method throughout the study, and agree to repeat urine pregnancy test at designated visits. The applied methods of contraception have to meet the criteria for a highly effective method of birth control according to the note for guidance on non-clinical safety studies for the conduct of human clinical trials and marketing authorization for pharmaceuticals. Postmenopausal women must be amenorrheic for at least 12 months.

### COMBO II

The inclusion criteria for the COMBO II study were as follows. Patients with hypercholesterolemia and established CHD or CHD risk equivalents (see below for definitions) who are not adequately controlled with a maximally tolerated stable daily dose of statin for at least 4 weeks prior to the screening visit.

Definition of maximally tolerated dose (any of the following are acceptable): rosuvastatin 20 mg or 40 mg daily, atorvastatin 40 mg or 80 mg daily, and simvastatin 80 mg daily (if already on this dose for >1 year - see exclusion criterion). Patients not able to be on any of the above statin doses should be treated with the dose of daily atorvastatin, rosuvastatin, or simvastatin which is considered appropriate for the patient as per the investigator's judgment or concerns. Some examples of acceptable reasons for a patient taking a lower statin dose include: adverse effects on higher doses, advanced age, low BMI, regional practices, local prescribing information, concomitant medications, and comorbid conditions such as impaired glucose tolerance/impaired fasting glucose.

Established CHD includes one or more of the following: acute MI; silent MI; unstable angina; coronary revascularization procedure (e.g. PCI or CABG); clinically significant CHD diagnosed by invasive or non-invasive testing (such as coronary angiography, stress test using treadmill, stress echocardiography, or nuclear imaging).

CHD risk equivalents includes one or more of the following 4 criteria: 1) documented PAD (one of the following criteria [a, b, or c] must be satisfied): a) current intermittent claudication of resumed atherosclerotic origin together with ankle-brachial index equal to or less than 0.90 in either leg at rest, or b) history of intermittent claudication together with endovascular procedure or surgical intervention in one or both legs because of atherosclerotic disease, or c) history of critical limb ischemia together with thrombolysis, endovascular procedure or surgical intervention in one or both legs because of atherosclerotic disease; 2) documented previous ischemic stroke with a focal ischemic neurological deficit that persisted more than 24 hours, considered as being of atherothrombotic origin. CT or MRI must have been performed to rule out hemorrhage and non-ischemic neurological disease; 3) documented CKD as defined by eGFR ≥30- <60 ml/min/1.73 m2 for 3 months or more, including the screening visit; and/or 4) known history of diabetes mellitus and 2 or more additional risk factors: a) history of hypertension (established on antihypertensive medication), b) documented history of ankle-brachial index ≤0.90, c) documented history of microalbuminuria or macroalbuminuria or dipstick urinalysis at screening visit (Week -3) with >2+ protein, d) documented history of preproliferative or proliferative retinopathy or laser treatment for retinopathy, e) known family history of premature CHD (CHD in father or brother before 55 years of age; CHD in mother or sister before 65 years of age).

Patients who met all the above inclusion criteria were screened for the following exclusion criteria.

Exclusion criteria related to study methodology were as follows. Patients without established CHD or CHD risk equivalents; LDL-C <70 mg/dl (<1.81 mmol/l) at the screening visit in patients with an history of documented CVD; LDL-C <100 mg/dl (<2.59 mmol/l) at the screening visit in patients without history of documented CVD; change in statin dose or dose regimen from screening to randomization; currently taking a statin that is not simvastatin, atorvastatin, or rosuvastatin; simvastatin, atorvastatin, or rosuvastatin is not taken daily or not taken at a registered dose; daily doses above atorvastatin 80 mg, rosuvastatin 40 mg, or simvastatin 40 mg (except for patients on simvastatin 80 mg for >1 year, who are eligible); use of cholesterol absorption inhibitor (i.e., ezetimibe), omega-3 fatty acid (at doses ≥1000 mg daily), nicotinic acid, bile acid-binding sequestrant, or red yeast rice products within the past 4 weeks prior to screening visit or between screening and randomization visits; use of fibrates in the past 6 weeks prior to screening visit; use of nutraceutical products or over-the-counter therapies that may affect lipids which have not been at a stable dose/amount for at least 4 weeks prior to the screening visit or between screening and randomization visits; patients who have received plasmapheresis treatment within 2 months prior to the screening visit, or have plans to receive it during the study; recent (within 3 months prior to the screening visit or between screening and randomization visits) MI, unstable angina leading to hospitalization, PCI, CABG, uncontrolled cardiac arrhythmia, stroke, transient ischemic attack, carotid revascularization, endovascular procedure or surgical intervention for peripheral vascular disease; planned to undergo scheduled PCI, CABG, carotid or peripheral revascularization during the study; systolic BP >160 mmHg or diastolic BP >100 mmHg at screening visit or randomization visit; history of NYHA Class III or IV heart failure within the past 12 months; known history of hemorrhagic stroke; age <18 years or legal age of majority at the screening visit whichever is greater; patients not previously instructed on a cholesterol-lowering diet prior to the screening visit; newly diagnosed (within 3 calendar months prior to randomization visit) or poorly controlled (HbA1c >9% at the screening visit) diabetes; presence of any clinically significant uncontrolled endocrine disease known to influence serum lipids or lipoproteins (note: patients on thyroid replacement therapy can be included if the dosage has been stable for at least 12 weeks prior to screening and between screening and randomization visits, and TSH level is within the normal range of the Central Laboratory at the screening visit); history of bariatric surgery within 12 months prior to the screening visit; unstable weight defined by a variation >5 kg within 2 months prior to the screening visit; known history of homozygous or heterozygous FH; known history of loss of function of PCSK9 (i.e., genetic mutation or sequence variation); use of systemic corticosteroids, unless used as replacement therapy for pituitary/adrenal disease with a stable regimen for at least 6 weeks prior to randomization visit (note: topical, intra-articular, nasal, inhaled, and ophthalmic steroid therapies are not considered as 'systemic' and are allowed); use of continuous estrogen or testosterone hormone replacement therapy unless the regimen has been stable in the past 6 weeks prior to the screening visit and there are no plans to change the regimen during the study; history of cancer within the past 5 years, except for adequately treated basal cell skin cancer, squamous cell skin cancer, or in situ cervical cancer; known history of a positive HIV test; patients who have taken any investigational drugs other than the alirocumab training placebo kits within 1 month or 5 half-lives, whichever is longer; patients who have been previously treated with at least 1 dose of alirocumab or any other anti-PCSK9 monoclonal antibody in other clinical trials; patients who withdraw consent during the screening period (patients who are not willing to continue or fail to return).

Additional exclusion criteria were conditions/situations such as any clinically significant abnormality identified at the time of screening that in the judgment of the investigator or any sub-investigator would preclude safe completion of the study or constrain endpoint assessment such as major systemic diseases; patients with short life expectancy; considered by the investigator or any sub-investigator as inappropriate for this study for any reason, e.g. deemed unable to meet specific protocol requirements such as scheduled visits; deemed unable to administer or tolerate long-term injections as per the patient or the investigator; investigator or any sub-investigator, pharmacist, study coordinator, other study staff or relative thereof directly involved in the conduct of the protocol, etc.; presence of any other conditions (e.g., geographic, social) actual or anticipated, that the investigator feels would restrict or limit the patient's participation for the duration of the study.

Laboratory findings during the screening period (not including randomization assessments): positive test for Hepatitis B surface antigen or Hepatitis C antibody; positive serum beta-hCG or urine pregnancy test (including Week 0) in women of childbearing potential; triglycerides >400 mg/dl (>4.52 mmol/l) (1 repeat assessment is allowed); eGFR <30 ml/min/1.73 m2; ALT or AST >3 x ULN (1 repeat assessment is allowed); CPK >3 x ULN (one repeat assessment is allowed); TSH <LLN or >ULN (one repeat assessment is allowed).

Exclusion criteria related to the active comparator and mandatory background therapy were as follows. All contraindications to ezetimibe or warnings/precautions of use (when appropriate) as displayed in the respective National Product Labeling; all contraindications to the background statins or warning/precaution of use (when appropriate) as displayed in the respective National Product Labeling.

Exclusion criteria related to alirocumab were as follows. Known hypersensitivity to monoclonal antibody or any component of the drug product; pregnant or breast-feeding women; women of childbearing potential not protected by highly effective method(s) of birth control and/or who are unwilling or unable to be tested for pregnancy. Women of childbearing potential must have a confirmed negative pregnancy test at screening and randomization visits. They must use an effective contraceptive method throughout the entire duration of the study treatment, and for 10 weeks after the last intake of study medication (injection or capsule, whichever comes last), and agree to repeat urine pregnancy test at designated visits. (The applied methods of contraception have to meet the criteria for a highly effective method of birth control according to the "Note for guidance on non-clinical safety studies for the conduct of human clinical trials and marketing authorization for pharmaceuticals. Postmenopausal women must be amenorrheic for at least 12 months).

### STUDY PROCEDURES

Patients meeting the inclusion criteria entered a screening period of up to 2 (COMBO I) or 3 (COMBO II) weeks prior to randomization. During screening, patients completed informed consent,inclusion/exclusion criteria were further assessed, patient information was collected and patients were trained in the use of the auto-injector device. In addition, vital signs were taken, a 12-lead electrocardiogram was performed, and fasting blood and urine samples were obtained for analysis. AEs were assessed from the screening visit throughout the study.

### COMBO I

Eligible patients were randomized (2:1 alirocumab:placebo), with stratification by 1) prior history of myocardial infarction (MI) or ischemic stroke, and 2) intensity of statin treatment to ensure balance between arms for these factors. After randomization, patients entered a double-blind treatment period of 52 weeks. In addition to existing statin and other existing lipid lowering therapies if appropriate, patients randomized to alirocumab received a 75-mg subcutaneous (SC) dose every 2 weeks (Q2W), administered as a single 1 ml injection utilizing an auto-injector, from randomization to Week 12. Patients randomized to placebo received a 1 ml SC placebo injection from an identical auto-injector.

At Week 12, patients randomized to alirocumab were up-titrated to 150 mg Q2W if the Week 8 LDL-C was ≥70 mg/dl (1.81 mmol/l). To maintain blinding, the patient and investigator were not informed of the Week 8 LDL-C levels (or any lipid values after randomization); continuation or up-titration of dose occurred in an automated and blinded manner. The 150 mg Q2W dose of alirocumab was also administered as a 1 ml solution in an auto-injector.

On-site patient assessments during the treatment period were scheduled at randomization and then Weeks 4, 8, 12, 16, 24, 36, and 52 (end of treatment visit) (**Figure 1A**). After the treatment period, there was an 8-week follow-up period.

### COMBO II

Eligible patients were randomized (2:1 alirocumab:ezetimibe), with stratification for 1) prior history of MI or ischemic stroke, 2) intensity of statin treatment and 3) geographic region, to ensure balance between arms in these factors. After randomization, patients entered a double-blind, double-dummy treatment period of 104 weeks. Patients were randomized to either alirocumab 75 mg SC Q2W plus placebo for ezetimibe per os (PO) daily or placebo for alirocumab SC Q2W plus ezetimibe 10 mg PO daily. At Week 12, patients randomized to alirocumab were up-titrated to 150 mg Q2W if the Week 8 LDL-C was ≥70 mg/dl (1.81 mmol/l).

On-site patient assessments were scheduled at regular intervals from randomization to Week 104 (end of treatment visit) (**Figure 1B**). After the treatment period, there was an 8-week follow-up period.

### COMBO I and II

In both studies, patients were asked to remain on a stable diet (National Cholesterol Education Program Adult Treatment Panel III therapeutic lifestyle changes diet or equivalent) and the daily statin dose should be stable throughout the entire study duration from screening to the follow-up visit. Modification to the statin (and in the case of COMBO I, other background lipid lowering therapy) was only allowed under special circumstances.

### SAFETY ASSESSMENTS

Safety assessments during both studies were as follows. Safety was assessed through adverse event (AE) reporting (including adjudicated cardiovascular events), laboratory analyses, and vital signs measurement. Since the LDL-C-lowering effects of PCSK9 inhibition on top of a statin are unknown, a number of AEs were defined as being of special interest and were monitored, including alanine aminotransferase abnormalities, allergic events, hemolytic anemia, pregnancy, overdose with study drug, neurologic events, ophthalmic events, and local injection site reactions. AEs were determined to be of special interest based on review of previous clinical trials, correspondence with regulatory authorities and the Data Monitoring Committee, the use of a monoclonal antibody in this study, and the route of drug administration.

Safety Analyses were as follows. AEs (including adjudicated cardiovascular events), laboratory parameters, and vital signs were reported descriptively, based on the safety population (all randomized patients who received at least 1 dose or partial dose of study treatment). The safety analysis focused on the treatment-emergent AE period defined as the time from the first double-blind dose to the last double-blind dose of the investigational product + 70 days (10 weeks).

Safety: Occurrence of treatment emergent adverse events (TEAEs) reported by the patient or noted by the investigator, serious adverse events (SAEs), TEAEs leading to treatment discontinuation, AEs of special interest (local Injection site reactions, allergic events, selected neurological events and cardiovascular events with adjudication result), occurrence of PCSA (potentially clinically significant abnormalities) in laboratory parameters, specific analysis for diabetes or impaired glucose control and patients with 2 consecutives LDL-C < 25 mg/dL (O.65 mmol/L).

### COMBO I: STATISTICAL METHODS

### Sample size determination

The final total sample size was 306, with a randomization ratio of 2:1 (alirocumab 204: placebo 102).

### Combo I: Analysis populations

The primary efficacy analysis population was the intent-to-treat (ITT) population, defined as all randomized patients who had an evaluable primary endpoint, that is, those with an available baseline calculated LDL-C value, and at least one available calculated LDL-C value within one of the analysis windows up to Week 24 (including all calculated LDL-C on-treatment and off-treatment).

The secondary efficacy analysis population was the modified intent-to-treat (mITT) population, defined as all randomized patients who took at least one dose or part of a dose of the double-blind investigational medicinal product (IMP) and who had an available calculated LDL-C value at baseline and at least one within one of the analysis windows up to Week 24 during the efficacy treatment period. The efficacy treatment period was defined as the time from the first double-blind IMP administration up to 21 days after the last double-blind injection.

The safety population included all randomized patients who received at least one dose or part of a dose of the double-blind IMP.

### Combo I: Efficacy analyses

Primary analyses of efficacy endpoints were conducted using an ITT approach (based on the ITT population defined above), including all lipid data, regardless of whether the patient was continuing therapy or not. This corresponds to ITT estimands, defined for primary and key secondary endpoints. In addition, analyses were also conducted using an on-treatment approach (based on the mITT population defined above), including lipid data collected during the efficacy treatment period. This corresponds to on-treatment estimands of key secondary endpoints.

The ITT approach analyzed all patients, irrespective of their adherence to the treatment; it assessed the benefit of the treatment strategy and reflected as much as possible the effect in a population of patients. The on-treatment approach analyzed the effect of treatment, restricted to the period during which patients actually received the treatment. It assessed the benefit that a treatment would achieve in patients adherent to treatment up to the considered time point.

Efficacy analyses were performed according to treatment as-randomized.

All measurements, scheduled or unscheduled, fasting or not fasting, were assigned to analysis windows in order to provide an assessment for Week 4 to Week 52 time points.

With regards to the primary efficacy analysis (ITT approach), the percent change in calculated LDL-C from baseline to Week 24 was analyzed using a mixed-effect model with repeated measures (MMRM) approach. All post-baseline data available from Week 4 to Week 52 analysis windows were used and missing data were accounted for by the MMRM. The model included the fixed categorical effects of treatment group (placebo versus alirocumab), randomization strata (as per IVRS), time point (Week 4 to Week 52), treatment-by-time point interaction and strata-by-time point interaction, as well as, the continuous fixed covariates of baseline LDL-C value and baseline value-by-time- point interaction. This model provided baseline adjusted least-squares means (LSmeans) estimates at Week 24 for both treatment groups with their corresponding 95% confidence interval. To compare the alirocumab to the placebo group, an appropriate statement was used to test the differences of these estimates at the 5% alpha level.

A hierarchical procedure has been defined to test key secondary endpoints while controlling for multiplicity (using above order of key secondary endpoints). The first key secondary endpoint was the percent change in calculated LDL-C from baseline to Week 24 using an on-treatment approach.

Continuous secondary variables anticipated to have a normal distribution (i.e., lipids other than TG and Lp(a)) were analyzed using the same MMRM model as for the primary endpoint. Continuous endpoints anticipated to have a non-normal distribution (i.e., TG and Lp(a)) were analyzed using multiple imputation approach for handling of missing values followed by robust regression model with endpoint of interest as response variable using M-estimation (using SAS ROBUSTREG procedure) with treatment group, randomization strata (as per IVRS) and corresponding baseline value(s) as effects to compare treatment effects. Combined estimate for mean in both treatment groups, as well as the differences of these estimates, with their corresponding SEs, 95% CIs and p-value were provided (through SAS MIANALYZE procedure).

Binary secondary efficacy endpoints were analyzed using multiple imputation approach for handling of missing values followed by stratified logistic regression with treatment group as main effect and corresponding baseline value(s) as covariate, stratified by randomization factors (as per IVRS). Combined estimates of odds ratio versus placebo, 95% CI, and p-value were provided (through SAS MIANALYZE procedure).

### Combo I: Safety analyses

Safety analyses were descriptive, performed on the safety population according to treatment actually received. For analysis of TEAE and PCSA, the safety analysis focused on the TEAE period defined as the time from the first dose of double-blind IMP up to 70 days after the last double-blind injection. Analyses of laboratory data and vital signs overtime were performed on the period up to 21 days after the last IMP injection.

### COMBO I: RESULTS

### Patient Accountability

Of the 316 randomized patients, two patients in the alirocumab group were not treated, and therefore were not included in the safety population. Five randomized patients were not included in the ITT population (no LDL-C value within one analysis window through Week 24). Seven randomized patients were excluded from the mITT population (patients excluded from the ITT population and patients with no LDL-C value within one of the analysis window up to Week 24 during the efficacy treatment period).

**Table 1 - Analysis populations**

| | **Placebo** | **Alirocumab 75 Q2W/Up150 Q2W** | **All** |
|---|---|---|---|
| Randomized population | 107 (100%) | 209 (100%) | 316 (100%) |
| Efficacy populations | | | |
| Intent-to-Treat (ITT) | 106 (99.1%) | 205 (98.1%) | 311 (98.4%) |
| Modified Intent-to-Treat (mITT) | 105 (98.1%) | 204 (97.6%) | 309 (97.8%) |
| Safety population | 107 | 207 | 314 |

| | | | |
|---|---|---|---|
| Note: The safety population patients are tabulated according to treatment actually received (as treated). For the other populations, patients are tabulated according to their randomized treatment. | | | |

In the alirocumab group, among the 191 patients who received at least one injection after Week 12, 32 (16.8%) patients received automatic up-titration at Week 12 from alirocumab 75 mg Q2W to 150 mg Q2W in a blinded manner.

### COMBO I: Study disposition

Of 640 patients screened for eligibility, 316 were subsequently randomized to alirocumab (209) or placebo (107). Of the 316 randomized patients, 2 (alirocumab) were not treated; 231 patients (73.1%) completed the 52 week study treatment (75 placebo; 156 alirocumab); 311 (98.4%) comprised the ITT population (106 placebo; 205 alirocumab, 5 patients with missing LDL-C levels); 309 patients comprised the on-treatment population (105 placebo; 204 alirocumab) and 314 patients comprised the safety population (107 placebo; 207 alirocumab).

Over the 52-week double-blind period, adherence to study medication (receipt of ≥80% scheduled injections) was similar between treatment groups (98% alirocumab; 99% placebo). Based on week 8 LDL-C levels ≥70 mg/dL, 32/191 patients (16.8%) had their alirocumab dose increased to 150 mg subcutaneously every 2 weeks at week 12 (among patients with at least 1 injection after week 12).

The double-blind IMP was prematurely discontinued before Week 52 for 32 (29.9%) randomized patients in the placebo group and 51 (24.4%) randomized patients in the alirocumab group. The main reasons for study treatment discontinuation were "other reasons" and adverse events. These "other reasons" included the following: patient did not meet criteria for completion of treatment due to either missed / out of window Week 50 injection or out of window Week 52 visit (17 patients), subject withdrawal was not otherwise specified (8 patients), site closure (4 patients), sudden death / death (3 patients) (there was no decision of investigator to stop the treatment prior death), miscellaneous (2 patients).

### COMBO I: Summary of Population Characteristics

Demographics characteristics, disease characteristics and lipid parameters at baseline were similar in the alirocumab group as compared to the placebo group. Baseline demographics characteristics are set forth in **Table 2.** Baseline lipid medication and lipid levels are set forth in **Table 3.** The definition of CHD included acute myocardial infarction (MI), silent MI, unstable angina, coronary revascularization, or clinically significant CHD diagnosed by invasive or non-invasive testing. CHD risk equivalents included peripheral arterial disease, ischemic stroke, chronic kidney disease (estimated glomerular filtration rate ≥30 to <60 mL/min/1.73 m² for ≥3 months), or diabetes mellitus in combination with 2 or more additional risk factors (hypertension, ankle-brachial index ≤0.90, micro- or macroalbuminuria, dipstick urinalysis with >2+ protein, retinopathy, or family history of premature CHD [<55 years in father/brother or <65 years in mother/sister]).

There were numerically higher proportions of female patients and patients with type 2 diabetes in the alirocumab group. Similarly, a higher percentage of patients in the placebo group were using other LLTs in addition to statin (49.5% versus 38.3% in the alirocumab group). However, these differences between the treatment groups were not found to be statistically significant.

All patients but one were treated with a statin, 57.6% receiving high intensity statin (atorvastatin 40 to 80 mg daily or rosuvastatin 20 to 40 mg daily) and 8.2% receiving ezetimibe in addition to the statin. Mean (SD) calculated LDL-C at baseline was 102.2 (31.6) mg/dL (2.646 (0.820) mmol/L). Approximately 78% of the randomized population had a coronary heart disease (CHD) history (**Table 5**).

Exposure to injections was similar across treatment groups with a mean exposure of 46 weeks. In the alirocumab group, among the 191 patients who received at least one injection after Week 12, 32 (16.8%) patients received automatic up-titration at Week 12 from alirocumab 75 mg Q2W to 150 mg Q2W in a blinded manner.

**Table 2 - Baseline demographic characteristics**

| Characteristic | Alirocumab (N=209) | Placebo (N=107) | P-value vs placebot |
|---|---|---|---|
| Age, years, mean (SD) | 63.0 (9.5) | 63.0 (8.8) | 0.77 |
| Male, % (n) | 62.7% (131) | 72.0% (77) | 0.11 |
| Race | | | 0.61 |
| White, % (n) | 81.3% (170) | 82.2% (88) | |
| Black or African-American | 16.3% (34) | 15.9% (17) | |
| Ethnicity | | | 0.44 |
| Hispanic/Latino | 12.0% (25) | 8.4% (9) | |
| BMI, kg/m2, mean (SD) | 32.6 (6.3) | 32.0 (7.0) | 0.23 |
| CHD history, % (n) | 78.5% (164) | 77.6% (83) | 0.97 |
| Hypertension, % (n) | 88.5% (185) | 88.8% (95) | 0.91 |
| Type 2 diabetes, % (n) | 45.0% (94) | 39.3% (42) | 0.39 |

| | | | |
|---|---|---|---|
| All patients were on background maximally tolerated statin ±other LLT. ^{†}*P*-values comparing baseline data between treatment groups are provided for descriptive purpose, as a screening tool, using Fisher exact test for qualitative data and the asymptotic one-way ANOVA test for Wilcoxon scores (Kruskal-Wallis test) for continuous data. | | | |

**Table 3 - Baseline lipid medication and lipid levels**

| All patients on background maximally tolerated statin ± other LLT | Alirocumab (N=209) | Placebo (N=107) | P-value vs placebot |
|---|---|---|---|
| Any statin^{†},% (n) | 99.5% (208) | 100% (107) | 1.00 |
| High dose statin at screening^{††}, % (n) | 61.7% (129) | 64.5% (69) | 0.71 |
| Other LLT^{‡}, % (n) | 38.3% (80) | 49.5% (53) | 0.07 |
| Ezetimibe, % (n) | 7.2% (15) | 10.3% (11) | 0.46 |
| LDL-C, calculated | 100.2 (29.5) | 106.0 (35.3) | 0.42 |
| Median (IQR) | 98.0 (81.0:114.0) | 97.0 (86.0:120.0) | |
| LDL-C, measured‡‡ | 94.8 (29.3) | 100.2 (34.4) | 0.41 |
| Non-HDL-C | 130.0 (34.0) | 133.4 (39.8) | 0.72 |
| Apo B | 90.8(21.4) | 91.4 (24.1) | 0.98 |
| Lp(a), median (IQR) | 31.0 (8:81) | 38.0 (10:70) | 0.70 |
| Fasting TGs, median (IQR) | 130.0 (92:189) | 123.0 (95:177) | 0.57 |
| HDL-C | 48.3(14.4) | 48.8 (12.7) | 0.46 |

**Table 4 - Disease characteristics and other relevant baseline data - Randomized population**

| | **Placebo (N=107)** | **Alirocumab 75 Q2W/Up150 Q2W (N=209)** | **All (N=316)** |
|---|---|---|---|
| Time from hypercholesterolemia diagnosis (years) | | | |
| Number | 107 | 209 | 316 |
| Mean (SD) | 10.60 (7.98) | 11.20 (8.01) | 10.99 (7.99) |
| Median | 9.70 | 10.05 | 9.84 |
| Min: Max | 0.2 : 42.4 | 0.4 : 46.1 | 0.2 : 46.1 |
| | | | |

| Fredrickson classification of Hyperlipoproteinemia | | | |
|---|---|---|---|
| Number | 99 | 188 | 287 |
| IIa | 42 (39.3%) | 83 (39.7%) | 125 (39.6%) |
| IIb | 56 (52.3%) | 96 (45.9%) | 152 (48.1%) |
| IV | 1 (0.9%) | 9 (4.3%) | 10 (3.2%) |

There were 4 patients without fully documented CHD or CHD risk equivalents.

**Table 5 - Medical history of specific interest: cardiovascular history and risk factors - Randomized population**

| | **Placebo (N=107)** | **Alirocumab 75 Q2W/Up150 Q2W (N=209)** | **All (N=316)** |
|---|---|---|---|
| Any cardiovascular history/risk factors | 106 (99.1%) | 206 (98.6%) | 312 (98.7%) |
| | | | |
| Coronary heart disease^{a} | 83 (77.6%) | 164 (78.5%) | 247 (78.2%) |
| Acute Myocardial Infarction | 47 (43.9%) | 83 (39.7%) | 130 (41.1%) |
| Silent Myocardial Infarction | 2 (1.9%) | 12 (5.7%) | 14 (4.4%) |
| Unstable Angina | 20 (18.7%) | 34 (16.3%) | 54(17.1%) |
| Coronary Revascularization Procedures | 60 (56.1 %) | 133 (63.6%) | 193 (61.1%) |
| Other Clinically Significant CHD^{b} | 14 (13.1%) | 38 (18.2%) | 52 (16.5%) |
| CHD associated with 1 or more comorbidity (among hypertension, diabetes or moderate CKD) and/or associated with other CVD (ischemic stroke, PAD) | 76 (71.0%) | 150 (71.8%) | 226 (71.5%) |
| | | | |
| Coronary heart disease risk equivalents^{a} | 51 (47.7%) | 85 (40.7%) | 136 (43.0%) |
| Ischemic Stroke | 6 (5.6%) | 21 (10.0%) | 27 (8.5%) |
| Peripheral Arterial Disease | 8 (7.5%) | 3 (1.4%) | 11 (3.5%) |
| Moderate Chronic Kidney Disease | 24 (22.4%) | 37 (17.7%) | 61 (19.3%) |
| Known history of diabetes mellitus AND 2 or more additional risk factors | 25 (23.4%) | 42 (20.1 %) | 67 (21.2%) |
| At least 2 CHD risk equivalents or 1 CHD risk equivalent associated with hypertension or diabetes | 50 (46.7%) | 82 (39.2%) | 132 (41.8%) |

| | | | |
|---|---|---|---|
| Note: A patient can be counted in several categories. ^{a} according to the items pre-listed in the e-CRF. ^{b} diagnosed by invasive or non-invasive testing. PAD defined as Intermittent claudication TOGETHER WITH ankle-brachial index ≤ 0.90 OR TOGETHER WITH peripheral revascularization procedure/surgery OR critical limb ischemia TOGETHER WITH peripheral revascularization procedure/surgery or thrombolysis. | | | |

**Table 6 - Medical history of specific interest: other medical history of special interest - Randomized population**

| | **Placebo (N=107)** | **Alirocumab 75 Q2W/Up150 Q2W (N=209)** | **All (N=316)** |
|---|---|---|---|
| Other medical history of special interest | 102 (95.3%) | 202 (96.7%) | 304 (96.2%) |
| Hypertension ^{a} | 95 (88.8%) | 185 (88.5%) | 280 (88.6%) |
| Type 1 Diabetes ^{a} | 0 | 0 | 0 |
| Type 2 Diabetes ^{a} | 42 (39.3%) | 94 (45.0%) | 136 (43.0%) |
| Family history of premature coronary heart disease ^{a} | 37 (34.6%) | 72 (34.4%) | 109 (34.5%) |
| Family history of type 2 diabetes ^{a} | 31 (29.0%) | 60 (28.7%) | 91 (28.8%) |
| Menopause (females) ^{a} | 23 (21.5%) | 67 (32.1 %) | 90 (28.5%) |

| | | | |
|---|---|---|---|
| ^{a} according to the items pre-listed in the e-CRF. Note: A patient can be counted in several categories. | | | |

**Table 7 - Background LMT at randomization - Randomized population**

| | **Placebo (N=107)** | **Alirocumab 75 Q2W/Up150 Q2W (N=209)** | **All (N=316)** |
|---|---|---|---|
| Any statin | 107 (100%) | 208 (99.5%) | 315 (99.7%) |
| | | | |
| Taking high intensity statin | 60 (56.1%) | 122 (58.4%) | 182 (57.6%) |
| Atorvastatin daily dose (mg) | 34 (31.8%) | 70 (33.5%) | 104 (32.9%) |
| 10 | 0 | 2 (1.0%) | 2 (0.6%) |
| 20 | 5 (4.7%) | 4 (1.9%) | 9 (2.8%) |
| 40 | 17 (15.9%) | 40 (19.1%) | 57 (18.0%) |
| 80 | 12(11.2%) | 24 (11.5%) | 36 (11.4%) |
| Other doses | 0 | 0 | 0 |
| Rosuvastatin daily dose (mg) | 34 (31.8%) | 66 (31.6%) | 100 (31.6%) |
| 5 | 1 (0.9%) | 3 (1.4%) | 4 (1.3%) |
| 10 | 1 (0.9%) | 5 (2.4%) | 6 (1.9%) |
| 20 | 16 (15.0%) | 33 (15.8%) | 49 (15.5%) |
| 40 | 15 (14.0%) | 25 (12.0%) | 40 (12.7%) |
| Other doses | 1 (0.9%) | 0 | 1 (0.3%) |
| Simvastatin daily dose (mg) | 39 (36.4%) | 71 (34.0%) | 110 (34.8%) |
| 10 | 0 | 1 (0.5%) | 1 (0.3%) |
| 20 | 9 (8.4%) | 16 (7.7%) | 25 (7.9%) |
| 40 | 21 (19.6%) | 45 (21.5%) | 66 (20.9%) |
| 80 | 9 (8.4%) | 8 (3.8%) | 17 (5.4%) |
| Other doses | 0 | 1 (0.5%) | 1 (0.3%) |
| | | | |
| Any LMT other than statins^{a} | 53 (49.5%) | 80 (38.3%) | 133 (42.1%) |
| Any LMT other than nutraceuticals | 46 (43.0%) | 68 (32.5%) | 114 (36.1%) |
| Ezetimibe | 11 (10.3%) | 15 (7.2%) | 26 (8.2%) |
| Nutraceuticals | 9 (8.4%) | 15 (7.2%) | 24 (7.6%) |

| | | | |
|---|---|---|---|
| ^{a} in combination with statins or not. High intensity statin corresponds to atorvastatin 40 to 80 mg daily or rosuvastatin 20 to 40 mg daily. | | | |

**Table 8 - Lipid efficacy parameters at baseline - Quantitative summary in conventional units - Randomized population**

| | **Placebo (N=107)** | **Alirocumab 75 Q2W/Up150 Q2W (N=209)** | **All (N=316)** |
|---|---|---|---|
| Calculated LDL-C (mg/dL) | | | |
| Number | 107 | 209 | 316 |
| Mean (SD) | 106.0 (35.3) | 100.2 (29.5) | 102.2 (31.6) |
| Median | 97.0 | 98.0 | 97.0 |
| Q1 : Q3 | 86.0: 120.0 | 81.0: 114.0 | 82.0: 115.0 |
| Min : Max | 61 : 256 | 33 : 240 | 33 : 256 |
| | | | |

| Measured LDL-C (mg/dL) | | | |
|---|---|---|---|
| Number | 70 | 138 | 208 |
| Mean (SD) | 100.2 (34.4) | 94.8 (29.3) | 96.6 (31.2) |
| Median | 92.0 | 91.0 | 91.5 |
| Q1 : Q3 | 75.0 : 119.0 | 76.0 : 109.0 | 75.5 : 110.0 |
| Min : Max | 53 : 238 | 28 : 239 | 28 : 239 |
| | | | |

| Non-HDL-C (mg/dL) | | | |
|---|---|---|---|
| Number | 107 | 209 | 316 |
| Mean (SD) | 133.4 (39.8) | 130.0 (34.0) | 131.1 (36.0) |
| Median | 125.0 | 125.0 | 125.0 |
| Q1 : Q3 | 106.0 : 147.0 | 103.0 : 149.0 | 104.5 : 148.5 |
| Min : Max | 79 : 304 | 61 : 270 | 61 : 304 |
| | | | |

| Total-C (mg/dL) | | | |
|---|---|---|---|
| Number | 107 | 209 | 316 |
| Mean (SD) | 182.3 (39.6) | 178.3 (35.7) | 179.7 (37.0) |
| Median | 176.0 | 173.0 | 174.0 |
| Q1 : Q3 | 156.0 : 204.0 | 151.0 : 199.0 | 154.0 : 200.5 |
| Min: Max | 119 : 348 | 119 : 316 | 119 : 348 |

| HDL-C (mg/dL) | | | |
|---|---|---|---|
| Number | 107 | 209 | 316 |
| Mean (SD) | 48.8 (12.7) | 48.3 (14.4) | 48.5 (13.8) |
| Median | 47.0 | 46.0 | 46.0 |
| Q1 : Q3 | 40.0 : 55.0 | 39.0 : 56.0 | 39.0 : 55.0 |
| Min : Max | 26 : 98 | 17 : 109 | 17 : 109 |
| | | | |

| Fasting TGs (mg/dL) | | | |
|---|---|---|---|
| Number | 106 | 209 | 315 |
| Mean (SD) | 140.3 (64.3) | 151.2 (93.2) | 147.5 (84.6) |
| Median | 123.0 | 130.0 | 127.0 |
| Q1 : Q3 | 95.0 : 177.0 | 92.0 : 189.0 | 92.0 : 186.0 |
| Min : Max | 52 : 431 | 35 : 999 | 35 : 999 |
| | | | |

| Lipoprotein-(a) (mg/d L) | | | |
|---|---|---|---|
| Number | 101 | 191 | 292 |
| Mean (SD) | 49.4 (48.3) | 49.6 (51.0) | 49.5 (50.0) |
| Median | 38.0 | 31.0 | 33.0 |
| Q1 : Q3 | 10.0 : 70.0 | 8.0 : 81.0 | 9.5 : 79.5 |
| Min : Max | 2 : 184 | 2 : 248 | 2 : 248 |
| | | | |

| Apo-B (mg/dL) | | | |
|---|---|---|---|
| Number | 101 | 191 | 292 |
| Mean (SD) | 91.4 (24.1) | 90.8 (21.4) | 91.0 (22.3) |
| Median | 88.0 | 87.0 | 87.5 |
| Q1 : Q3 | 76.0 : 103.0 | 75.0 : 105.0 | 76.0 : 105.0 |
| Min : Max | 50 : 194 | 51 : 166 | 50 : 194 |
| | | | |

| Apo-A1 (mg/dL) | | | |
|---|---|---|---|
| Number | 101 | 191 | 292 |
| Mean (SD) | 145.1 (23.7) | 143.1 (25.0) | 143.8 (24.5) |
| Median | 141.0 | 142.0 | 141.5 |
| Q1 : Q3 | 129.0 : 161.0 | 125.0 : 157.0 | 128.0 : 158.0 |
| Min : Max | 96 : 217 | 69 : 262 | 69 : 262 |
| | | | |

| Apo-B/Apo-A1 (ratio) | | | |
|---|---|---|---|
| Number | 101 | 191 | 292 |
| Mean (SD) | 0.647 (0.207) | 0.653 (0.192) | 0.651 (0.197) |
| Median | 0.620 | 0.620 | 0.620 |
| Q1 : Q3 | 0.520 : 0.720 | 0.520 : 0.760 | 0.520 : 0.740 |
| Min : Max | 0.30 : 1.52 | 0.30: 1.70 | 0.30 : 1.70 |
| | | | |

| Total-C/HDL-C (ratio) | | | |
|---|---|---|---|
| Number | 107 | 209 | 316 |
| Mean (SD) | 3.934 (1.199) | 3.931 (1.211) | 3.932 (1.205) |
| Median | 3.711 | 3.750 | 3.750 |
| Q1 : Q3 | 3.052 : 4.474 | 3.096 : 4.500 | 3.076 : 4.474 |
| Min: Max | 2.05:7.91 | 1.87 : 10.59 | 1.87 : 10.59 |

The collection of measured LDL-C was not planned in the initial protocol and was added in an amendment. Therefore measured LDL-C values are available for fewer patients compared to calculated LDL-C values.

### COMBO I: Dosage and duration

Exposure to injections was similar across treatment groups with a mean exposure of approximately 46 weeks. In the alirocumab group, among the 191 patients who received at least one injection after Week 12, 32 (16.8%) patients received automatic up-titration at Week 12 from alirocumab 75 mg Q2W to 150 mg Q2W in a blinded manner.

### COMBO I: Primary efficacy endpoint

The ITT analysis includes all LDL-C values collected on-treatment and off-treatment up to Week 52. The primary endpoint (percent change in calculated LDL-C from baseline to Week 24) analysis is provided based on a MMRM model on the ITT population, using LS means estimates at Week 24. Sixteen (7.8%) patients in the alirocumab group and 9 (8.5%) patients in the placebo group did not have a calculated LDL-C value at Week 24. These missing values were accounted for by the MMRM model.

Results of the primary endpoint analysis are presented in **Table 9**, in mmol/L and mg/dL.

A statistically significant decrease in percent change in LDL-C from baseline to Week 24 was observed in the alirocumab group (LS mean versus baseline - 48.2%) compared to the placebo group (LS mean versus baseline -2.3%) (LS mean difference vs. placebo of - 45.9%, p < 0.0001).

In the alirocumab group, LDL-C reduction from baseline was observed from Week 4 to Week 52.

A slight decrease in LDL-C reduction over time was observed in alirocumab group (LS mean versus baseline at Week 52 of -42.5 versus -48.2 at Week 24) probably due to the higher number of treatment discontinuations at Week 52 compared to Week 24 and as a result, higher number of post-treatment LDL-C included at Week 52 (8.8% at Week 52 versus 3.9% at Week 24).

Indeed the treatment effect is stable from Week 24 to Week 52 when only on-treatment LDL-C are considered (see **Figure 3**) whereas a slight decrease is observed when post-treatment LDL-C are also included (see **Figure 2**).

**Table 9 - Percent change from baseline in calculated LDL-C and other lipid parameters at Week 24 (ITT or On-Treatment analyses as indicated)**

| **All patients on maximally tolerated statin ± other LLT** | **Alirocuma b** | **Placebo** | **Alirocumab vs. placebo** | | |
|---|---|---|---|---|---|
| | | | **LS mean difference (SE) %** | **95% CI** | **p-value** |
| **ITT** | n = 205 | n = 106 | | | |
| Baseline, mg/dL (Mean (SD)) | 100.3 (29.7) | 104.6 (32.3) | | | |
| Median | 98.0 | 96.5 | | | |
| Min:Max | 33:240 | 31:243 | | | |
| LS mean (SE) change from baseline at week 24 (%) | -48.2 (1.9) | -2.3 (2.7) | -45.9 (3.3) | -52.5 to -39.3 | < 0.0001 |
| **On-treatment** | n = 204 | n = 105 | | | |
| Baseline, mg/dL | 99.8 (29.0) | 105.0 (32.2) | | | |
| Min:Max | 33:240 | 31:243 | | | |
| LS mean (SE) change from baseline at week 24 (%) | -50.7 (1.9) | -0.8 (2.6) | -49.9 (3.2) | -56.2 to -43.6 | < 0.0001 |

**Table 10 - Calculated LDL-C over time - ITT analysis - ITT population**

| | **Placebo** | | | **Alirocumab 75 Q2W/Up150 Q2W** | | |
|---|---|---|---|---|---|---|
| | **(N=106)** | | | **(N=205)** | | |
| **Calculated LDL-C** | **Value** | **Change from baseline** | **Percent change from baseline** | **Value** | **Change from baseline** | **Percent change from baseline** |
| LS Mean (SE) (mmol/L) | | | | | | |
| Baseline^{a} | 2.709 (0.081) | NA | NA | 2.597 (0.054) | NA | NA |
| Week 4 | 2.524 (0.062) | -0.112 (0.062) | -2.3 (2.4) | 1.327 (0.045) | -1.308 (0.045) | -50.6 (1.7) |
| Week 8 | 2.592 (0.060) | -0.043 (0.060) | -0.4 (2.4) | 1.264 (0.043) | -1.372 (0.043) | -51.6 (1.7) |
| Week 12 | 2.643 (0.071) | 0.007 (0.071) | 1.1 (2.5) | 1.428 (0.051) | -1.207 (0.051) | -46.3 (1.8) |
| Week 16 | 2.595 (0.069) | -0.041 (0.069) | 0.6 (2.6) | 1.291 (0.049) | -1.345 (0.049) | -50.5 (1.9) |
| Week 24 | 2.533 (0.073) | -0.102 (0.073) | -2.3 (2.7) | 1.332 (0.052) | -1.303 (0.052) | -48.2 (1.9) |
| Week 36 | 2.464 (0.078) | -0.172 (0.078) | -5.3 (3.4) | 1.411 (0.057) | -1.225 (0.057) | -44.8 (2.5) |
| Week 52 | 2.586 (0.090) | -0.049 (0.090) | 0.5 (3.6) | 1.483 (0.063) | -1.153 (0.063) | -42.5 (2.5) |
| | | | | | | |

| LS Mean (SE) (mg/dL) | | | | | | |
|---|---|---|---|---|---|---|
| Baseline^{a} | 104.6 (3.1) | NA | NA | 100.3 (2.1) | NA | NA |
| Week 4 | 97.5 (2.4) | -4.3 (2.4) | -2.3 (2.4) | 51.2 (1.7) | -50.5 (1.7) | -50.6 (1.7) |
| Week 8 | 100.1 (2.3) | -1.7 (2.3) | -0.4 (2.4) | 48.8 (1.7) | -53.0 (1.7) | -51.6 (1.7) |
| Week 12 | 102.0 (2.8) | 0.3 (2.8) | 1.1 (2.5) | 55.1 (2.0) | -46.6 (2.0) | -46.3 (1.8) |
| Week 16 | 100.2 (2.7) | -1.6 (2.7) | 0.6 (2.6) | 49.8 (1.9) | -51.9 (1.9) | -50.5 (1.9) |
| Week 24 | 97.8 (2.8) | -3.9 (2.8) | -2.3 (2.7) | 51.4 (2.0) | -50.3 (2.0) | -48.2 (1.9) |
| Week 36 | 95.1 (3.0) | -6.6 (3.0) | -5.3 (3.4) | 54.5 (2.2) | -47.3 (2.2) | -44.8 (2.5) |
| Week 52 | 99.9 (3.5) | -1.9 (3.5) | 0.5 (3.6) | 57.2 (2.4) | -44.5 (2.4) | -42.5 (2.5) |

| | | | | | | |
|---|---|---|---|---|---|---|
| ^{a} Baseline is described using means and standard errors. Note: Least-squares (LS) means, standard errors (SE) and p-value taken from MMRM (mixed-effect model with repeated measures) analysis. The model includes the fixed categorical effects of treatment group, randomization strata as per IVRS, time point, treatment-by-time point interaction, strata-by-time point interaction, as well as the continuous fixed covariates of baseline LDL-C value and baseline LDL-C value-by-time point interaction MMRM model and baseline description run on patients with a baseline value and a post-baseline value in at least one of the analysis windows used in the model. | | | | | | |

### COMBO I: Key secondary efficacy endpoints

**Table 11** summarizes analysis results on key secondary endpoints in the hierarchical order. All key secondary endpoints are statistically significant according to the hierarchical testing procedure up to HDL-C endpoint at Week 24 (ITT estimand) included. Statistical significance was not reached for Fasting TG endpoint at week 24 (ITT estimand).

**Table 11 - Key secondary efficacy endpoints**

| **Endpoint** | **Analysis** | **Results** | **P-value** |
|---|---|---|---|
| Calculated LDL-C - Percent change from baseline to Week 24 | On-treatment | LS mean difference vs. placebo of -49.9% | <0.0001 |
| Calculated LDL-C - Percent change from baseline to Week 12 | ITT | LS mean difference vs. placebo of -47.4% | <0.0001 |
| Calculated LDL-C - Percent change from baseline to Week 12 | On-treatment | LS mean difference vs. placebo of -49.3% | <0.0001 |
| Apo-B - Percent change from baseline to Week 24 | ITT | LS mean difference vs. placebo of -35.8% | <0.0001 |
| Apo-B - Percent change from baseline to Week 24 | On-treatment | LS mean difference vs. placebo of -37.5% | <0.0001 |
| Non-HDL-C - Percent change from baseline to Week 24 | ITT | LS mean difference vs. placebo of -37.5% | <0.0001 |
| Non-HDL-C - Percent change from baseline to Week 24 | On-treatment | LS mean difference vs. placebo of -40.4% | <0.0001 |
| Total-C - Percent change from baseline to Week 24 | ITT | LS mean difference vs. placebo of -25% | <0.0001 |
| Apo-B - Percent change from baseline to Week 12 | ITT | LS mean difference vs. placebo of -38.2% | <0.0001 |
| Non-HDL-C - Percent change from baseline to Week 12 | ITT | LS mean difference vs. placebo of -40.1 % | <0.0001 |
| Total-C - Percent change from baseline to Week 12 | ITT | LS mean difference vs. placebo of -26.4% | <0.0001 |
| Calculated LDL-C - Percent change from baseline to Week 52 | ITT | LS mean difference vs. placebo of-43% | <0.0001 |
| Proportion of patients reaching calculated LDL-C <70 mg/dL (1.81 mmol/L) at Week 24 | ITT | combined estimate for odds-ratio vs. placebo of 38.5 | <0.0001 |
| Proportion of patients reaching calculated LDL-C <70 mg/dL (1.81 mmol/L) at Week 24 | On-treatment | combined estimate for odds-ratio vs. placebo of 50 | <0.0001 |
| Lp(a) - Percent change from baseline to Week 24 | ITT | combined estimate for adjusted mean difference vs. placebo of -14.6% | <0.0001 |
| HDL-C - Percent change from baseline to Week 24 | ITT | LS mean difference vs. placebo of 7.3% | 0.0001 |
| Fasting TGs - Percent change from baseline to Week 24 | ITT | combined estimate for adjusted mean difference vs. placebo of -1.2% | 0.7597 |

The on-treatment analysis of LDL-C percent change from baseline to Week 24 shows consistent results with the ITT analysis with a LS mean difference vs. placebo of -49.9% in the on-treatment analysis versus - 45.9% in the ITT analysis. Indeed, few patients had LDL-C values collected post-treatment (ie more than 21 days after last injection) at Week 24: 5 patients (4.7%) in the placebo group and 8 patients (3.9%) in the alirocumab group.

A statistically significant decrease in percent change in LDL-C from baseline to Week 12 (i.e. before possible up-titration) in the ITT analysis was observed in the alirocumab group (LS mean versus baseline - 46.3%) compared to the placebo group (LS mean versus baseline + 1.1%) (LS mean difference vs. placebo of - 47.4%, p < 0.0001). The results obtained with the on-treatment analysis were similar.

Thirty-nine (19.5%) patients experienced two consecutive calculated LDL-C values <25 mg/dL. No particular safety concern has been observed in these 39 patients.

**Table 12 - Number (%) of patients with 2 consecutive LDL-C < 25mg/dL (<0.65 mmol/L) during the treatment period- Safety population**

| | **Placebo** | **Alirocumab 75 Q2W/Up150 Q2W** |
|---|---|---|
| | **(N=107)** | **(N=207)** |
| Patients with 2 consecutive calculated LDL-C value <25 mg/dL¹ | 0/104 | 39/200 (19.5%) |
| | | |
| Time to the first calculated LDL-C value < 25 mg/dL (weeks) ² | | |
| Number | 0 | 39 |
| Mean (SD) | | 11.48 (9.72) |
| Median | | 8.00 |
| Min : Max | | 3.9 : 36.3 |
| | | |
| Patients with 2 consecutive calculated LDL-C value <15 mg/dL¹ | 0/104 | 9/200 (4.5%) |
| | | |
| Time to the first calculated LDL-C value < 15 mg/dL (weeks) ² | | |
| Number | 0 | 9 |
| Mean (SD) | | 12.51 (6.83) |
| Median | | 13.57 |
| Min : Max | | 4.1 : 23.9 |

| | | |
|---|---|---|
| The number (n) represents the subset of the total number of patients who met the criteria The denominator (/N) within a treatment group is the number of patients for the treatment group who had at least two calculated LDL-C values assessed at least 21 days apart in the efficacy period ¹ 2 consecutive values are considered if spaced out by at least 21 days ² First calculated LDL-C value <25 or <15 mg/dL among the first 2 consecutive calculated LDL-C values <25 or <15 mg/dL per patient | | |

### COMBO I: Summary Efficacy Results

At Week 24, the percent change from baseline in calculated LDL-C in the ITT population was significantly greater in the alirocumab group (LS mean versus baseline - 48.2%) compared to the placebo group (LS mean versus baseline -2.3%) (LS mean difference vs. placebo of -45.9%, p < 0.0001) (see **Table 9**). The on-treatment analysis of LDL-C percent change from baseline to Week 24 shows consistent results with the ITT analysis with a LS mean difference vs. placebo of -49.9% in the on-treatment analysis (p<0.0001) (see **Table 9**).

At Week 12, before possible up-titration from 75 mg to 150 mg, statistical significance was also reached in the ITT population (LS mean versus baseline -46.3% and + 1.1%, for alirocumab and placebo groups respectively, LS mean difference vs. placebo of -47.4%; p <0.0001) (see **Table 11**).

In the alirocumab group, LDL-C reduction from baseline was observed from Week 4 to Week 52 (see **Table 10**). A slight decrease over time in LDL-C reduction was observed in alirocumab group (LS mean versus baseline at Week 52 of -42.5% versus -48.2% at Week 24) probably due to the higher number of treatment discontinuations at Week 52 compared to Week 24 and as a result, higher number of post-baseline LDL-C included at Week 52 (8.8% at Week 52 versus 3.9% at Week 24). Measured LDL-C results (beta-quantification) were consistent with those for calculated LDL-C. The results of a sensitivity analysis of the primary endpoint, using a pattern-mixture-model were consistent with the ITT analysis. Specifically, in the ITT group the combined estimate for LS mean (SE) change from baseline at week 24 (%) was -44.2 (2.1 for the alirocumab group and -1.5 (3.0) for the placebo group. The LS mean difference (SE) % for alirocumab vs. placebo was -42.7 (3.7) (95% CI of -49.9 to -35.4; p< 0.0001). The sensitivity analysis was conducted to further evaluate the impact of missing data on the primary endpoint. In this analysis, missing calculated LDL cholesterol values during the "on-treatment" period were multiply imputed using a model assuming Missing At Random and missing calculated LDL cholesterol values during the post-treatment period were multiply imputed using random draws from a normal distribution, with mean equal to subject's own baseline value.

**Figure 4** shows LDL-C levels over time for alirocumab-treated patients with/without dose increase at week 12. In patients with dose increase, LDL-C was reduced by an additional mean 22.8% (SD 27.1) at week 24 compared with week 12. Among the 32 patients (16.8%) with alirocumab dose increase at week 12 (based on LDL-C at week 8 ≥70 mg/dL), achieved LDL-C was comparable at weeks 24, 36 and 52 to that observed among patients in whom no dose increment was performed (week 8 LDL-C <70 mg/dL).

The relative proportions of patients achieving LDL-C <70 mg/dL (<1.81 mmol/L) at week 24 among the ITT (75.0%) and on-treatment (77.5%) populations were significantly greater with alirocumab than placebo (9.0% and 8.0% respectively, p < 0.0001).

All key secondary endpoints including Apo B, non-HDL-C, Total-C, Lp(a), HDL-C at various time points were statistically significant according to the hierarchical testing procedure except for Fasting TG endpoint at week 24 (ITT estimand) (see **Table 11**). Significant reductions from baseline to week 24 following therapy with alirocumab (vs. placebo) were observed in non-HDL-C (-39.1 [1.8] % alirocumab, -1.6 [2.5] % placebo), apolipoprotein B (-36.7 [1.6] % alirocumab, -0.9 [2.3] % placebo), total cholesterol (-27.9 [1.3] % alirocumab, -2.9 [1.8] % placebo) and lipoprotein(a) (-20.5 [2.0] % alirocumab, -5.9 [2.8] % placebo) (all p < 0.0001 alirocumab vs. placebo). No significant change was observed in triglyceride levels while a significant and directionally divergent increase in HDL-C (apolipoprotein A1) was observed the alirocumab group; 3.5 (1.1) % vs. -3.8 (1.5) % for placebo (p < 0.0001; see **Table 9).**

The percentage changes from baseline in LDL-C at week 24 by demographic characteristics and patient subgroups for alirocumab versus placebo are set forth in **Figure 5****.** All of the 95% confidence intervals for differences in percent LDL-C reduction between alirocumab and placebo within each level of the subgroups defined by age, gender, race, ethnicity, intensity of background statin therapy, other LLT therapy (in addition to statin) and history of myocardial infarction or ischemic stroke are completely to the left of the no effect line. The treatment effect of was seen at every level within each subgroup. In addition, tests for interaction by age, gender, race, ethnicity, intensity of background statin therapy did not reach significance at the 5% level and hence demonstrate consistency of treatment effect within these subgroups. However, patients with other LLT therapy (in addition to statin) and those with a history of myocardial infarction or ischemic stroke demonstrated a relatively greater treatment effect (p-value for interaction 0.012 and 0.018 respectively; see **Figure 5**).

Two consecutive calculated LDL-C values <25 mg/dL (< 0.65 mmo/L) were observed in 39 (19.5%) patients. No particular safety concern has been observed in these patients.

### COMBO I: Summary Safety Results

The percentages of patients who experienced treatment emergent adverse event (TEAE), serious TEAE, TEAE leading to death and TEAE leading to treatment discontinuation were similar between treatment groups. Safety data collected until last patient visit at week 52 is set forth in **Table 13.**

Of the 209 patients originally randomized to alirocumab, 2 did not receive treatment, meaning that the safety population comprised 207 patients for alirocumab. All randomized patients in the placebo arm received their allocated treatment, hence the safety population was 107 patients for placebo.

**Table 13 - Adverse Events and Safety Laboratory Values (Safety Population)**

| **No. (%) of patients** | **Alirocumab** | **Placebo** |
|---|---|---|
| All patients on maximally tolerated statin ± other LLT | (n = 207) | (n = 107) |
| TEAEs | 157 (75.8) | 81 (75.7) |
| Treatment-emergent SAEs | 26 (12.6) | 14 (13.1) |
| TEAE leading to death | 2 (1.0) | 3 (2.8) |
| TEAEs leading to discontinuation | 13 (6.3) | 8 (7.5) |

| **TEAEs by preferred term occurring in ≥5% of patients in either group** | | |
|---|---|---|
| Upper respiratory tract infection | 16 (7.7) | 11 (10.3) |
| Arthralgia | 8 (3.9) | 8 (7.5) |
| Nasopharyngitis | 15 (7.2) | 5 (4.7) |
| Urinary tract infection | 13 (6.3) | 4 (3.7) |
| Dizziness | 11 (5.3) | 6 (5.6) |
| Non-cardiac chest pain | 2 (1.0) | 7 (6.5) |
| Sinusitis | 11 (5.3) | 4 (3.7) |
| Injection site reaction | 11 (5.3) | 3 (2.8) |

| **AEs of special interest*** | | |
|---|---|---|
| Local injection site reactions | 11 (5.3) | 3 (2.8) |
| Potential general allergic reaction events | 18 (8.7) | 7 (6.5) |
| Neurological events | 5 (2.4) | 2 (1.9) |
| Neurocognitive disorders | 0 (0) | 1 (0.9) |

| **Cardiovascular TEAEs confirmed by adjudication** | | |
|---|---|---|
| Any patients with treatment emergent cardiovascular events confirmed by adjudication** | - 6 (2.9) | 3 (2.8) |
| Coronary heart disease death (including undetermined cause) | 1 (0.5) | 1 (0.9) |
| Non-fatal myocardial infarction | 1 (0.5) | 1 (0.9) |
| Fatal and non-fatal ischemic stroke (including stroke not otherwise specified) | - 2 (1.0) | 0 |
| Unstable angina requiring hospitalization | 0 | 0 |
| Congestive heart failure requiring hospitalization | 0 | 1 (0.9) |
| Ischemia driven coronary revascularization procedure | 3 (1.4) | 1 (0.9) |

| | | |
|---|---|---|
| *Company MedDRA Queries (CMQ). **One patient in the placebo group and 1 patient in the alirocumab group each experienced 2 events that were positively adjudicated: Non-fatal myocardial infarction and ischemia-driven coronary revascularization procedure. TEAEs are adverse events that developed, worsened, or became serious during the TEAE period (time from first to last injection of study treatment + 70 days). SAEs = serious adverse events; TEAEs = treatment-emergent adverse events; ULN = upper limit of normal. | | |

The most frequently reported and noteworthy SOC with a higher frequency in alirocumab group as compared to placebo group were: "Infections and infestations": 37.2% in the alirocumab group vs 27.1% in the placebo group; "Metabolism and nutrition disorder": 10.1% in the alirocumab group vs 5.6% in the placebo; and "Cardiac disorders": 8.2% in the alirocumab group vs 4.7% in the placebo group, although no striking imbalance was noted for a particular event, it is noteworthy that 9 ischemic events occurred in alirocumab group vs 3 in placebo group. When referring to the results of adjudication, 5 patients experienced cardiovascular events that were positively adjudicated (2.4%) against 2 patients in the placebo group (1.9%).

Among the SOCs with a lower frequency, the SOC "eye disorders" was observed in 4.3% of patients in the alirocumab group vs 0.9% in the placebo group with no imbalance for a particular event.

Two (1.0%) and 3 (2.8%) deaths occurred during treatment period in alirocumab (myocardial infarction and pulmonary embolism) and placebo (esophageal adenocarcinoma, dementia and sudden cardiac death) groups respectively. In addition one post-treatment death (aggravation of coronary artery disease) occurred in alirocumab group.

Adjudicated CV events include all CV AEs positively adjudicated. The adjudication categories are the following: CHD death, non-fatal MI, fatal and non-fatal ischemic stroke, unstable angina requiring hospitalization, congestive heart failure requiring hospitalization, and ischemia-driven coronary revascularization procedure [PCI, CABG].

SAEs were reported by 12.6% patients in alirocumab and 13.1% in placebo during the TEAE period. There is no particular clinical pattern among SAEs preferred terms which were individually reported with low frequency.

No specific pattern was noted among the TEAEs leading to permanent treatment discontinuation.

Among the events of interest, no particular signal was detected for TEAEs related to neurological events and neurocognitive disorders.

Fourteen patients, 11 (5.3%) in alirocumab group and 3 (2.8%) in placebo group) experienced a treatment-emergent local injection site reaction. Among general allergic reactions, an imbalance was noted for asthma with 5 (2.4%) cases including 2 serious cases in the alirocumab group versus no case in the placebo group.

Five (7.4%) patients in the alirocumab group and 1 (2.7%) patient in the placebo group reported as being impaired glucose control at baseline were classified as diabetic in the TEAE period. No patient classified as having a normal status at baseline became diabetic.

Imbalance for PCSA values was noted for decrease of hemoglobin from baseline ≥20 g/L (6.3% in the alirocumab group vs 3.9% in placebo group) and for new cases of erythrocytes values ≥6 Tera/L (2.5% in the alirocumab group vs no case in the placebo group). No other relevant abnormalities for PCSA was observed.

LDL-C levels <25 mg/dL (on 2 consecutive measurements ≥21 days apart) were observed in 39 alirocumab treated patients, of whom 9 had LDL-C <15 mg/dL. The overall adverse event profile appears similar to those patients without such low LDL-C levels, although there were slightly higher rates of myalgia (7.7% in patients with LDL-C <25 mg/dL vs 3.4% in the alirocumab group as a whole, 3.7% with placebo), osteoarthritis (7.7% vs 3.9% and 4.7%), arthralgia (5.1% vs 3.9% and 7.5%) and invertebral disc protusion (5.1% vs 1.0% and 0%).

Antibodies to alirocumab were detected in a total of 18 patients (of 296 evaluable patients; 6.1%), with antibodies to alirocumab being detected at baseline in five total patients subsequently randomized to alirocumab (3/197; 1.5%) or placebo (2/99; 2.0%). Treatment-emergent low antibody positivity was observed in 13/197 (6.6%) alirocumab-treated patients with no titers exceeding 240. In seven of these patients, the antibodies were transient and resolved despite continued alirocumab treatment. The median time to detection of antibodies to alirocumab was 12 weeks; no specific clinical events were observed in antibody positive patients. However, the presence of anti-alirocumab antibodies had no observed effect on safety and efficacy.

### COMBO I: Overall results

In this population of patients, with HeFH and high baseline levels of LDL-C despite maximally tolerated statin (with or without other LLT), the following observations were made: 1) self-administered alirocumab produced significantly greater LDL-C reductions versus placebo at Week 24; 2) the absolute mean decrease from baseline in LDL-C was -90.8 mg/dL at Week 24 with alirocumab versus -15.5 mg/dL with placebo; 3) the mean achieved LDL-C levels were 107 mg/dL at Week 24 with alirocumab versus 182 mg/dL with placebo; 4) 32% of alirocumab patients reached LDL-C <70 mg/dL despite baseline LDL-C >190 mg/dL; 5) 57% of alirocumab patients achieved LDL-C <100 mg/dL at Week 24; and 6) alirocumab was generally well tolerated and TEAEs were generally comparable with the placebo group.

### COMBO II: STATISTICAL METHODS

### Sample size determination

The final total sample size was 660 with a randomization ratio 2:1 (alirocumab 440: ezetimibe 220).

### Combo II: Timing of analyses

The first step analysis included efficacy endpoints up to Week 52 (final efficacy analysis) and interim safety analysis, which was performed on all safety data up to the common study cut-off date (last patient Week 52 visit). Analysis of lipid data beyond Week 52 was descriptive. These results are presented herein.

The second step (final) analysis will be conducted at the end of the study and will consist of the final analysis of efficacy endpoints up to Week 104 and final safety analysis.

### Combo II: Analysis populations

The primary efficacy analysis population was the intent-to-treat (ITT) population, defined as all randomized patients who had an evaluable primary endpoint, that is, those with an available baseline calculated LDL-C value, and at least one available calculated LDL-C value within one of the analysis windows up to Week 24 (including all calculated LDL-C on-treatment and off-treatment).

The secondary efficacy analysis population was the modified intent-to-treat (mITT) population, defined as all randomized patients who took at least one dose or part of a dose of the double-blind investigational medicinal product (IMP) and who had an available calculated LDL-C value at baseline and at least one within one of the analysis windows up to Week 24 during the efficacy treatment period. The efficacy treatment period was defined as the time period from the first double-blind IMP administration (capsule or injection, whichever comes first) up to the day of last injection + 21 days or the day of last capsule intake + 3 days, whichever comes first.

The safety population included all randomized patients who received at least one dose or part of a dose of the double-blind IMP.

### Combo II: Efficacy analyses

Primary analyses of efficacy endpoints were conducted using an ITT approach (based on the ITT population defined above), including all lipid data, regardless of whether the patient was continuing therapy or not. This corresponds to ITT estimands, defined for primary and key secondary endpoints. In addition, analyses were also conducted using an on-treatment approach (based on the mITT population defined above), including lipid data collected during the efficacy treatment period. This corresponds to on-treatment estimands of key secondary endpoints.

The ITT approach analyzed all patients, irrespective of their adherence to the treatment; it assessed the benefit of the treatment strategy and reflected as much as possible the effect in a population of patients. The on-treatment approach analyzed the effect of treatment, restricted to the period during which patients actually received the treatment. It assessed the benefit that a treatment would achieve in patients adherent to treatment up to the considered time point.

Efficacy analyses were performed according to treatment as-randomized.

All measurements, scheduled or unscheduled, fasting or not fasting, were assigned to analysis windows in order to provide an assessment for Week 4 to Week 104 time points.

With regards to the primary efficacy analysis (ITT approach), the percent change in calculated LDL-C from baseline to Week 24 was analyzed using a mixed-effect model with repeated measures (MMRM) approach. All post-baseline data available from Week 4 to Week 52 analysis windows were used and missing data were accounted for by the MMRM. The model included the fixed categorical effects of treatment group (ezetimibe versus alirocumab), randomization strata (as per IVRS), time point (Week 4 to Week 52), treatment-by-time point interaction and strata-by-time point interaction, as well as, the continuous fixed covariates of baseline LDL-C value and baseline value-by-time- point interaction. This model provided baseline adjusted least-squares means (LSmeans) estimates at Week 24 for both treatment groups with their corresponding 95% confidence interval. To compare the alirocumab to the ezetimibe group, an appropriate statement was used to test the differences of these estimates at the 5% alpha level.

A hierarchical procedure has been defined to test key secondary endpoints while controlling for multiplicity (using above order of key secondary endpoints). The first key secondary endpoint was the percent change in calculated LDL-C from baseline to Week 24 using an on-treatment approach.

Continuous secondary variables anticipated to have a normal distribution (i.e., lipids other than TGs and Lp(a)) were analyzed using the same MMRM model as for the primary endpoint. Continuous endpoints anticipated to have a non-normal distribution (i.e., TGs and Lp(a)) were analyzed using multiple imputation approach for handling of missing values followed by robust regression model with endpoint of interest as response variable using M-estimation (using SAS ROBUSTREG procedure) with treatment group, randomization strata (as per IVRS) and corresponding baseline value(s) as effects to compare treatment effects. Combined estimate for mean in both treatment groups, as well as the differences of these estimates, with their corresponding SEs, 95% CIs and p-value were provided (through SAS MIANALYZE procedure).

Binary secondary efficacy endpoints were analyzed using multiple imputation approach for handling of missing values followed by stratified logistic regression with treatment group as main effect and corresponding baseline value(s) as covariate, stratified by randomization factors (as per IVRS). Combined estimates of odds ratio versus ezetimibe, 95% Cl, and p-value were provided (through SAS MIANALYZE procedure).

### Combo II: Safety analyses

Safety analyses were descriptive, performed on the safety population according to treatment actually received. For analysis of TEAE and PCSA, the safety analysis focused on the TEAE period defined as the time from the first dose of double-blind IMP administration (capsule or injection, whichever comes first) up to 70 days after the last double-blind injection. Analyses of laboratory data and vital signs overtime were performed on the period up to 21 days after the last IMP injection.

### COMBO II: RESULTS

### Patient Accountability

Of the 720 randomized patients, all patients were treated and were therefore included in the safety population.

Thirteen randomized patients (12 in the alirocumab group and 1 in the ezetimibe group) were not included in the ITT population (no LDL-C value within one of the analysis windows through Week 24).

Twenty-one randomized patients were excluded from the mITT population (patients excluded from the ITT population and patients with no LDL-C value within one of the analysis windows up to Week 24 during the efficacy treatment period).

In the alirocumab group, among the 446 patients who received at least one injection after Week 12, 82 (18.4%) patients received automatic up-titration at Week 12 from alirocumab 75 mg Q2W to 150 mg Q2W in a blinded manner.

**Table 14 - Analysis populations**

| | **Ezetimibe 10** | **Alirocumab 75 Q2W/Up150 Q2W** | **All** |
|---|---|---|---|
| Randomized population | 241 (100%) | 479 (100%) | 720 (100%) |
| | | | |
| Efficacy populations | | | |
| Intent-to-Treat (ITT) | 240 (99.6%) | 467 (97.5%) | 707 (98.2%) |
| | | | |
| Modified Intent-to-Treat (mITT) | 235 (97.5%) | 464 (96.9%) | 699 (97.1%) |
| | | | |
| Safety population | 241 | 479 | 720 |
| Note: The safety population patients are tabulated according to treatment actually received (as treated) . | | | |
| For the other populations, patients are tabulated according to their randomized treatment. | | | |

### Combo II: Study disposition

Study disposition, exposure and safety analyses were assessed using all data up to the study common cut-off date (defined as the date of the last patient's Week 52 visit). Therefore, this first step analysis includes data beyond Week 52 for some patients. The results presented herein are from this first step analysis, and include efficacy data up to week 52, safety data up to week 52-102 (including all data collected until the last patient visit at W52).

No randomized patients completed the 104 weeks double-blind study treatment period and 612 (85.0%) randomized patients (N=406 in the alirocumab arm and N=206 in the ezetimibe arm) were treatment ongoing at the time of the first-step analysis cut-off date.

The double-blind IMP was prematurely discontinued before Week 104 for 73 (15.2%) randomized patients in the alirocumab group (including 71 patients who discontinued before Week 52) and 35 (14.5%) randomized patients in the ezetimibe group (including 33 patients who discontinued before Week 52).

The main reasons for study treatment discontinuation were adverse event and other reasons. The category "other" represented the most frequently reported and included the following: patient did not meet protocol inclusion/exclusion criteria (3 patients), subject withdrawal for personal reasons (14 patients), sudden death / death (5 patients) (there was no decision of investigator to stop the treatment prior death), lost to follow-up (1 patient). In this first step analysis, final results are available for the primary efficacy endpoint at Week 24 and key secondary efficacy endpoints assessed at Week 12, Week 24 and Week 52. At Week 24, the primary efficacy endpoint was available for 428 (91.6%) in the alirocumab and 221 (92.1 %) in the ezetimibe groups. At Week 52, the primary efficacy endpoint was available for 414 (88.7%) in the alirocumab and 211 (87.9%) in the ezetimibe groups.

The primary endpoint was missing for 58 patients (39 patients (8.4%) in the alirocumab and 19 patients (7.9%) in the ezetimibe groups). At the Week 24 visit, the reasons for missingness were as follows: 29 samples not done due to earlier study discontinuation; 8 samples done outside analysis time window; 9 missing samples while visit Week 24 was done; 12 samples done but measurement could not be done (lipemia, insufficient quantity, TGs > 400 mg/dL [> 4.52 mmol/L], sample lost).

### COMBO II: Demographics and baseline characteristics

Demographic characteristics, disease characteristics and lipid parameters at baseline were generally similar in the alirocumab group as compared to the ezetimibe group. For the alirocumab group (N=479), the age (mean years (SD)) was 61.7 (9.4), the percentage of males was 75.2% (N=360), the percentage white race was 84.3% (N=404), the percentage black or African American race was 4.4% (N=21), the percentage of other races was 11.3% (N=54), and the BMI (mean kg/m² (SD)) was 30.0 (5.4). For the ezetimibe group (N=241), the age (mean years (SD)) was 61.3 (9.2), the percentage of males was 70.5% (N=170), the percentage white race was 85.5% (N=206), the percentage black or African American race was 2.9% (N=7), the percentage of other races was 11.6% (N=28), and the BMI (mean kg/m² (SD)) was 30.3 (5.1).

Baseline characteristics of the alirocumab and ezetimibe groups are set forth in Tables 15-19. Mean (SD) baseline LDL-C was 107.3 (35.7) mg/dL (2.778 (0.926) mmol/L). The HbA_{1c} (mean (SD), %) was 6.05 (0.75) for the alirocumab group and 6.07 (0.77) for the ezetimibe group. Approximately 90% of the randomized population had a history of CHD (i.e., at least one of the 5 below subcategories for CHD) and the remaining patients had CHD risk equivalents (**Table 16**), except for 2 patients, in the alirocumab group, who had no fully documented CHD or CHD risk equivalents. Globally 30% had a history of diabetes. All patients but one were treated with a statin, 66.7% receiving high intensity statin (atorvastatin 40 to 80 mg daily or rosuvastatin 20 to 40 mg daily) and 2.1% receiving simvastatin 80 mg daily.

In summary, 1112 high cardiovascular risk patients were screened, 720 of whom were eligible and willing to participate in the study. Of these, 479 were randomly assigned to alirocumab and 241 to ezetimibe. The mean (SD) age was 61.6 (9.3) years, 73.6% of participants were men, 90.1% had coronary heart disease, 30.7% had type 2 diabetes, the mean (SD) baseline calculated LDL-C concentration was 107.3 (35.7) mg/dL, and 66.7% were taking high-intensity statin treatment (i.e. atorvastatin 40/80 mg/day or rosuvastatin 20/40 mg/day). Fifteen patients were on simvastatin 80 mg. Baseline characteristics were balanced between the two groups.

**Table 15 - Disease characteristics and other relevant baseline data - Randomized population**

| | **Ezetimibe 10** | | **Alirocumab 75 Q2W/Up150 Q2W** | | **All** | |
|---|---|---|---|---|---|---|
| | **(N=241)** | | **(N=479)** | | **(N=720)** | |
| Time from hypercholesterolemia diagnosis (years) | | | | | | |
| Number | 240 | | 479 | | 719 | |
| Mean (SD) | 8.81 (7.62) | | 9.43 (7.23) | | 9.22 (7.36) | |
| Median | 7.48 | | 7.89 | | 7.85 | |
| Min : Max | 0.1 : 53.9 | | 0.1 : 46.8 | | 0.1 : 53.9 | |
| | | | | | | |
| Fredrickson classification of Hyperlipoproteinemia | | | | | | |
| Number | 185 | | 369 | | 554 | |
| IIa | 113 | (46.9%) | 232 | (48.4%) | 345 | (47.9%) |
| IIb | 72 | (29.9%) | 137 | (28.6%) | 209 | (29.0%) |
| IV | 0 | | 0 | | 0 | |

**Table 16 - Medical history of specific interest: cardiovascular history and risk factors - Randomized population**

| | **Ezetimibe 10** | | **Alirocumab 75 Q2W/Up150 Q2W** | | **All** | |
|---|---|---|---|---|---|---|
| | **(N=241)** | | **(N=479)** | | **(N=720)** | |
| Any cardiovascular history/risk factors | 241 | (100%) | 477 | (99.6%) | 718 (99.7%) | |
| | | | | | | |
| Coronary heart disease ^{a} | 212 | (88.0%) | 437 | (91.2%) | 649 (90.1%) | |
| Acute Myocardial Infarction | 139 | (57.7%) | 277 | (57.8%) | 416 (57.8%) | |
| Silent Myocardial Infarction | 4 | (1.7%) | 11 | (2.3%) | 15(2.1%) | |
| Unstable Angina | 46 | (19.1 %) | 106 | (22.1%) | 152 (21.1%) | |
| Coronary Revascularization Procedures | 165 | (68.5%) | 330 | (68.9%) | 495 (68.8%) | |
| Other Clinically Significant CHD ^{b} | 82 | (34.0%) | 184 | (38.4%) | 266 (36.9%) | |
| CHD associated with 1 or more comorbidity (among hypertension, diabetes or moderate CKD) and/or associated with other CVD (ischemic stroke, PAD) | 178 | (73.9%) | 366 | (76.4%) | 544 (75.6%) | |
| | | | | | | |
| Coronary heart disease risk equivalents ^{a} | 72 | (29.9%) | 151 | (31.5%) | 223 (31.0%) | |
| Ischemic Stroke | 20 | (8.3%) | 40 | (8.4%) | 60 | (8.3%) |
| Peripheral Arterial Disease | 11 | (4.6%) | 24 | (5.0%) | 35 | (4.9%) |
| Moderate Chronic Kidney Disease | 23 | (9.5%) | 61 | (12.7%) | 84 | (11.7%) |
| Known history of diabetes mellitus AND 2 or more additional risk factors | 31 | (12.9%) | 59 | (12.3%) | | 90 (12.5%) |
| At least 2 CHD risk equivalents or 1 CHD risk equivalent associated with hypertension or diabetes | 67 | (27.8%) | 141 | (29.4%) | 208 | (28.9%) |

| | | | | | | |
|---|---|---|---|---|---|---|
| Note: A patient can be counted in several categories. ^{a} according to the items pre-listed in the e-CRF. ^{b} diagnosed by invasive or non-invasive testing. PAD defined as Intermittent claudication TOGETHER WITH ankle-brachial index ≤ 0.90 OR TOGETHER WITH peripheral revascularization procedure/surgery OR critical limb ischemia TOGETHER WITH peripheral revascularization procedure/surgery or thrombolysis. | | | | | | |

**Table 17 - Medical history of specific interest: other medical history of special interest - Randomized population**

| | **Ezetimibe 10** | | **Alirocumab 75 Q2W/Up150 Q2W** | | **All** | |
|---|---|---|---|---|---|---|
| | **(N=241)** | | **(N=479)** | | **(N=720)** | |
| Other medical history of special interest | 219 | (90.9%) | 424 | (88.5%) | 643 | (89.3%) |
| Hypertension ^{a} | 198 | (82.2%) | 382 | (79.7%) | 580 | (80.6%) |
| Type 1 Diabetes ^{a} | 0 | | 2 | (0.4%) | 2 | (0.3%) |
| Type 2 Diabetes ^{a} | 76 | (31.5%) | 145 | (30.3%) | 221 | (30.7%) |
| Family history of premature coronary heart disease ^{a} | 59 | (24.5%) | 94 | (19.6%) | 153 | (21.3%) |
| Family history of type 2 diabetes ^{a} | 38 | (15.8%) | 60 | (12.5%) | 98 | (13.6%) |
| Menopause (females) ^{a} | 65 | (27.0%) | 106 | (22.1 %) | 171 | (23.8%) |

| | | | | | | |
|---|---|---|---|---|---|---|
| ^{a} according to the items pre-listed in the e-CRF. Note: A patient can be counted in several categories. | | | | | | |

**Table 18 - Background LMT at randomization - Randomized population**

| | **Ezetimibe 10** | | **Alirocumab 75 Q2W/Up150 Q2W** | | **All** | |
|---|---|---|---|---|---|---|
| | **(N=241)** | | **(N=479)** | | **(N=720)** | |
| Any statin | 241 | (100%) | 478 | (99.8%) | 719 | (99.9%) |
| | | | | | | |
| Taking high intensity statin | 160 | (66.4%) | 320 | (66.8%) | 480 | (66.7%) |
| Atorvastatin daily dose (mg) | 118 | (49.0%) | 237 | (49.5%) | 355 | (49.3%) |
| 10 | 6 | (2.5%) | 5 | (1.0%) | 11 | (1.5%) |
| 20 | 11 | (4.6%) | 26 | (5.4%) | | 37(5.1%) |
| 40 | 55 | (22.8%) | 115 | (24.0%) | 170 | (23.6%) |
| 80 | 46 | (19.1%) | 90 | (18.8%) | 136 | (18.9%) |
| Other doses | 0 | | 1 | (0.2%) | 1 | (0.1%) |
| Rosuvastatin daily dose (mg) | 75 | (31.1%) | 137 | (28.6%) | 212 | (29.4%) |
| 5 | 2 | (0.8%) | 9 | (1.9%) | 11 | (1.5%) |
| 10 | 14 | (5.8%) | 12 | (2.5%) | 26 | (3.6%) |
| 20 | 41 | (17.0%) | 80 | (16.7%) | 121 | (16.8%) |
| 40 | 17 | (7.1%) | 34 | (7.1 %) | 51 | (7.1%) |
| Other doses | 1 | (0.4%) | 2 | (0.4%) | 3 | (0.4%) |
| Simvastatin daily dose (mg) | 49 | (20.3%) | 105 | (21.9%) | 154 | (21.4%) |
| 10 | 4 | (1.7%) | 5 | (1.0%) | 9 | (1.3%) |
| 20 | 16 | (6.6%) | 28 | (5.8%) | 44 | (6.1%) |
| 40 | 22 | (9.1%) | 61 | (12.7%) | 83 | (11.5%) |
| 80 | 5 | (2.1%) | 10 | (2.1%) | 15 | (2.1%) |
| Other doses | 2 | (0.8%) | 1 | (0.2%) | 3 | (0.4%) |
| | | | | | | |
| Any LMT other than statins^{a} | 12 | (5.0%) | 29 | (6.1%) | 41 | (5.7%) |
| Any LMT other than nutraceuticals | 4 | (1.7%) | 13 | (2.7%) | 17 | (2.4%) |
| Nutraceuticals | 8 | (3.3%) | 16 | (3.3%) | 24 | (3.3%) |

| | | | | | | |
|---|---|---|---|---|---|---|
| ^{a} in combination with statins or not. High intensity statin corresponds to atorvastatin 40 to 80 mg daily or rosuvastatin 20 to 40 mg daily. | | | | | | |

**Table 19 - Lipid efficacy parameters at baseline - Quantitative summary in conventional units - Randomized population**

| | **Ezetimibe 10** | **Alirocumab 75 Q2W/Up150 Q2W** | **All** |
|---|---|---|---|
| | **(N=241)** | **(N=479)** | **(N=720)** |
| Calculated LDL-C (mg/dL) | | | |
| Number | 241 | 479 | 720 |
| Mean (SD) | 104.6 (34.1) | 108.6 (36.5) | 107.3 (35.7) |
| Median | 98.0 | 100.0 | 99.0 |
| Q1 : Q3 | 81.0 : 122.0 | 83.0 : 126.0 | 83.0 : 124.0 |
| Min : Max | 38 : 243 | 22 : 303 | 22 : 303 |
| | | | |
| Measured LDL-C (mg/dL) | | | |
| Number | 213 | 429 | 642 |
| Mean (SD) | 101.1 (35.0) | 103.8 (34.8) | 102.9 (34.9) |
| Median | 93.0 | 96.0 | 95.0 |
| Q1 : Q3 | 75.0 : 118.0 | 79.0 : 120.0 | 78.0 : 119.0 |
| Min : Max | 47 : 237 | 26 : 284 | 26 : 284 |
| | | | |
| Non-HDL-C (mg/dL) | | | |
| Number | 241 | 479 | 720 |
| Mean (SD) | 136.8 (40.4) | 139.1 (40.4) | 138.3 (40.4) |
| Median | 128.0 | 129.0 | 128.0 |
| Q1 : Q3 | 109.0 : 154.0 | 110.0 : 161.0 | 110.0: 158.0 |
| Min: Max | 65 : 317 | 61 : 322 | 61 : 322 |
| | | | |
| Total-C (mg/dL) | | | |
| Number | 241 | 479 | 720 |
| Mean (SD) | 183.9 (41.8) | 186.5 (41.2) | 185.6 (41.4) |
| Median | 175.0 | 178.0 | 177.0 |
| Q1 : Q3 | 156.0 : 201.0 | 159.0 : 209.0 | 157.0 : 206.5 |
| Min: Max | 117 : 347 | 90 : 411 | 90 : 411 |
| | | | |
| HDL-C (mg/dL) | | | |
| Number | 241 | 479 | 720 |
| Mean (SD) | 47.2 (13.5) | 47.4 (13.3) | 47.3 (13.4) |
| Median | 44.0 | 45.0 | 45.0 |
| Q1 : Q3 | 37.0 : 56.0 | 38.0 : 54.0 | 38.0 : 54.0 |
| Min : Max | 21 : 112 | 16 : 117 | 16 : 117 |
| | | | |
| Fasting TGs (mg/dL) | | | |
| Number | 241 | 479 | 720 |
| Mean (SD) | 159.2 (77.5) | 153.9 (77.0) | 155.7 (77.1) |
| Median | 141.0 | 133.0 | 137.0 |
| Q1 : Q3 | 106.0 : 198.0 | 98.0 : 193.0 | 100.0 : 195.0 |
| Min : Max | 47 : 456 | 46 : 532 | 46 : 532 |

| Lipoprotein-(a) (mg/d L) | | | |
|---|---|---|---|
| Number | 234 | 470 | 704 |
| Mean (SD) | 40.5 (45.0) | 45.2 (46.3) | 43.6 (45.9) |
| Median | 23.0 | 28.0 | 25.0 |
| Q1 : Q3 | 8.0 : 56.0 | 8.0 : 70.0 | 8.0 : 66.0 |
| Min : Max | 2 : 233 | 2 : 245 | 2 : 245 |
| | | | |

| Apo-B (mg/dL) | | | |
|---|---|---|---|
| Number | 234 | 470 | 704 |
| Mean (SD) | 93.5 (23.1) | 94.3 (23.2) | 94.0 (23.1) |
| Median | 89.0 | 90.0 | 90.0 |
| Q1 : Q3 | 78.0 : 105.0 | 78.0 : 108.0 | 78.0 : 107.0 |
| Min : Max | 43 : 193 | 45 : 180 | 43 : 193 |
| | | | |

| Apo-A1 (mg/dL) | | | |
|---|---|---|---|
| Number | 234 | 470 | 704 |
| Mean (SD) | 140.2 (25.5) | 140.7 (23.6) | 140.5 (24.2) |
| Median | 137.0 | 139.0 | 139.0 |
| Q1 : Q3 | 121.0 : 159.0 | 124.0 : 155.0 | 123.0 : 156.0 |
| Min : Max | 87 : 248 | 89 : 237 | 87 : 248 |
| | | | |

| Apo-B/Apo-A1 (ratio) | | | |
|---|---|---|---|
| Number | 234 | 470 | 704 |
| Mean (SD) | 0.688 (0.213) | 0.689 (0.206) | 0.689 (0.208) |
| Median | 0.650 | 0.660 | 0.655 |
| Q1 : Q3 | 0.540 : 0.790 | 0.540 : 0.810 | 0.540 : 0.800 |
| Min : Max | 0.33 : 1.65 | 0.30: 1.79 | 0.30 : 1.79 |
| | | | |

| Total-C/HDL-C (ratio) | | | |
|---|---|---|---|
| Number | 241 | 479 | 720 |
| Mean (SD) | 4.155 (1.436) | 4.188 (1.342) | 4.177 (1.373) |
| Median | 3.864 | 3.914 | 3.902 |
| Q1 : Q3 | 3.228 : 4.761 | 3.254 : 4.818 | 3.249 : 4.815 |
| Min : Max | 2.10 : 11.57 | 1.87 : 9.40 | 1.87 : 11.57 |

The collection of measured LDL-C was not planned in the initial protocol and was added in an amendment. Therefore measured LDL-C values are available for fewer patients compared to calculated LDL-C values.

### COMBO II: Dosage and duration

Exposure to injections (alirocumab or placebo) and to capsules (ezetimibe or placebo) was similar across treatment groups with a mean exposure of approximately 57 weeks.

In the alirocumab group, among the 446 patients who received at least one injection after Week 12, 82 (18.4%) patients received automatic up-titration at Week 12 from alirocumab 75 mg Q2W to 150 mg Q2W in a blinded manner.

The mean (SD) duration of injection exposures was 58.0 (18.7) weeks (with a mean [SD] of 26.6 [8.8] injections) in the alirocumab arm and 57.7 (19.0) weeks (26.6 [9.0] injections) in the ezetimibe arm. At the time of this analysis, 84.8% of patients in alirocumab arm and 85.5% in the ezetimibe arm had treatment ongoing (active or placebo). Eighty-two (18.4%) patients in the alirocumab arm had the dose increased at week 12 to the 150 mg Q2W dosing regimen because their LDL-C at week 8 was ≥70 mg/dL.

### COMBO II: Primary efficacy endpoint

The ITT analysis includes all calculated LDL-C values collected on-treatment and off-treatment up to Week 52. The primary endpoint (percent change in calculated LDL-C from baseline to Week 24) analysis is provided based on a MMRM model on the ITT population, using LS means estimates at Week 24. Thirty-nine (8.4%) patients in the alirocumab group and 19 (7.9%) patients in the ezetimibe group did not have a calculated LDL-C value at Week 24. These missing values were accounted for by the MMRM model.

Results of the primary endpoint analysis are presented in **Table 20**, in mmol/L and mg/dL.

Statistically significant decrease in percent change in LDL-C from baseline to Week 24 was observed in the alirocumab group (LS mean versus baseline -50.6%) compared to the ezetimibe group (LS mean versus baseline -20.7%) (LS mean difference vs. ezetimibe of - 29.8%, p <0.0001).

In the alirocumab group, LDL-C reduction from baseline was observed from Week 4 to Week 52 (see **Figure 6** and **Table 20**). The LDL-C reduction was maintained up to Week 52 in the alirocumab group (LS mean versus baseline at Week 52 of -49.5% versus -50.6% at Week 24).

**Table 20- Percent change from baseline in calculated LDL-C at Week 24: MMRM - ITT analysis - ITT population and On-treatment population**

| **ITT POPULATION** | **Ezetimibe 10** | **Alirocumab 75 Q2W/Up150 Q2W** |
|---|---|---|
| **Calculated LDL Cholesterol** | **(N=240)** | **(N=467)** |
| Baseline (mmol/L) | | |
| Number | 240 | 467 |
| Mean (SD) | 2.706 (0.884) | 2.805 (0.946) |
| Median | 2.538 | 2.590 |
| Min : Max | 0.98 : 6.29 | 0.57 : 7.85 |
| Baseline (mg/dL) | | |
| Number | 240 | 467 |
| Mean (SD) | 104.5 (34.1) | 108.3 (36.5) |
| Median | 98.0 | 100.0 |
| Min : Max | 38 : 243 | 22 : 303 |
| | | |
| Week 24 percent change from baseline (%) | | |
| LS Mean (SE) | -20.7 (1.9) | -50.6 (1.4) |
| LS mean difference (SE) vs ezetimibe | | -29.8 (2.3) |
| 95% Cl | | (-34.4 to -25.3) |
| p-value vs ezetimibe | | <0.0001* |

**Table 21 - Calculated LDL-C over time - ITT analysis - ITT population**

| | **Ezetimibe 10** | | | **Alirocumab 75 Q2W/Up150 Q2W** | | |
|---|---|---|---|---|---|---|
| | **(N=240)** | | | **(N=467)** | | |
| **Calculated LDL-C** | **Value** | **Change from baseline** | **Percent change from baseline** | **Value** | **Change from baseline** | **Percent change from baseline** |
| LS Mean (SE) (mmol/L) | | | | | | |
| Baseline ^{a} | 2.706 (0.057) | NA | NA | 2.805 (0.044) | NA | NA |
| Week 4 | 2.002 (0.044) | -0.770 (0.044) | -25.3 (1.6) | 1.254 (0.031) | -1.518 (0.031) | -54.7 (1.1) |
| Week 8 | 2.047 (0.045) | -0.725 (0.045) | -24.3 (1.7) | 1.259 (0.032) | -1.512 (0.032) | -54.3 (1.2) |
| Week 12 | 2.102 (0.049) | -0.670 (0.049) | -21.8 (1.8) | 1.350 (0.035) | -1.422 (0.035) | -51.2 (1.3) |
| Week 16 | 2.069 (0.048) | -0.702 (0.048) | -23.0 (1.8) | 1.194 (0.035) | -1.578 (0.035) | -56.4 (1.3) |
| Week 24 | 2.138 (0.051) | -0.634 (0.051) | -20.7 (1.9) | 1.337 (0.036) | -1.435 (0.036) | -50.6 (1.4) |
| Week 36 | 2.187 (0.058) | -0.585 (0.058) | -19.3 (2.0) | 1.337 (0.041) | -1.434 (0.041) | -51.2 (1.4) |
| Week 52 | 2.210 (0.061) | -0.562 (0.061) | -18.3 (2.1) | 1.381 (0.044) | -1.390 (0.044) | -49.5 (1.5) |
| | | | | | | |
| LS Mean (SE) (mg/dL) | | | | | | |
| Baseline ^{a} | 104.5 (2.2) | NA | NA | 108.3 (1.7) | NA | NA |
| Week 4 | 77.3 (1.7) | -29.7 (1.7) | -25.3 (1.6) | 48.4 (1.2) | -58.6 (1.2) | -54.7 (1.1) |
| Week 8 | 79.0 (1.7) | -28.0 (1.7) | -24.3 (1.7) | 48.6 (1.2) | -58.4 (1.2) | -54.3 (1.2) |
| Week 12 | 81.1 (1.9) | -25.9 (1.9) | -21.8 (1.8) | 52.1 (1.4) | -54.9 (1.4) | -51.2 (1.3) |
| Week 16 | 79.9 (1.9) | -27.1 (1.9) | -23.0 (1.8) | 46.1 (1.3) | -60.9 (1.3) | -56.4 (1.3) |
| Week 24 | 82.5 (2.0) | -24.5 (2.0) | -20.7 (1.9) | 51.6 (1.4) | -55.4 (1.4) | -50.6 (1.4) |
| Week 36 | 84.4 (2.2) | -22.6 (2.2) | -19.3 (2.0) | 51.6 (1.6) | -55.4 (1.6) | -51.2 (1.4) |
| Week 52 | 85.3 (2.4) | -21.7 (2.4) | -18.3 (2.1) | 53.3 (1.7) | -53.7 (1.7) | -49.5 (1.5) |

| | | | | | | |
|---|---|---|---|---|---|---|
| ^{a} Baseline is described using means and standard errors. Note: Least-squares (LS) means, standard errors (SE) and p-value taken from MMRM (mixed-effect model with repeated measures) analysis. The model includes the fixed categorical effects of treatment group, randomization strata as per IVRS, time point, treatment-by-time point interaction, strata-by-time point interaction, as well as the continuous fixed covariates of baseline LDL-C value and baseline LDL-C value-by-time point interaction MMRM model and baseline description run on patients with a baseline value and a post-baseline value in at least one of the analysis windows used in the model. | | | | | | |

### COMBO II: Key secondary efficacy endpoints and additional analyses

The following table summarizes analysis results on key secondary endpoints in the hierarchical order. All key secondary endpoints are statistically significant according to the hierarchical testing procedure up to HDL-C endpoint at Week 24 (ITT estimand) included.

Statistical significance was not reached for fasting TG at Week 24 (ITT estimand) and therefore the testing procedure was stopped, p-values are provided from this endpoint for descriptive purpose only.

**Table 22**

| **Endpoint** | **Analysis** | **Results** | **Results** | **P-value** |
|---|---|---|---|---|
| Calculated LDL-C - Percent change from baseline to Week 24 | On-treatment | LS mean difference vs. ezetimibe of - 30.6% | LS mean difference vs. baseline of - 52.4% | <0.0001 |
| Calculated LDL-C - Percent change from baseline to Week 24, beta - quantification method | ITT | LS mean difference vs. ezetimibe of - 29.7% | LS mean difference vs. baseline of - 47.7% | <0.0001 |
| Calculated LDL-C - Percent change from baseline to Week 12 | ITT | LS mean difference vs. ezetimibe of - 29.4% | LS mean difference vs. baseline of -51.2 % | <0.0001 |
| Calculated LDL-C - Percent change from baseline to Week 12 | On-treatment | LS mean difference vs. ezetimibe of - 29.7% | LS mean difference vs. baseline of -52.4 | <0.0001 |
| Apo-B - Percent change from baseline to Week 24 | ITT | LS mean difference vs. ezetimibe of - 22.4% | LS mean difference vs. baseline of -40.7 | <0.0001 |
| Apo-B - Percent change from baseline to Week 24 | On-treatment | LS mean difference vs. ezetimibe of -23% | LS mean difference vs. baseline of -42.1 | <0.0001 |
| Non-HDL-C - Percent change from baseline to Week 24 | ITT | LS mean difference vs. ezetimibe of - 22.9% | LS mean difference vs. baseline of-42.1 | <0.0001 |
| Non-HDL-C - Percent change from baseline to Week 24 | On-treatment | LS mean difference vs. ezetimibe of - 23.5% | LS mean difference vs. baseline of -43.7 | <0.0001 |
| Total-C - Percent change from baseline to Week 24 | ITT | LS mean difference vs. ezetimibe of - 14.7% | LS mean difference vs. baseline of -29.3 | <0.0001 |
| Apo-B - Percent change from baseline to Week 12 | ITT | LS mean difference vs. ezetimibe of - 22.5% | LS mean difference vs. baseline of -39.7 | <0.0001 |
| Non-HDL-C - Percent change from baseline to Week 12 | ITT | LS mean difference vs. ezetimibe of -22% | LS mean difference vs. baseline of -42.6 | <0.0001 |
| Total-C - Percent change from baseline to Week 12 | ITT | LS mean difference vs. ezetimibe of - 14.3% | LS mean difference vs. baseline of -29.4 | <0.0001 |
| Calculated LDL-C - Percent change from baseline to Week 52 | ITT | LS mean difference vs. ezetimibe of - 31.2% | LS mean difference vs. baseline of -49.5 | <0.0001 |
| Proportion of patients reaching calculated LDL-C <70 mg/dL (1.81 mmol/L) at Week 24 | ITT | combined estimate for odds-ratio vs. ezetimibe of 5.4 | | <0.0001 |
| Proportion of patients reaching calculated LDL-C <70 mg/dL (1.81 mmol/L) at Week 24 | On-treatment | combined estimate for odds-ratio vs. ezetimibe of 5.9 | | <0.0001 |
| Lp(a) - Percent change from baseline to Week 24 | ITT | combined estimate for adjusted mean difference vs. ezetimibe of -21.7% | LS mean difference vs. baseline of -27.8 | <0.0001 |
| HDL-C - Percent change from baseline to Week 24 | ITT | LS mean difference vs. ezetimibe of 8.1% | LS mean difference vs. baseline of _+8.6 | <0.0001 |
| Fasting TGs - Percent change from baseline to Week 24 | ITT | combined estimate for adjusted mean difference vs. ezetimibe of -0.3% | | 0.9117 |
| Apolipoprotein A-1 | ITT | LS mean difference vs. ezetimibe of 6.3% | LS mean difference vs. baseline of +5.0 | <0.0001 |

The on-treatment analysis of the LDL-C percent change from baseline to Week 24 shows very consistent results with the ITT analysis (LS mean difference vs. ezetimibe of -30.6% in the on-treatment analysis versus -29.8% in the ITT analysis).

The following key secondary endpoints including Apo B, non-HDL-C, Total-C, Lp(a), HDL-C at various time points as well as the proportion of patients reaching their LDL-C goals at Week 24 were statistically significant according to the hierarchical testing procedure.

Most high cardiovascular risk patients receiving Alirocumab on background statin achieved their LDL-C goal. The proportion of patients reaching calculated LDL-C <70 mg/dL (1.81 mmol/L) at Week 24 was significantly higher in the alirocumab than in the ezetimibe group (combined estimate for proportion of 77.0% in the alirocumab group vs. 45.6% in the ezetimibe group, p<0.0001).

The difference in percent change in fasting TGs from baseline to Week 24 in the ITT analysis was non-statistically significant. The LS mean versus baseline was -13.0% in the alirocumab group and -12.8% in the ezetimibe group (LS mean difference vs. ezetimibe of-0.3%, p=0.9117).

One hundred and five (22.8%) patients experienced two consecutive calculated LDL-C values <25 mg/dL. No particular safety concern has been observed in these 105 patients.

**Table 23 - Number (%) of patients with 2 consecutive LDL-C < 25mg/dL (<0.65 mmol/L) during the treatment period- Safety population**

| | **Ezetimibe 10 (N=241)** | **Alirocumab 75 Q2W/Up150 Q2W (N=479)** |
|---|---|---|
| Patients with 2 consecutive calculated LDL-C value <25 mg/dL ¹ | 0/230 | 105/460 (22.8%) |
| | | |
| Time to the first calculated LDL-C value < 25 mg/dL (weeks) ² | | |
| Number | 0 | 105 |
| Mean (SD) | | 12.56 (12.31) |
| Median | | 5.14 |
| Min : Max | | 3.1 : 64.1 |
| | | |
| Patients with 2 consecutive calculated LDL-C value <15 mg/dL ¹ | 0/230 | 31/460 (6.7%) |
| | | |
| Time to the first calculated LDL-C value < 15 mg/dL (weeks) ² | | |
| Number | 0 | 31 |
| Mean (SD) | | 15.07 (13.15) |
| Median | | 9.14 |
| Min : Max | | 3.1 : 52.1 |

| | | |
|---|---|---|
| The number (n) represents the subset of the total number of patients who met the criteria The denominator (/N) within a treatment group is the number of patients for the treatment group who had at least two calculated LDL-C values assessed at least 21 days apart in the efficacy period ¹ 2 consecutive values are considered if spaced out by at least 21 days ² First calculated LDL-C value <25 or <15 mg/dL among the first 2 consecutive calculated LDL-C values <25 or <15 mg/dL per patient | | |

**Table 24 - Percent change from baseline to week 24 in LDL-C and secondary lipid parameters (on-treatment analysis)**

| | **Alirocumab 75 mg SC Q2W (n=464)** | **Ezetimibe 10 mg ezetimibe daily (n=235)** | **Alirocumab vs ezetimibe** | | |
|---|---|---|---|---|---|
| | | | **LS mean difference (SE) %** | **95% CI** | ***P* Value** |
| LDL-C | -52.4 (1.3) | -21.8 (1.8) | -30.6 (2.2) | -34.9 to -26.2 | <.0001 |
| Apo B | -42.1 (1.0) | -19.1 (1.4) | -23.0 (1.8) | -26.5 to -19.6 | <.0001 |
| Non-HDL-C | -43.7 (1.1) | -20.2 (1.6) | -23.5 (1.9) | -27.2 to -19.7 | <.0001 |
| Total cholesterol | -30.4 (0.9) | -15.2 (1.2) | -15.1 (1.5) | -18.0 to -12.2 | <.0001 |
| Lipoprotein(a) | -28.1 (1.4) | -5.6 (1.9) | -22.5 (2.4) | -27.2 to -17.8 | <.0001 |
| Triglycerides (fasted) | -13.5 (1.4) | -13.3 (2.0) | -0.2 (2.5) | -5.1 to 4.6 | .93 |
| HDL-C | 9.0 (0.8) | 0.8 (1.1) | 8.2 (1.4) | 5.6 to 10.9 | <.0001 |

### COMBO II: Summary efficacy results

At Week 24, the percent change from baseline in calculated LDL-C in the ITT population was significantly greater in the alirocumab group (LS mean versus baseline - 50.6%) compared to the ezetimibe group (LS mean versus baseline -20.7%) (LS mean difference versus ezetimibe of -29.8%, p < 0.0001) (**Table 20**). The on-treatment analysis of LDL-C percent change from baseline to Week 24 shows consistent results with the ITT analysis with a LS mean difference versus ezetimibe of -30.6% in the on-treatment analysis (p<0.0001) (**Table 24**).

The time-course of changes in LDL-C concentrations in the alirocumab and ezetimibe arms from baseline to 52 weeks is shown in **Figure 6**. Mean LDL-C concentrations dropped rapidly in the first 4 weeks, but to a greater degree in the alirocumab arm. In the alirocumab group, LDL-C reduction from baseline was observed from Week 4 and maintained up to Week 52 (LS mean versus baseline at Week 52 of -49.5% versus -50.6% at Week 24) (Table 21).

The distribution of baseline and achieved LDL-C values at 24 weeks are shown in **Figure 7**. The median was 40 mg/dL for alirocumab compared and 70 mg/dL for ezetimibe. Mean achieved LDL-C at week 24 was 51.6 (1.4) mg/dL with alirocumab and 82.5 (2.0) mg/dL with ezetimibe; these differences were maintained to week 52.

The time-course of changes in LDL-C concentrations according to dose-increase status in the alirocumab arm is shown in **Figure 8**. Only 18% percent of the patients in alirocumab group had the dose increased to 150 mg SC Q2W. These patients had much higher baseline LDL-C values versus patients who did not require a dose increase (140.4 [47.4] mg/dL vs. 101.1 [29.7] mg/dL), and the percent reductions in LDL-C achieved at weeks 12 and 24 were lower. The dose increase at 12 weeks led to an additional reduction of 12.4%. Moreover, the absolute reduction in LDL-C by week 24 was slightly greater in the dose-increased group (63.5 vs 56.9 mg/dL) relative to the remainder of the alirocumab group.

For key secondary efficacy endpoints, statistically significant reductions were observed for Apo B (22.4%), Lp(a) (21.7%), and non-HDL-C (22.9%) (all *P*<.0001), and there was an 8.1% increase in HDL-C at week 24 in the alirocumab arm compared with ezetimibe (*P*<.0001) (**Table 22**). Statistically significant difference was not reached with fasting TGs at Week 24 (reduced from baseline by 13.5% in the alirocumab group and 13.3% in the ezetimibe group) and therefore the testing procedure was stopped from this endpoint. Alirocumab efficacy versus ezetimibe was consistent across several subgroups in the ITT population (**Figure 9**). **In Table 22**, the beta-quantification method for determining LDL-C percent change from baseline to week 24 was a sensitivity analysis conducted in 180 patients in the ezetimibe group and 361 patients in the alirocumab group.

### COMBO II: Summary safety results

Rates of treatment-emergent adverse events (TEAEs) over a mean of 58 (19) weeks' follow-up are shown in **Table 25**. The overall percentage of patients who experienced at least one TEAE was 71.2% in the alirocumab arm and 67.2% in the ezetimibe arm. A TEAE leading to death occurred in 2 (0.4%) patients in the alirocumab arm (both of cardiac origin) and in 4 (1.7%) patients in the ezetimibe arm (two of cardiac origin). Similar percentages of subjects in both groups experienced a serious adverse event (18.8% alirocumab vs 17.8% ezetimibe). A higher proportion of patients in the alirocumab group experienced TEAEs leading to treatment discontinuation (7.5% vs 5.4%), with no specific pattern at the preferred term level **(Table 25)**.

There was no indication of imbalance in TEAEs at the system organ class level, with the exception of slightly higher rates with alirocumab for infections and infestations (27.1% vs 25.3% ezetimibe), musculoskeletal and connective tissue disorders (19.6% vs 17.0%), and gastrointestinal disorders (15.0% vs 13.7%) **(Table 25)**. Adjudicated cardiovascular events were also more frequent in the alirocumab group (4.8%, n =23; vs 3.7%, n = 9) due to higher rates of non-fatal myocardial infarctions and coronary revascularizations.

Rates of treatment-emergent local injection site reactions appeared slightly higher in the alirocumab arm (2.5% vs 0.8% for ezetimibe) **(Table 25)**. Reactions were of mild intensity, except for one of moderate intensity and none were serious. Two events led to discontinuation in the alirocumab group. A low percentage of patients in both groups developed myalgia.

Abnormalities in laboratory measurements were uncommon and occurred at similar rates in both groups with the exception of alanine aminotrasaminase and aspartate aminotransferase, which were more frequent in the alirocumab group, and impaired glucose control, which was less frequent in the alirocumab group (**Table 25**).

One-hundred and five (22.8% of 460) patients in the alirocumab arm and none in the ezetimibe arm had two consecutive LDL-C values of <25 mg/dL during the treatment period. Rates of TEAEs in this group were similar to those in the ezetimibe group, with the exception of nasopharyngitis, which was more frequent in the alirocumab group (**Table 25**).

**Table 25 - TEAEs and laboratory parameters (safety population) at 52 weeks by system-organ class and preferred term (including those occurring in patients with two LDL-C values <25 mg/dl alirocumab treatment)**

| All patients on maximal statin therapy ± other lipid-lowering therapy | **Alirocumab 75 mg SC Q2W (n = 479)** | **Ezetimibe 10 mg daily (n = 241)** | **Alirocumab 75 mg SC Q2W With Two Consecutive LDL-C ≤25 mg/dL (n=105)** |
|---|---|---|---|
| Patients experiencing any TEAE | 341 (71.2) | 162 (67.2) | 72 (68.6) |
| TEAEs by system organ class occurring in ≥3% of patients in either group | | | |
| Infections and infestations | 130 (27.1) | 61 (25.3) | 35 (33.3) |
| Musculoskeletal and connective tissue disorders | 94 (19.6) | 41 (17.0) | 19 (18.1) |
| Nervous system disorders | 77 (16.1) | 40 (16.6) | 17 (16.2) |
| Gastrointestinal disorders | 72 (15.0) | 33 (13.7) | 19 (18.1) |
| Injury, poisoning, and procedural complications | 61 (12.7) | 33 (13.7) | 10 (9.5) |
| Cardiac disorders | 60 (12.5) | 29 (12.0) | 14 (13.3) |
| General disorders and administration site conditions | 56 (11.7) | 26 (10.8) | 10 (9.5) |
| Respiratory, thoracic, and mediastinal disorders | 45 (9.4) | 22 (9.1) | 11 (10.5) |
| Investigations | 35 (7.3) | 21 (8.7) | 7 (6.7) |
| Vascular disorders | 33 (6.9) | 20 (8.3) | 4 (3.8) |
| Skin and subcutaneous tissue disorders | 35 (7.3) | 15 (6.2) | 10 (9.5) |
| Metabolism and nutrition disorders | 27 (5.6) | 18 (7.5) | 6 (5.7) |
| Psychiatric disorders | 21 (4.4) | 12 (5.0) | 2 (1.9) |
| Renal and urinary disorders | 21 (4.4) | 13 (5.4) | 7 (6.7) |
| Eye disorders | 23 (4.8) | 9 (3.7) | 2 (1.9) |
| Blood and lymphatic system disorders | 11 (2.3) | 8 (3.3) | 4 (3.8) |
| Neoplasms, benign, malignant and unspecified (including cysts and polyps) | 15 (3.1) | 7 (2.9) | 5 (4.8) |
| Reproductive system and breast disorders | 12 (2.7) | 2 (0.8) | 4 (3.8) |
| TEAEs by preferred term occurring in ≥3% of patients in either group or TEAEs of interest | | | |
| Accidental overdose | 30 (6.3) | 16 (6.6) | 6 (5.7) |
| Upper respiratory tract infection | 31 (6.5) | 14 (5.8) | 4 (3.8) |
| Dizziness | 23 (4.8) | 13 (5.4) | 5 (4.8) |
| Myalgia | 21 (4.4) | 12 (5.0) | 4 (3.8) |
| Headache | 21 (4.4) | 10 (4.1) | 4 (3.8) |
| Arthralgia | 19 (4.0) | 9 (3.7) | 5 (4.8) |
| Nasopharyngitis | 18 (3.8) | 9 (3.7) | 10 (9.5) |
| Hypertension | 18 (3.8) | 10 (4.1) | 3 (2.9) |
| Influenza | 17 (3.5) | 7 (2.9) | 3 (2.9) |
| Angina pectoris | 12 (2.5) | 11 (4.6) | 2 (1.9) |
| Laboratory parameters | | | |
| Blood glucose increased^{c} | 1 (0.2) | 3 (1.2) | 1 (1.0) |
| Hemoglobin decreased^{c} | 4 (0.8) | 2 (0.8) | 3 (2.9) |

### COMBO II: Overall results

In this population of high CV risk patients who had poorly controlled LDL-C on maximally tolerated statin therapy the following observations were made: 1) self-administered alirocumab produced significantly greater LDL-C reductions than ezetimibe after 24 weeks (LS mean difference -29.8%); 2) self-administered alirocumab had good compliance and was well-tolerated; 3) 77% of alirocumab patients achieved LDL-C goal of <0.81 mmol/L (70 mg/dL) at Week 24; LDL-C reductions of -50% vs. baseline maintained to Week 52 with alirocumab; 4) mean LDL-C levels of 1.4 mmol/L (53.3 mg/dL) were achieved at Week 52 with alirocumab; 5) approximately 80% of patients did not require uptitration to alirocumab 150mg Q2W suggesting that 75 mg Q2W may be sufficient for most patients; and 6) TEAEs occurred in a similar frequency in alirocumab and ezetimibe arms.

### Example 3: Long-term safety and tolerability of Alirocumab in high cardiovascular risk patients with hypercholesterolemia not adequately controlled with their lipid modifying therapy: a randomized, double-blind, placebo-controlled study

### INTRODUCTION

This study was undertaken to assess the long-term safety and tolerability of alirocumab in patients at high cardiovascular risk who are not at LDL-C goal. This population that is not at LDL-C goal on optimized LMT represents a highest risk group with a well identified unmet medical need that can be addressed by adding alirocumab to their LDL-C modifying therapies. Two sets of results are reported: (1) a pre-specified interim analysis was performed when all patients reached one year and approximately 25 percent of patients reached 18 months of treatment; and (2) the final analysis of the safety population, when all patients had completed the study.

### STUDY OBJECTIVES

The primary study objective was to evaluate the long-term safety and tolerability of alirocumab in high cardiovascular risk patients with hypercholesterolemia not adequately controlled with their lipid modifying therapy.

The secondary study objectives were: 1) to evaluate the effect of alirocumab on low-density lipoprotein cholesterol (LDL-C) levels after 24 weeks of treatment in comparison with placebo; 2) to evaluate the efficacy of alirocumab on LDL-C levels at other time points; 3) to evaluate the effect of alirocumab on Apolipoprotein B (Apo B), non-high-density lipoprotein cholesterol (non-HDL-C), total cholesterol (total-C), lipoprotein a (Lp [a]), high-density lipoprotein cholesterol (HDL-C), triglyceride (TG) levels, and apolipoprotein A-1 (Apo A-1) after 24 weeks of treatment and at other time points in comparison with placebo; 4) to evaluate the development of anti-alirocumab antibodies; and 5) to evaluate the pharmacokinetics (PK) of alirocumab.

### STUDY DESIGN

This was a randomized, double-blind, placebo-controlled, unbalanced (2:1, alirocumab:placebo), parallel-group, multi-center, multi-national study evaluating the long-term safety and tolerability of alirocumab in high cardiovascular risk patients with hypercholesterolemia who were not adequately controlled with a maximally tolerated daily registered dose of a statin with or without other lipid modifying therapy. See **Figure 10**. Patients were stratified according to heFH population, prior history of MI or ischemic stroke, statin treatment and geographic region. Patients at high cardiovascular risk were defined as 1) having heFH (who may or may not have CHD/CHD risk equivalents) or 2) no prior diagnosis of heFH but having hypercholesterolemia together with established CHD or CHD risk equivalents. Patients must be hypercholesterolemic and not adequately controlled (i.e., LDL-C ≥ 70 mg/dL [≥1.81 mmol/L]) despite therapy with maximally tolerated daily registered dose of a statin with or without other lipid modifying therapy at a stable dose for at least 4 weeks (6 weeks for fenofibrate) prior to screening.

### Description of the protocol

Patients randomized to alirocumab received 150 mg every 2 weeks. The treatment scheme could be adjusted, if needed, during the course of the study, through a protocol amendment, to introduce a lower dose with a titration scheme.

The study consisted of 3 periods: screening, double-blind treatment, and follow up. The screening period was up to 3 weeks in duration including an intermediate visit during which the patient (or another designated person such as spouse, relative, etc.) was trained to self-inject/inject with placebo. Eligibility assessments were performed to permit the randomization of the patients into the study. The double-blind treatment period was a randomized, double-blind study treatment period of 18 months. The first injection during the double-blind period was done at the site on the day of randomization and as close as possible after randomization into the study. The subsequent injections were done by the patient (self-injection), or another designated person (such as spouse, relative, etc.) at a patient-preferred location (home...). The follow-up period was a period of 8 weeks after the end of the double-blind treatment period.

Patients who achieved 2 consecutive calculated LDL-C levels < 25 mg/dL (0.65 mmol/L) during the study were monitored and managed.

Statin and other lipid modifying therapy (if applicable) should be stable (including dose) during the first 24 weeks of the double-blind treatment period barring exceptional circumstances whereby overriding concerns (including but not limited to triglyceride alert posted by the central lab) warrant such changes, as per the investigator's judgment. At Week 24 onwards, background lipid modifying therapy could be modified only under certain conditions.

Patients were to be on a stable diet (NCEP-ATPIII TLC diet or equivalent) throughout the entire study duration from screening. The dietician or site staff with appropriate training reviewed the patient's diet at the screening visit and periodically throughout the study. **Table 26** provides a summary of TLC Diet for high cholesterol.

**Table 26**

| | |
|---|---|
| Total Fat | 25% - 35% total calories* |
| Saturated fat* | < 7% total calories |
| Polyunsaturated fat | up to 10% total calories |
| Monounsaturated fat | up to 20% total calories |
| Carbohydrates† | 50% - 60% total calories* |
| Protein | ∼15% total calories |
| Cholesterol | < 200 mg/day (5.172 mmol/†day) |
| Plant Sterols | 2g |
| Soluble Fiber such as psyllium | 10g - 25g |

| | |
|---|---|
| * ATP III allows an increase of total fat to 35 percent of total calories and a reduction in carbohydrate to 50 percent for persons with the metabolic syndrome. Any increase in fat intake should be in the form of either polyunsaturated or monounsaturated fat. Trans fatty acids are another LDL-raising fat that should be kept at a low intake. †Carbohydrate should derive predominantly from foods rich in complex carbohydrates including grains-especially whole grains-fruits, and vegetables. | |

Patients with cardiovascular events from randomization until the final follow up visit had an adjudication package prepared and sent to the independent Clinical Events Committee.

Patients underwent color vision testing periodically throughout the study. An ophthalmologic sub-study was conducted in a sub-set of the study population.

### Duration of study participation

The study duration included up to 3 weeks of screening period, 18 months of double-blind study treatment period, and 8 weeks of follow up period. Thus, the study duration per patient was approximately 20 months. The end of the study per patient was the last protocol planned visit or the resolution/stabilization of all SAEs, and AESI, whichever came last. The end of the study was defined as being the last visit of the follow-up period for each patient.

### SELECTION OF PATIENTS

The study was designed to randomize approximately 2100 patients in a 2:1 randomization ratio to the following: Alirocumab - approximately 1400 patients; placebo - approximately 700 patients.

Patients meeting the following criteria were considered for enrollment into the study: 1) patients with heterozygous Familial Hypercholesterolemia (heFH)* with or without established coronary heart disease (CHD) or CHD risk equivalents who are not adequately controlled with a maximally tolerated stable daily dose of statin** for at least 4 weeks prior to the screening visit (Week -3) with or without other lipid modifying therapy (LMT); or 2) patients with hypercholesterolemia and established CHD or CHD risk equivalents (see below for definitions) who are not adequately controlled with a maximally tolerated stable daily dose of statin** for at least 4 weeks prior to the screening visit (Week -3) with or without other lipid modifying therapy (LMT).

*Diagnosis of heFH must be made either by genotyping or by clinical criteria. For those patients not genotyped, the clinical diagnosis may be based on either the WHO criteria/Dutch Lipid Clinical Network criteria with a score > 8 points or the Simon Broome register diagnostic criteria with a criterion for definite FH.

** Definition of maximally tolerated dose (any of the following are acceptable): 1) rosuvastatin 20 mg or 40 mg daily; 2) atorvastatin 40 mg or 80 mg daily; 3) simvastatin 80 mg daily (if already on this dose for >1 year); 4) patients not able to be on any of the above statin doses, should be treated with the dose of daily atorvastatin, rosuvastatin or simvastatin which is considered appropriate for the patient as per the investigator's judgment or concerns. Some examples of acceptable reasons for a patient taking a lower statin dose include, but are not limited to: adverse effects on higher doses, advanced age, low body mass index, regional practices, local prescribing information, concomitant medications, co-morbid conditions such as impaired glucose tolerance/impaired fasting glucose. The reason(s) had to be documented.

Documented history of CHD includes one or more of the following: i) acute myocardial infarction (MI); ii) silent myocardial infarction; iii) unstable angina; iv) coronary revascularization procedure (eg, percutaneous coronary intervention [PCI] or coronary artery bypass graft surgery [CABG]); and/or v) clinically significant CHD diagnosed by invasive or non-invasive testing (such as coronary angiography, stress test using treadmill, stress echocardiography or nuclear imaging).

CHD risk equivalents includes one or more of the following 4 criteria: i) Documented peripheral arterial disease (one of the following criteria [a, b, or c] must be satisfied): current intermittent claudication (muscle discomfort in the lower limb that is both reproducible and produced by exercise and relieved by rest within 10 minutes) of presumed atherosclerotic origin together with ankle-brachial index < 0.90 in either leg at rest, or b) history of intermittent claudication (muscle discomfort in the lower limb that is both reproducible and produced by exercise and relieved by rest within 10 minutes) together with endovascular procedure or surgical intervention in one or both legs because of atherosclerotic disease or c) history of critical limb ischemia together with thrombolysis, endovascular procedure or surgical intervention in one or both legs because of atherosclerotic disease; ii) Documented previous ischemic stroke with a focal ischemic neurological deficit that persisted more than 24 hours, considered as being of atherothrombotic origin. CT or MRI must have been performed to rule out hemorrhage and non-ischemic neurological disease; iii) Documented moderate chronic kidney disease (CKD) as defined by 30 ≤ eGFR <60 mL/min/1.73 m² for 3 months or more, including the screening visit; iv) Known history of diabetes mellitus AND 2 or more additional risk factors (as listed below): *a* history of hypertension (established on antihypertensive medication), *b* Documented history of ankle-brachial index ≤0.90, *c* Documented history of microalbuminuria or macroalbuminuria OR dipstick urinalysis at screening visit (Week-3) with >2+ protein, d Documented history of pre-proliferative or proliferative retinopathy or laser treatment for retinopathy, e Known family history of premature CHD (CHD in father or brother before 55 years of age; CHD in mother or sister before 65 years of age).

According to the Simon Broome Register Diagnostic Criteria for Heterozygous Familial Hypercholesterolemia, definite familial hypercholesterolemia is defined as: Total-C >6.7 mmol/l (260 mg/dL) or LDL cholesterol above 4.0 mmol/l (155 mg/dL) in a child <16 years or Total-C >7.5 mmol/l (290 mg/dL) or LDL cholesterol above 4.9 mmol/l (190 mg/dL) in an adult. (Levels either pre-treatment or highest on treatment) PLUS Tendon xanthomas in patient, or in 1st degree relative (parent, sibling, child), or in 2nd degree relative (grandparent, uncle, aunt) OR DNA-based evidence of an LDL receptor mutation or familial defective apo B-100.

According to the Simon Broome Register Diagnostic Criteria for Heterozygous Familial Hypercholesterolemia, possible familial hypercholesterolemia is defined as: Total-C >6.7 mmol/l (260 mg/dL) or LDL cholesterol above 4.0 mmol/l (155 mg/dL) in a child <16 years or Total-C >7.5 mmol/l (290 mg/dL) or LDL cholesterol above 4.9 mmol/l (190 mg/dL) in an adult. (Levels either pre-treatment or highest on treatment) and at least one of the following: family history of myocardial infarction below 50 years of age in 2nd degree relative or below 60 years of age in 1st degree relative, or family history of raised cholesterols >7.5 mmol/l (290 mg/dL) in adult 1st or 2nd degree relative or >6.7 mmol/l (260 mg/dL) in child or sibling under 16 years of age.

The WHO Criteria (Dutch Lipid Network clinical criteria) for diagnosis of Heterozygous Familial Hypercholesterolemia (heFH) is set forth in **Table 27**.

**Table 27**

| **Diagnostic Scoring for Heterozygous Familial Hypercholesterolemia** | | | |
|---|---|---|---|
| Family history | | | |
| a First degree relative with known premature (men <55 yrs, women <60 yrs) coronary and | | | 1 |
| b First degree relative with known LDL-cholesterol >95th percentile for age and sex. | | | |

| and/or | | | |
|---|---|---|---|
| a First degree relative with tendon xanthomata and/or arcus cornealis. | | | 2 |
| b Children below 18 yrs. with LDL-cholesterol >95th percentile for age | | | |
| | | | |

| Clinical history | | | |
|---|---|---|---|
| a Patient has premature (men <55 yrs, women <60 yrs) coronary artery | | | 2 |
| b Patient has premature (men <55 yrs, women <60 yrs) cerebral or | | | 1 |
| | | | |

| Physical examination | | | |
|---|---|---|---|
| a Tendon xanthomata | | | 6 |
| b Arcus cornealis below the age of 45 yrs. | | | 4 |
| | | | |

| Laboratory analysis | | | |
|---|---|---|---|
| | mmol/L | mg/dL | |
| a LDL-cholesterol | >8.5 | >330 | 8 |
| b LDL-cholesterol | 6.5-8.4 | 250- | 5 |
| c LDL-cholesterol | 5.0-6.4 | 190- | 3 |
| d LDL-cholesterol | 4.0-4.9 | 155- | 1 |

| (HDL-cholesterol and triglycerides are normal) | | | |
|---|---|---|---|
| | | | |

| DNA-analysis | | | |
|---|---|---|---|
| a Functional mutation low-density lipoprotein receptor gene present | | | 8 |
| | | | |
| Diagnosis of heFH is: | | | |
| Certain When | | >8 points | |
| Probable When | | 6-8 points | |
| Possible When | | 3-5 points | |

Patients who met all the above inclusion criteria were screened for the following exclusion criteria.

Exclusion criteria related to study methodology were: 1) Without established history of CHD or CHD risk equivalents or without a diagnosis of heFH based on genotyping or clinical criteria; 2) LDL-C <70 mg/dL (<1.81 mmol/L) at the screening visit (Week-3); 3) Not on a stable dose of LMT (including statin) for at least 4 weeks and/or fenofibrate for at least 6 weeks, as applicable, prior to the screening visit (Week -3) and from screening to randomization; 4) Currently taking a statin that is not simvastatin, atorvastatin, or rosuvastatin; 5) Simvastatin, atorvastatin, or rosuvastatin is not taken daily or not taken at a registered dose; 6) Daily doses above atorvastatin 80 mg, rosuvastatin 40 mg or simvastatin 40 mg (except for patients on simvastatin 80 mg for more than one year, who are eligible); 7) Use of fibrates other than fenofibrate within 6 weeks prior to screening visit (Week -3) or between screening and randomization visits; 8) Use of nutraceutical products or over-the-counter therapies that may affect lipids which have not been at a stable dose for at least 4 weeks prior to the screening visit (Week -3) or between screening and randomization visits; 9) Use of red yeast rice products within 4 weeks of the screening visit (Week-3) or between screening and randomization visits; 10) Patient who has received plasmapheresis treatment within 2 months prior to the screening visit (Week -3), or has plans to receive it; 11) Recent (within 3 months prior to the screening visit [Week -3] or between screening and randomization visits) MI, unstable angina leading to hospitalization, uncontrolled cardiac arrhythmia, CABG, PCI, carotid surgery or stenting, cerebrovascular accident, transient ischemic attack (TIA), endovascular procedure or surgical intervention for peripheral vascular disease; 12) Planned to undergo scheduled PCI, CABG, carotid or peripheral revascularization during the study; 13) History of New York Heart Association (NYHA) Class III or IV heart failure within the past 12 months; 14) Systolic blood pressure >180 mmHg or diastolic blood pressure >110 mmHg at screening visit or randomization visit; 15) Known history of hemorrhagic stroke; 16) Age < 18 years or legal age of majority at the screening visit (Week-3), whichever is greater; 17) Known history of active optic nerve disease; 18) Patients not previously instructed on a cholesterol-lowering diet prior to the screening visit (Week-3); 19) Known history of homozygous FH; 20) Known history of loss of function of PCSK9 (ie, genetic mutation or sequence variation); 21) Use of systemic corticosteroids, unless used as replacement therapy for pituitary/adrenal disease with a stable regimen for at least 6 weeks prior to randomization. Note: topical, intra-articular, nasal, inhaled and ophthalmic steroid therapies are not considered as "systemic" and are allowed; 22) Use of continuous hormone replacement therapy unless the regimen has been stable in the past 6 weeks prior to the Screening visit (Week-3) and no plans to change the regimen during the study; 23) History of cancer within the past 5 years, except for adequately treated basal cell skin cancer, squamous cell skin cancer, or in situ cervical cancer; 24) Known history of HIV positivity; 25) Conditions/situations such as: a) Any clinically significant abnormality identified at the time of screening that in the judgment of the Investigator or any sub-Investigator would preclude safe completion of the study or constrain endpoints assessment such as major systemic diseases, patients with short life expectancy, b) Patients considered by the Investigator or any sub-Investigator as inappropriate for this study for any reason, eg.: i) Those deemed unable to meet specific protocol requirements, such as scheduled visits; ii) Those deemed unable to administer or tolerate long-term injections as per the patient or the investigator; iii) Investigator or any sub-Investigator, pharmacist, study coordinator, other study staff or relative thereof directly involved in the conduct of the protocol, etc; iv) Presence of any other conditions (eg, geographic, social....) actual or anticipated, that the Investigator feels would restrict or limit the patient's participation for the duration of the study; 26) Patient who has been previously treated with at least one dose of alirocumab or any other anti-PCSK9 monoclonal antibody in other clinical trials; 27) Patient who has taken any investigational drugs other than alirocumab training placebo kits within 1 month or 5 half lives, whichever is longer; 28) Patient who withdraws consent during the screening period (patient who is not willing to continue or fails to return); 29) Laboratory findings during the screening period (not including randomization labs): A) Positive test for Hepatitis B surface antigen and/or Hepatitis C antibody (confirmed by reflexive testing), B) Triglycerides (TG) >400 mg/dL (>4.52 mmol/L) (1 repeat lab is allowed); C) Positive serum or urine pregnancy test in women of childbearing potential; D) eGFR <30 mL/min/1.73 m² according to 4-variable MDRD equation; E) HbA_{1c} >10%; F) ALT or AST >3 x ULN (1 repeat lab is allowed); G) CPK >3 x ULN (1 repeat lab is allowed).

Physicians often assess the stage of heart failure according to the New York Heart Association (NYHA) functional classification system. This system relates symptoms to everyday activities and the patient's quality of life, as shown in **Table 28**.

**Table 28**

| **Class** | **Patient Symptoms** |
|---|---|
| Class I (mild) | No limitation of physical activity. Ordinary physical activity does not cause undue fatigue, palpitation, or dyspnea (shortness of breath). |
| Class II (mild) | Slight limitation of physical activity. Comfortable at rest, but ordinary physical activity results in fatigue, palpitation, or dyspnea. |
| Class III (moderate) | Marked limitation of physical activity. Comfortable at rest, but less than ordinary activity causes fatigue, palpitation, or dyspnea. |
| Class IV (severe) | Unable to carry out any physical activity without discomfort. Symptoms of cardiac insufficiency at rest. If any physical activity is undertaken, discomfort is increased. |

Exclusion criteria related to the active comparator and/or mandatory background therapies were: 30) all contraindications to the background therapies or warning/precaution of use (when appropriate) as displayed in the respective National Product Labeling.

Exclusion criteria related to the current knowledge of alirocumab were: 31) Known hypersensitivity to monoclonal antibody therapeutics; 32) Pregnant or breast-feeding women; 33) Women of childbearing potential not protected by highly-effective method(s) of birth control and/or who are unwilling or unable to be tested for pregnancy. Note: Women of childbearing potential must have a confirmed negative serum pregnancy test at screening and urine pregnancy test at randomization visit. They must use effective contraceptive methods throughout the study and agree to repeat urine pregnancy test at designated visits. The applied methods of contraception have to meet the criteria for a highly effective method of birth control according to the "Note for guidance on non-clinical safety studies for the conduct of human clinical trials for pharmaceuticals (CPMP/ICH/286/95)". Postmenopausal women must be amenorrheic for at least 12 months.

### STUDY TREATMENTS

Sterile alirocumab drug product (IMP) was supplied at a concentration of 150 mg/mL as 1 mL volume filled into syringe. During the double-blind treatment period, alirocumab or placebo was administered subcutaneously as 1 mL injection every 2 weeks, starting at Week 0 continuing up to the last injection (ie, Week 76) which was at 2 weeks before the end of the double blind treatment period. IMP should have ideally been administered every two weeks subcutaneously at approximately the same time of the day, however it was acceptable to have a window period of ± 3 days.

The following classes of drugs were identified as non-investigational medicinal products (NIMP) because the medication was either a background therapy or a potential rescue medication: statins (rosuvastatin, atorvastatin, simvastatin); cholesterol absorption inhibitors (ezetimibe); bile acid-binding sequestrants (such as cholestyramine, colestipol, colesevelam); nicotinic acid; fenofibrate; omega-3 fatty acids (≥ 1000 mg daily).

Patients were randomized to receive either placebo or alirocumab during the double-blind study treatment period. The randomization ratio alirocumab:placebo was 2:1. The randomization was stratified by heFH population (Yes, No), prior history of acute or silent MI or ischemic stroke (Yes, No), statin treatment (atorvastatin 40 to 80 mg daily or rosuvastatin 20 to 40 mg daily, vs. simvastatin whatever the daily dose, atorvastatin below 40 mg daily or rosuvastatin below 20 mg daily) and region (North America, Western Europe, Eastern Europe and Rest of World).

A concomitant medication was any treatment received by the patient concomitantly to the study (until follow-up visit). Concomitant medications were to be kept to a minimum during the study. However, if these were considered necessary for the patient's welfare and unlikely to interfere with the IMP, they could given at the discretion of the Investigator, with a stable dose (when possible). Besides the specific information related to concomitant medications provided in this section, any other concomitant medication(s) will be allowed and will have to be recorded. If the patient had an LDL-C ≥160 mg/dL (4.14 mmol/L) at the screening visit (Week -3) and was treated with a statin only, ie, without additional LMT, the investigator had to report the reason for the patient not being on a second LMT. Nutraceutical products or over-the-counter therapies that may affect lipids were allowed only if they wereused at a stable dose for at least 4 weeks prior to screening visit, during the screening period and maintained during the first 24 weeks of the double-blind treatment period. After the Week 24 visit, modification to these nutraceutical products or over-the-counter therapieswais allowed but in general should be avoided. Examples of such nutraceutical products or over-the-counter therapies included omega-3 fatty acids at doses <1000 mg, plant stanols such as found in Benecol, flax seed oil, and psyllium.

Patients were on maximally tolerated daily registered doses of statins with or without other lipid modifying therapy during the study. From the screening visit (Week -3) until the first 24 weeks of the double-blind treatment period (Week 24), the background lipid modifying therapy should not be changed. No dose adjustment, discontinuation or initiation of other statins or other lipid modifying therapy should take place during this time, barring exceptional circumstances whereby overriding concerns (including but not limited to triglyceride alert posted by the central lab) warrant such changes, as per the investigator's judgment.

The following therapies were not allowed during the study (including the screening period until the follow-up visit): fibrates other than fenofibrate; red yeast rice products; and statin other than simvastatin, atorvastatin, or rosuvastatin.

### SAFETY ASSESSMENT

Safety was assessed by the following parameters: recording of adverse events (including adjudicated cardiovascular events); standard laboratory tests (hematology, chemistry and urinalysis); liver panel (ALT, AST, Alkaline Phosphatase [ALP], and total bilirubin); creatine phospho kinase (CPK); hepatitis C Antibody (if positive, then confirmed with reflexive testing); vitamin E (alpha-tocopherol) and other fat soluble vitamins; cortisol (with reflexive ATCH levels, as needed, and followed by ACTH stimulation test, as needed); gonadal hormone assessments; electrocardiogram (ECG); vital signs (systolic and diastolic blood pressure and heart rate); physical examination (including neurological exam); color vision test (as a screening test for more comprehensive ophthalmological testing, as needed). Safety parameters (adverse events [including adjudicated cardiovascular events], laboratory data, vital signs, and ECG) were assessed throughout the study.

Patients who achieve 2 consecutive calculated LDL-C <25 mg/dL (0.65 mmol/L) will be monitored.

Patients who have a titer at or above 240 for anti-alirocumab antibody at the Follow Up visit will have additional antibody sample(s) at 6 to 12 months after the last dose and thereafter, about every 3 to 6 months until titer returns below 240.

Safety endpoints assessed in this trial were: cardiovascular events; allergic events; local tolerability at injection site; other adverse events (including hemolytic anemia); laboratory tests: urinalysis, hematology (red blood cell count, red blood cell distribution width (RDW), reticulocyte count, hemoglobin, hematocrit, platelets, white blood cell count with differential blood count), standard chemistry (sodium, potassium, chloride, bicarbonate, calcium, phosphorous, urea nitrogen, creatinine, uric acid, total protein, albumin, LDH, γ Glutamyl Transferase [yGT]), Vitamin E (alpha tocopherol) and other Fat Soluble Vitamins, cortisol (and reflexive ACTH levels, as needed, followed by ACTH stimulation test, as needed), Gonadal Hormone Assessments, Hepatitis C antibody, liver panel (ALT, AST, ALP, and total bilirubin), and CPK; vital signs including heart rate and blood pressure; and 12-lead ECG.

### STUDY PROCEDURES

For all visits after Day 1/Week 0 (randomization visit), a timeframe of a certain number of days was allowed. The window period for visits at Weeks 12 and 24 were ± 3 days, at Week it was 52 ± 5 days, and for all other site visits it was ± 7 days during the double-blind treatment period, and follow-up period. A window period of +3 days was allowed for the randomization visit (Day1/Week 0) and ± 7 days was allowed for the screening visit for injection training (Week -1). A window period of ± 7 days was allowed for all phone call visits. For all visits after Day 1/randomization visit, if one visit date was changed, then the next visit took place according to the original schedule.

### OPHTHALMOLOGIC SUB-STUDY

Despite evidence that ocular abnormalities noted during toxicology studies in rats were not due to the administration of alirocumab, thorough ophthalmic evaluations were conducted in a sub-set of patients in the protocol to verify the ophthalmic safety of alirocumab.

The primary objective was to assess the clinical ophthalmic safety of alirocumab in high cardiovascular risk patients with hypercholesterolemia not adequately controlled with their LMT.

Patients underwent ophthalmologic assessments by ophthalmologists/optometrists at the screening period. If the patients were deemed eligible for the main study trial and fulfilled the additional eligibility criteria for the sub-study, then the patients underwent ophthalmologic assessments every 6 months during the 18-month double-blind treatment period

The primary endpoint was adverse events related to ophthalmologic abnormalities (using SMQ "optic nerve disorders", "retinal disorders" and "corneal disorders") over the study duration.

Approximately 270 patients were expected to participate in this sub-study. All subjects must meet eligibility criteria for the main study and the additional eligibility criteria outlined for this sub-study in order to be eligible for this sub-study.

The only additional inclusion criteria was signed written informed consent.

Additional exclusion criteria for the sub-study were: patient who withdraws consent for Ophthalmologic Sub-study during the screening period (patient who is not willing to continue); patient unable to complete all of the ophthalmologic assessments during the screening period; patient with active optic nerve disease (eg, optic neuritis) detected during the ophthalmologic assessments at the screening period. Note - Patient with controlled open-angle glaucoma may be eligible for the study; and patient with active chorio-retinal disease detected during the ophthalmologic assessments at the screening period.

### Statistical methods:

### Sample size determination:

For safety assessment, a sample size of 2100 patients (randomization ratio 2:1, i.e., alirocumab:1400 and placebo:700) allows the collection of long-term safety data in a broad database (at least 1000 patients exposed to alirocumab for a minimum of 12 months, of which approximately 900 patients exposed to alirocumab for 18 months).

With this sample size and the following assumptions: duration of recruitment of 16 months, 44% of patients recruited in the first 9 months, and a dropout rate of 25% and 35% at 12 months and 18 months, respectively, the expected number of patients exposed to alirocumab for a minimum of 12 months and 18 months of treatment is given below:

Expected number of patients exposed to alirocumab according to timing of analysis:

| Analysis | Patients exposed ≥12 months | Patients exposed 18 months |
|---|---|---|
| First step analysis ^{a} | 970 patients | 400 patients |
| Final analysis | 1050 patients | 910 patients |

| | | |
|---|---|---|
| ^{a} First step analysis planned when approximately 600 patients have completed 18 months of the double-blind treatment period | | |

Moreover, a sample size of 1400 patients treated with alirocumab allows detecting adverse event with a rate ≥0.002 with 95% confidence in the alirocumab group. It was anticipated that between 122 and 142 patients exposed to alirocumab would be evaluated in the ophthalmologic sub-study for at least 12 months considering a sample size of 270 patients and assuming a discontinuation rate of 25% over 12 months. This would allow detection of an ophthalmological event with a true occurrence between 0.021 and 0.024 in the alirocumab group, with 95% confidence.

### Timing of analyses:

The first step analysis included final efficacy endpoints up to Week 52 (including primary efficacy endpoint at Week 24) and interim safety analysis, which was performed on all safety data up to the common study cut-off date (date when approximately 600 randomized patients had completed 18 months of the double-blind treatment period). Analysis of lipid data beyond Week 52 was descriptive. Results are presented herein.

The second step (final) analysis will be conducted at the end of the study and will consist of the final analysis of efficacy data at time points beyond time points analyzed at first step analysis and final safety analysis.

### Analysis populations:

The primary efficacy analysis population was the intent-to-treat (ITT) population, defined as all randomized patients who had an evaluable primary efficacy endpoint, that is, those with an available baseline calculated LDL-C value, and at least one available calculated LDL-C value within one of the analysis windows up to Week 24 (including all calculated LDL-C on-treatment and off-treatment).

The secondary efficacy analysis population was the modified intent-to-treat (mITT) population, defined as all randomized patients who took at least one dose or part of a dose of the double-blind investigational medicinal product (IMP) and who had an available calculated LDL-C value at baseline and at least one available calculated LDL-C value within one of the analysis windows up to Week 24 during the efficacy treatment period. The efficacy treatment period was defined as the time from the first double-blind IMP administration up to 21 days after the last double-blind injection.

The safety population included all randomized patients who received at least one dose or part of a dose of the double-blind IMP.

### Efficacy analyses:

Primary analyses of efficacy endpoints were conducted using an ITT approach (based on the ITT population defined above), including all lipid data, regardless of whether the patient was continuing therapy or not. This corresponds to ITT estimands, defined for primary and key secondary endpoints. In addition, analyses were also conducted using an on-treatment approach (based on the mITT population defined above), including lipid data collected during the efficacy treatment period. This corresponds to on-treatment estimands of key secondary endpoints.

The ITT approach analyzed all patients, irrespective of their adherence to the treatment; it assessed the benefit of the treatment strategy and reflected as much as possible the effect in a population of patients. The on-treatment approach analyzed the effect of treatment, restricted to the period during which patients actually received the treatment. It assessed the benefit that a treatment would achieve in patients adherent to treatment up to the considered time point.

Efficacy analyses were performed according to treatment as-randomized.

All measurements, scheduled or unscheduled, fasting or not fasting, were assigned to analysis windows in order to provide an assessment from Week 4 to Week 78 time points.

With regards to the primary efficacy analysis (ITT approach), the percent change in calculated LDL-C from baseline to Week 24 was analyzed using a mixed-effect model with repeated measures (MMRM) approach. All post-baseline data available from Week 4 to Week 52 analysis windows were used and missing data were accounted for by the MMRM. The model included the fixed categorical effects of treatment group (placebo versus alirocumab), randomization strata (as per IVRS), time point (Week 4 to Week 52), treatment-by-time point interaction and strata-by-time point interaction, as well as, the continuous fixed covariates of baseline LDL-C value and baseline value-by-time- point interaction. This model provided baseline adjusted least-squares means (LSmeans) estimates at Week 24 for both treatment groups with their corresponding SEs and 95% confidence intervals. To compare the alirocumab to the placebo group, an appropriate contrast statement was used to test the differences of these estimates at the 5% alpha level.

A hierarchical procedure has been defined to test key secondary endpoints while controlling for multiplicity (using above order of key secondary endpoints). The first key secondary endpoint was the percent change in calculated LDL-C from baseline to Week 24 using an on-treatment approach.

Continuous secondary variables anticipated to have a normal distribution (i.e., lipids other than TGs and Lp(a)) were analyzed using the same MMRM model as for the primary endpoint. Continuous endpoints anticipated to have a non-normal distribution (i.e., TG and Lp(a)) were analyzed using multiple imputation approach for handling of missing values followed by robust regression model with endpoint of interest as response variable using M-estimation (using SAS ROBUSTREG procedure) with treatment group, randomization strata (as per IVRS) and corresponding baseline value(s) as effects to compare treatment effects. Combined estimate for mean in both treatment groups, as well as the differences of these estimates, with their corresponding SEs, 95% CIs and p-value were provided (through SAS MIANALYZE procedure).

Binary secondary efficacy endpoints were analyzed using multiple imputation approach for handling of missing values followed by stratified logistic regression with treatment group as main effect and corresponding baseline value(s) as covariate, stratified by randomization factors (as per IVRS). Combined estimates of odds ratio versus placebo, 95% Cl, and p-value were provided (through SAS MIANALYZE procedure).

### Safety analyses:

Safety analyses were descriptive, performed on the safety population according to treatment actually received. The safety analysis focused on the TEAE period defined as the time from the first dose of double-blind injection up to 70 days after the last double-blind injection. TEAE which developed, worsened or became serious or PCSA occurring after the patient's first injection in the open-label extension study (LTS13643) were not considered in the TEAE period. TEAE period was truncated at the common study cut-off date.

### RESULTS I- PRE-SPECIFIED ANALYSIS

### Patient accountability

A pre-specified interim analysis was performed when all patients reached one year and approximately 25 percent of patients reached 18 months of treatment. Of the 2341 randomized patients, three patients were not treated and were not therefore included in the safety population.

Thirty-one randomized patients were not included in the ITT population (No LDL-C value within one of the analysis windows up to week 24).

Forty-one randomized patients were excluded from the mITT population (patients excluded from the ITT population and patients with no LDL-C value within one of the analysis windows up to week 24 during the efficacy treatment period).

**Table 29- Analysis populations**

| | **Placebo** | **Alirocumab 150 Q2W** | **All** |
|---|---|---|---|
| Randomized population | 788 (100%) | 1553 (100%) | 2341 (100%) |
| Efficacy populations Intent-to-Treat (ITT) | 780 (99.0%) | 1530 (98.5%) | 2310 (98.7%) |
| Modified Intent-to-Treat (mITT) | 777 (98.6%) | 1523 (98.1%) | 2300 (98.2%) |
| Safety population | 788 | 1550 | 2338 |

| | | | |
|---|---|---|---|
| Note: The safety population patients are tabulated according to treatment actually received (as treated). For the other populations, patients are tabulated according to their randomized treatment. | | | |

### Study disposition

At the cut-off date in this pre-specified analysis, 607 (25.9%) patients have completed the 18-months double-blind treatment period (defined as an exposure of at least 76 weeks and a Week 78 visit performed), including at least 400 patients in the alirocumab group, as agreed with Health Authorities during "End of Phase 2" meeting consultation: 405 patients (26.1%) in the alirocumab group and 202 patients (25.6%) in the placebo group.

In the alirocumab group, 1550 of the 1553 actually received alirocumab. 23% (n=349) of these patients completed 78 weeks, 20% (n=311) discontinued treatment, and 57% (n=890) are still receiving treatment. Alirocumab-group patients that discontinued treatment did so because of poor compliance to the protocol (n = 54), an adverse event (n = 98), or another reason (n = 159). The ITT safety populations for the alirocumab group were 1530 and 1550 patients, respectively. In the placebo group, all 788 received placebo. 22% (n=176) of these patients completed 78 weeks, 19% (n=146) discontinued treatment and 59% (n=466) are still receiving treatment. Placebo-group patients that discontinued treatment did so because of poor compliance to the protocol (n = 34), an adverse event (n = 44), or another reason (n = 67). The ITT safety populations for the alirocumab group were 780 and 788 patients, respectively. Patients in the ITT population were included in all efficacy endpoints up to Week 52. Patients in the safety population received either alirocumab or placebo for at least 52 weeks.

Study disposition, exposure and safety analyses were assessed using all data up to the study common cut-off date and therefore include data beyond week 52 and up to week 78 or up to the Follow-up visit. Final results for primary efficacy endpoint (at Week 24) and key secondary efficacy endpoints (assessed up to Week 52) are provided in this first step analysis.

There were in total 525 (22.4%) randomized patients who completed the study treatment period up to the cut-off date in this pre-specified analysis, i.e. last IMP injection was taken (Week 76) and end of treatment visit (Week 78) was performed within 21 days after the last IMP injection and at least 525 days after randomization.

The double-blind IMP was prematurely discontinued for 455 (19.4%) randomized patients (310 (20.0%) patients in the alirocumab group and 145 (18.4%) patients in the placebo group). For 1358 (58.0%) randomized patients, treatment was ongoing at the time of the first-step analysis cut-off date.

Overall, the main reasons for study treatment discontinuation were "other reasons - other" and "adverse event": These "other reasons" included the following: subject refusal to continue (93 patients), patient did not meet eCRF criteria for completion of treatment due to either missed/out of window week 78 visit (71 patients), death (6 patients) (there was no decision of investigator to stop the treatment prior to death), miscellaneous (5 patients), lost-to-follow-up (4 patients), site closure (3 patients) and subject moved (2 patients).

In addition, among these 455 patients who prematurely discontinued, 219 (14.1%) randomized patients had prematurely discontinued the double-blind IMP before the Week 52 visit in the alirocumab group and 111 (14.1%) in the placebo group.

Finally, 139 (5.9%) patients participated in the optional ophthalmologic sub-study (instead of the planned 270 patients).

In this first step analysis, final results are available for primary efficacy endpoint at Week 24 and key secondary efficacy endpoints assessed at Week 12, Week 24 and Week 52. At week 24, the primary efficacy endpoint was available for 1384 (90.5%) in the alirocumab group and 708 (90.8%) in the placebo group.

The primary efficacy endpoint was missing for 218 patients. At the week 24 visit, the reasons for missingness were as follows: 108 samples not done due to earlier study discontinuation; 47 samples done outside analysis windows; 10 samples not done; 51 samples done but measurement could not be done (insufficient quantity, TGs>400 mg/dL (4.52 mmol/L) ...); and 2 samples not done for patients randomized but not treated.

### Demographics and baseline characteristics

A total of 2341 patients were randomized in the study (1553 in alirocumab versus 788 in placebo group). Demographics characteristics, disease characteristics and lipid parameters at baseline were similar in the alirocumab group as compared to the placebo group. For the alirocumab group (N=1553), the age (mean years (SD)) was 60.4 (10.4), the percentage of males was 63.3% (N=983), the percentage white race was 92.8% (N=1441), and the BMI (mean kg/m² (SD)) was 30.2 (5.7). For the placebo group (N=788), the age (mean years (SD)) was 60.6 (10.4), the percentage of males was 60.2% (N=474), the percentage white race was 92.6% (N=730), and the BMI (mean kg/m² (SD)) was 30.5 (5.5).

17.7% of patients (17.8% (N=276) of the alirocumab patients and 17.6% (N=139) of the placebo group) had heFH with or without established CHD or CHD risk equivalents. 82.3% of patients were enrolled with non-familial hypercholesterolemia with established CHD or CHD risk equivalents. A large majority of the randomized population (90.6%) had a history of Coronary Heart Disease (CHD) or CHD risk equivalents. CHD was reported for 68.6% of patients. 91.5% of the patients were classified as very high risk (see the note below).

Demographics characteristics, disease characteristics and lipid parameters at baseline were similar in the alirocumab group as compared to the placebo group. Mean (SD) baseline calculated LDL-C was 122.4 (42.2) mg/dL (3.171 (1.092) mmol/L). With regards to background lipid modifying therapy, 1032 (44.1%) patients took high intensity statin at randomization (i.e. atorvastatin 40 to 80 mg or rosuvastatin 20 to 40 mg daily) and 334 (14.3%) were receiving ezetimibe in addition to the statin.

A summary of the baseline characteristics of the alirocumab and placebo groups is set forth in **Table 34**.

Exposure to injections was similar across treatment groups with a median exposure of 68 weeks. At the cut-off date (7 May 2014), 607 (25.9%) patients have completed the 18-months double-blind treatment period (ie. at least 76 weeks of exposure and the Week 78 visit completed), including at least 400 patients in the alirocumab group, as agreed with Health Authorities during End of Phase 2 meeting consultation: 405 patients (26.1%) in the alirocumab group and 202 patients (25.6%) in the placebo group.

**Table 30 - Disease characteristics and other relevant baseline data - Randomized population**

| | **Placebo (N=788)** | **Alirocumab 150 Q2W (N=1553)** | **All (N=2341)** |
|---|---|---|---|
| Type of hypercholesterolemia | | | |
| Heterozygous Familial Hypercholesterolemia (heFH) | 139 (17.6%) | 276 (17.8%) | 415 (17.7%) |
| Non-Familial Hypercholesterolemia (non-FH) | 649 (82.4%) | 1277 (82.2%) | 1926 (82.3%) |

| Non-Familial Hypercholesterolemia patients Time from hypercholesterolemia diagnosis (years) | | | |
|---|---|---|---|
| Number | 647 | 1273 | 1920 |
| Mean (SD) | 8.95 (7.29) | 9.13 (7.65) | 9.07 (7.53) |
| Median | 7.36 | 7.46 | 7.44 |
| Min : Max | -0.0 : 42.1 | 0.0 : 49.4 | -0.0 : 49.4 |
| | | | |

| Fredrickson classification of Hyperlipoproteinemia | | | |
|---|---|---|---|
| Number | 649 | 1277 | 1926 |
| IIa | 455 (57.7%) | 885 (57.0%) | 1340 (57.2%) |
| IIb | 194 (24.6%) | 392 (25.2%) | 586 (25.0%) |
| IV | 0 | 0 | 0 |
| | | | |

| Heterozygous Familial Hypercholesterolemia patients Time from hypercholesterolemia diagnosis (years) | | | |
|---|---|---|---|
| Number | 139 | 276 | 415 |
| Mean (SD) | 10.58 (10.66) | 11.09 (11.40) | 10.92 (11.15) |
| Median | 7.37 | 7.22 | 7.24 |
| Min : Max | -1.4 : 44.2 | -1.5 : 48.2 | -1.5 : 48.2 |
| | | | |

| Confirmation of diagnosis | | | |
|---|---|---|---|
| By genotyping | 56 (7.1%) | 111 (7.1%) | 167 (7.1%) |
| By WHO/Simon Broome^{a} | 83 (10.5%) | 165 (10.6%) | 248 (10.6%) |

| | | | |
|---|---|---|---|
| ^{a} for heFH diagnosis not confirmed by genotyping. | | | |

**Table 31 - Medical history of specific interest: cardiovascular history and risk factors - Randomized population**

| | **Placebo (N=788)** | **Alirocumab 150 Q2W (N=1553)** | **All (N=2341)** |
|---|---|---|---|
| Any cardiovascular history/risk factors | 719 (91.2%) | 1403 (90.3%) | 2122 (90.6%) |
| | | | |
| Coronary heart disease ^{a} | 552 (70.1%) | 1055 (67.9%) | 1607 (68.6%) |
| | **Placebo (N=788)** | **Alirocumab 150 Q2W (N=1553)** | **All (N=2341)** |
| Acute Myocardial Infarction | 309 (39.2%) | 563 (36.3%) | 872 (37.2%) |
| Silent Myocardial Infarction | 16 (2.0%) | 53 (3.4%) | 69 (2.9%) |
| Unstable Angina | 108 (13.7%) | 183 (11.8%) | 291 (12.4%) |
| Coronary Revascularization Procedures | 368 (46.7%) | 713 (45.9%) | 1081 (46.2%) |
| Other Clinically Significant CHD ^{b} | 233 (29.6%) | 445 (28.7%) | 678 (29.0%) |
| CHD associated with 1 or more comorbidity (among hypertension, diabetes or moderate CKD) and/or associated with other CVD (ischemic stroke, PAD) | 449 (57.0%) | 845 (54.4%) | 1294 (55.3%) |
| | | | |
| Coronary heart disease risk equivalents ^{a} | 323 (41.0%) | 639 (41.1%) | 964 (41.2%) |
| Ischemic Stroke | 74 (9.4%) | 158 (10.2%) | 232 (9.9%) |
| Peripheral Arterial Disease | 42 (5.3%) | 80 (5.2%) | 122 (5.2%) |
| Moderate Chronic Kidney Disease | 103 (13.1%) | 223 (14.4%) | 326 (13.9%) |
| Known history of diabetes mellitus AND 2 or more additional risk factors | 171 (21.7%) | 316 (20.3%) | 487 (20.8%) |
| At least 2 CHD risk equivalents or 1 CHD risk equivalent associated with hypertension or diabetes | 308 (39.1%) | 592 (38.1 %) | 900 (38.4%) |
| Categorization of CV risk per protocol | | | |
| Very high CV risk | 728 (92.4%) | 1413 (91.0%) | 2141 (91.5%) |
| High CV risk | 60 (7.6%) | 140 (9.0%) | 200 (8.5%) |

| | | | |
|---|---|---|---|
| Note: A patient can be counted in several categories. ^{a} according to the items pre-listed in the e-CRF. ^{b} diagnosed by invasive or non-invasive testing. PAD defined as Intermittent claudication TOGETHER WITH ankle-brachial index ≤ 0.90 OR TOGETHER WITH peripheral revascularization procedure/surgery OR critical limb ischemia TOGETHER WITH peripheral revascularization procedure/surgery or thrombolysis. Very high CV risk is defined as heFH patients with CHD or CHD risk equivalents or non-FH patients. High CV risk is defined as heFH patients without CHD or CHD risk equivalents. | | | |

Note: 219 patients had neither a medical history of coronary heart disease nor a medical history of coronary heart disease risk equivalents. 200 of them are heFH patients (corresponding to high CV risk category). The other 19 are non-FH without established history of CHD or CHD risk equivalents (i.e. exclusion criterion E01). By convention at the program level, these 19 patients with deviation are taken into account in the very high CV risk category.

The following summarizes the history of Lipid Modifying Therapy (LMT) including statin at screening: 1245 (53.2%) patients did not take atorvastatin 40 to 80 mg, rosuvastatin 20 to 40 mg or simvastatin 80 mg daily. The most common reason was the regional practice and local labeling. Among the 656 (28.0%) patients who did not take high intensity statin or simvastatin 80 mg daily due to regional practice/local labeling, 375 (57.2%) patients took simvastatin 40 mg.

**Table 32 - Background LMT at randomization - Randomized population**

| | **Placebo** | | **Alirocumab 150 Q2W** | | **All** | |
|---|---|---|---|---|---|---|
| | **(N=788)** | | **(N=1553)** | | **(N=2341)** | |
| Any statin | 787 | (99.9%) | 1552 | (>99.9%) | 2339 | (>99.9%) |
| | | | | | | |
| Taking high intensity statin | 342 | (43.4%) | 690 | (44.4%) | 1032 | (44.1%) |
| Atorvastatin daily dose (mg) | 283 | (35.9%) | 623 | (40.1%) | 906 | (38.7%) |
| 10 | 33 | (4.2%) | 67 | (4.3%) | 100 | (4.3%) |
| 20 | 62 | (7.9%) | 138 | (8.9%) | 200 | (8.5%) |
| 40 | 112 | (14.2%) | 240 | (15.5%) | 352 | (15.0%) |
| 80 | 73 | (9.3%) | 174 | (11.2%) | 247 | (10.6%) |
| Other doses | 3 | (0.4%) | 4 | (0.3%) | 7 | (0.3%) |
| Rosuvastatin daily dose (mg) | 199 | (25.3%) | 362 | (23.3%) | 561 | (24.0%) |
| 5 | | 9 (1.1%) | 20 | (1.3%) | 29 | (1.2%) |
| 10 | 34 | (4.3%) | 66 | (4.2%) | 100 | (4.3%) |
| 20 | 74 | (9.4%) | 134 | (8.6%) | 208 | (8.9%) |
| 40 | 80 | (10.2%) | 140 | (9.0%) | 220 | (9.4%) |
| Other doses | 2 | (0.3%) | 2 | (0.1%) | 4 | (0.2%) |
| Simvastatin daily dose (mg) | 306 | (38.8%) | 567 | (36.5%) | 873 | (37.3%) |
| 10 | 21 | (2.7%) | 43 | (2.8%) | 64 | (2.7%) |
| 20 | 84 | (10.7%) | 150 | (9.7%) | 234 | (10.0%) |
| 40 | 166 | (21.1%) | 323 | (20.8%) | 489 | (20.9%) |
| 80 | 30 | (3.8%) | 39 | (2.5%) | 69 | (2.9%) |
| Other doses | 5 | (0.6%) | 12 | (0.8%) | 17 | (0.7%) |
| | | | | | | |
| Any LMT other than statins^{a} | 220 | (27.9%) | 437 | (28.1%) | 657 | (28.1%) |
| Any LMT other than nutraceuticals | 176 | (22.3%) | 337 | (21.7%) | 513 | (21.9%) |
| Ezetimibe | | 118 (15.0%) | 216 | (13.9%) | 334 | (14.3%) |
| Nutraceuticals | 68 | (8.6%) | 134 | (8.6%) | 202 | (8.6%) |

| | | | | | | |
|---|---|---|---|---|---|---|
| ^{a} in combination with statins or not. High intensity statin corresponds to atorvastatin 40 to 80 mg daily or rosuvastatin 20 to 40 mg daily. Note: two patients, one in each group, did not receive statin at randomization and received the IMP (up to Week 10 for one patient and up to Week 74 for the other patient). | | | | | | |

**Table 33 - Lipid efficacy parameters at baseline - Quantitative summary in conventional units - Randomized population**

| | **Placebo** | **Alirocumab 150 Q2W** | **All** |
|---|---|---|---|
| | **(N=788)** | **(N=1553)** | **(N=2341)** |
| Calculated LDL-C (mg/dL) | | | |
| Number | 788 | 1553 | 2341 |
| Mean (SD) | 121.9 (41.4) | 122.7 (42.6) | 122.4 (42.2) |
| Median | 112.7 | 114.7 | 114.0 |
| Q1 : Q3 | 95.0 : 140.9 | 94.2 : 141.0 | 94.6 : 140.9 |
| Min : Max | 19 : 404 | 39 : 424 | 19 : 424 |

| Measured LDL-C (mg/dL) | | | |
|---|---|---|---|
| Number | 677 | 1322 | 1999 |
| Mean (SD) | 115.6 (38.1) | 117.2 (39.0) | 116.7 (38.7) |
| Median | 108.9 | 110.4 | 109.7 |
| Q1 : Q3 | 91.5: 132.0 | 90.3 : 134.0 | 90.7 : 132.8 |
| Min : Max | 17 : 358 | 30 :359 | 17 :359 |
| | | | |

| Non-HDL-C (mg/dL) | | | |
|---|---|---|---|
| Number | 788 | 1553 | 2341 |
| Mean (SD) | 152.0 (45.8) | 152.6 (46.6) | 152.4 (46.3) |
| Median | 142.5 | 144.8 | 144.0 |
| Q1:Q3 | 122.0 : 174.5 | 121.2 : 173.0 | 121.6 : 174.0 |
| Min : Max | 31 : 420 | 54 : 475 | 31 : 475 |
| | | | |

| Total-C (mg/dL) | | | |
|---|---|---|---|
| Number | 788 | 1553 | 2341 |
| Mean (SD) | 201.9 (47.0) | 202.4 (47.4) | 202.2 (47.3) |
| Median | 192.8 | 194.2 | 193.8 |
| Q1 : Q3 | 170.3 : 225.3 | 171.0 : 223.2 | 170.7 : 224.7 |
| Min : Max | 78 : 477 | 108 : 521 | 78 : 521 |
| | | | |

| HDL-C (mg/dL) | | | |
|---|---|---|---|
| Number | 788 | 1553 | 2341 |
| Mean (SD) | 50.0 (12.4) | 49.8 (12.2) | 49.9 (12.3) |
| Median | 48.6 | 48.0 | 48.3 |
| Q1 : Q3 | 41.7 : 56.8 | 41.3 : 56.4 | 41.3 : 56.4 |
| Min : Max | 13 : 109 | 14 : 113 | 13 : 113 |
| | | | |

| Fasting TGs (mg/dL) | | | |
|---|---|---|---|
| Number | 787 | 1553 | 2340 |
| Mean (SD) | 151.5 (79.7) | 150.7 (85.3) | 150.9 (83.4) |
| Median | 135.0 | 132.0 | 132.7 |
| Q1 : Q3 | 94.7: 188.5 | 93.8 : 183.2 | 94.0 : 185.0 |
| Min : Max | 35 : 650 | 31 : 1422 | 31 : 1422 |
| | | | |

| Lipoprotein-(a)(mg/dL) | | | |
|---|---|---|---|
| Number | 777 | 1521 | 2298 |
| Mean (SD) | 42.1 (47.2) | 43.5 (48.7) | 43.0 (48.2) |
| Median | 20.9 | 22.2 | 22.0 |
| Q1 : Q3 | 6.5 : 668 | 7.6 : 66.5 | 7.3 : 667 |
| Min : Max | 2 : 262 | 2 : 363 | 2 : 363 |
| | | | |

| Apo-B (mg/dL) | | | |
|---|---|---|---|
| Number | 778 | 1523 | 2301 |
| Mean (SD) | 101.4 (27.3) | 101.9 (27.7) | 101.7 (27.6) |
| Median | 97.0 | 98.0 | 98.0 |
| Q1 : Q3 | 84.0: 115.0 | 83.0 : 116.0 | 83.0: 115.0 |
| Min : Max | 18 : 247 | 42 : 268 | 18 : 268 |

| Apo-A1 (mg/dL) | | | |
|---|---|---|---|
| Number | 778 | 1523 | 2301 |
| Mean (SD) | 147.3 (27.3) | 146.5 (25.1) | 146.8 (25.9) |
| Median | 145.0 | 145.0 | 145.0 |
| Q1 : Q3 | 130.0 : 162.0 | 129.0 : 161.0 | 129.0 : 161.0 |
| Min : Max | 30 : 291 | 30 : 295 | 30 : 295 |
| | | | |

| Apo-B/Apo-A1 (ratio) | | | |
|---|---|---|---|
| Number | 778 | 1523 | 2301 |
| Mean (SD) | 0.713 (0.250) | 0.719 (0.267) | 0.717 (0.261) |
| Median | 0.670 | 0.680 | 0.670 |
| Q1 : Q3 | 0.560 : 0.830 | 0.550 : 0.830 | 0.560 : 0.830 |
| Min : Max | 0.12: 3.17 | 0.27 : 4.80 | 0.12 : 4.80 |
| | | | |

| Total-C/HDL-C (ratio) | | | |
|---|---|---|---|
| Number | 788 | 1553 | 2341 |
| Mean (SD) | 4.228 (1.295) | 4.252 (1.367) | 4.244 (1.343) |
| Median | 3.965 | 4.040 | 4.020 |
| Q1 : Q3 | 3.355 : 4.835 | 3.350 : 4.820 | 3.350 : 4.830 |
| Min : Max | 1.66 : 12.61 | 1.81 : 16.64 | 1.66 : 16.64 |

| | | | |
|---|---|---|---|
| Note: Measured LDL-C was assessed via the beta-quantification method. | | | |

**Table 34 - Summary of Baseline Characteristics of the Alirocumab and Placebo Groups**

| **Characteristic** | **Alirocumab** | **Placebo** |
|---|---|---|
| | (n=1553) | (n=788) |
| Age, years, mean (*SD*) | 60.4 (*10.4*) | 60.6 (*10.4*) |
| Male, % (n) | 63.3% (983) | 60.2% (474) |
| Race, White | 92.8% (1441) | 92.6% (730) |
| Race, Black or African-American | 3.4% (53) | 3.0% (24) |
| Hispanic/Latino | 5.5% (85) | 4.6% (36) |
| BMI, kg/m², mean (*SD*) | 30.2 (5.7) | 30.5 (5.5) |
| HeFH, % (n) | 17.8% (276) | 17.6% (139) |
| Diagnosis by genotyping | 7.1% (111) | 7.1% (56) |
| Clinical diagnosis (WHO/Simon Broome) | 10.6% (165) | 10.5% (83) |
| CHD history, % (n) | 67.9% (1055) | 70.1% (552) |
| Type 2 diabetes, % (n) | 34.9% (542) | 33.9% (267) |
| Current smoker, % (n) | 20.9% (325) | 20.2% (159) |
| Any statin^{†}, % (n) | 99.9% (1552) | 99.9% (787) |
| High-intensity statin^{‡} % (n) | 44.4% (690) | 43.4% (342) |
| Any LLT other than statins, % (n) | 28.1% (437) | 27.9% (220) |
| High-dose statin ^{a} | 46.9% (728) | 46.8% (369) |
| Ezetimibe, % (n) | 13.9% (216) | 15.0% (118) |
| LDL-C, calculated mean (*SD*), mmol/L [mg/dL] | 3.2 (1.1) | 3.2 (*1.1*) |
| | [122.7 (42.6)] | [121.9 (*41.4*)] |
| Median (IQR) | 114.7 (94.2-141.0) | 112.7 (95.0-140.9) |
| Range | 39-424 | 19-404 |
| LDL-C, measured mean (*SD*), mg/dL | 117.2 (39.0) | 115.6 (38.1) |
| Median (IQR) | 110.4 (90.3-134.0) | 108.9 (91.5-132.0) |
| Range | 30-359 | 17-358 |
| Non-HDL-C, mean (SD), mmol/L [mg/dL] | 4.0 (1.2) | 3.9 (1.2) |
| | [152.6 (46.6)] | [152.0 (45.8)] |
| Apo B, mean (SD), mg/dL | 101.9 (27.7) | 101.4 (27.3) |
| Lp(a), mg/dL, median (IQR) | 22.2 (7.6-66.5) | 20.9 (6.5-66.8) |
| Fasting triglycerides, median (IQR) | 132 (93.8-183.2) | 135 (94.7-188.5) |
| HDL-C, mean (SD) | 49.8 (12.2) | 50.0 (12.4) |
| Apo A1, mean (SD), mg/dL | 146.5 (25.1) | 147.3 (27.3) |

| | | |
|---|---|---|
| All patients on background of max-tolerated statin ± other lipid-lowering therapy. ^{†}Patients should receive either rosuvastatin 20-40 mg, atorvastatin 40-80 mg daily, or simvastatin 80 mg daily unless not tolerated and/or appropriate other dose given according to the judgement of the investigator. ^{‡}High-intensity statin: atorvastatin 40-80 mg or rosuvastatin 20-40 mg daily. ^{a} High-dose statin: atorvastatin 40-80 mg, rosuvastatin 20-40 mg daily, or simvastatin 80 mg daily. | | |

### Dosage and duration

Exposure to injections was similar across treatment groups with a median exposure of 68 weeks. Of note, 817 (35.0%) patients were treated at least 76 weeks (543 (35.0%) patients in the alirocumab group and 274 (34.6%) patients in the placebo group). Among those 817 patients, 607 patients also completed the Week 78 visit at the time of the first step analysis cut-off date.

### Primary efficacy endpoint

The ITT analysis includes all LDL-C values collected on-treatment and off-treatment up to Week 52. The primary endpoint (percent change in calculated LDL-C from baseline to Week 24) analysis is provided based on a MMRM model on the ITT population, using LS means estimates at Week 24. 146 (9.5%) patients in the alirocumab group and 72 (9.2%) patients in the placebo group did not have a calculated LDL-C value at Week 24. These missing values were accounted for by the MMRM model.

Results of the primary endpoint analysis are presented in **Table 35**, in mmol/L and mg/dL.

A statistically significant decrease in percent change in LDL-C from baseline to Week 24 was observed in alirocumab group (LS mean versus baseline - 61.0%) compared to the placebo group (LS mean versus baseline 0.8%) (LS mean difference vs. placebo of - 61.9%, p<0.0001).

In the alirocumab group, a consistent LDL-C reduction from baseline was observed from week 4 to Week 52. Calculated LDL-C values, change from baseline, and percent change from baseline for this period are presented in **Table 36** and **Figure 11** (ITT analysis of all available measurements over the 52 week observation period), and demonstrate a marked difference between the alirocumab and placebo treated groups.

**Table 35 - Percent change from baseline in calculated LDL-C at Week 24: MMRM - ITT analysis - ITT population**

| **Calculated LDL Cholesterol** | **Placebo** | **Alirocumab 150 Q2W** |
|---|---|---|
| | **(N=780)** | **(N=1530)** |
| Baseline (mmol/L) | | |
| Number | 780 | 1530 |
| Mean (SD) | 3.159 (1.077) | 3.180 (1.106) |
| Median | 2.920 | 2.974 |
| Min : Max | 0.49 : 10.47 | 1.01 : 10.99 |
| Baseline (mg/dL) | | |
| Number | 780 | 1530 |
| Mean (SD) | 122.0 (41.6) | 122.8 (42.7) |
| Median | 112.7 | 114.8 |
| Min : Max | 19 : 404 | 39 : 424 |
| Absolute level at week 24 (mg/dL) | 118.9 (1.2) | 48.3 (0.9) |
| | | |
| Week 24 percent change from baseline (%) | | |
| LS Mean (SE) | 0.8 (1.0) | -61.0 (0.7) |
| LS mean difference (SE) vs placebo | | -61.9 (1.3) |
| 95% CI | | (-64.3 to -59.4) |
| p-value vs placebo | | <0.0001* |

| | | |
|---|---|---|
| Note: Least-squares (LS) means, standard errors (SE) and p-value taken from MMRM (mixed-effect model with repeated measures) analysis. The model includes the fixed categorical effects of treatment group, randomization strata as per IVRS, time point, treatment-by-time point and strata-by-time point interaction, as well as the continuous fixed covariates of baseline calculated LDL-C value and baseline calculated LDL-C value-by-time point interaction MMRM model and baseline description run on patients with a baseline value and a post-baseline value in at least one of the analysis windows used in the model. The p-value is followed by a '*' if statistically significant according to the fixed hierarchical approach used to ensure a strong control of the overall type-I error rate at the 0.05 level | | |

**Table 36 - Calculated LDL-C overtime - ITT analysis - ITT population**

| | **Placebo** | | | **Alirocumab 150 Q2W** | | |
|---|---|---|---|---|---|---|
| | **(N=780)** | | | **(N=1530)** | | |
| **Calculated LDL-C** | **Value** | **Change from baseline** | **Percent change from baseline** | **Value** | **Change from baseline** | **Percent change from baseline** |
| LS Mean (SE) (mmol/L) | | | | | | |
| Baseline ^{a} | 3.159 (0.039) | NA | NA | 3.180 (0.028) | NA | NA |
| Week 4 | 3.109 (0.025) | -0.064 (0.025) | 1.1 (0.9) | 1.145 (0.018) | -2.028 (0.018) | -64.8 (0.6) |
| Week 8 | 3.084 (0.026) | -0.089 (0.026) | 1.0 (0.9) | 1.111 (0.019) | -2.061 (0.019) | -65.8 (0.7) |
| Week 12 | 3.112 (0.029) | -0.061 (0.029) | 1.5 (1.0) | 1.184 (0.020) | -1.989 (0.020) | -63.3 (0.7) |
| Week 16 | 3.076 (0.029) | -0.097 (0.029) | 0.9 (1.0) | 1.143 (0.021) | -2.030 (0.021) | -64.5 (0.7) |
| Week 24 | 3.081 (0.031) | -0.092 (0.031) | 0.8 (1.0) | 1.252 (0.022) | -1.921 (0.022) | -61.0 (0.7) |
| Week 36 | 3.066 (0.032) | -0.107 (0.032) | 0.3 (1.1) | 1.278 (0.023) | -1.895 (0.023) | -59.5 (0.8) |
| Week 52 | 3.187 (0.036) | 0.014 (0.036) | 4.4 (1.2) | 1.376 (0.026) | -1.797 (0.026) | -56.8 (0.8) |
| | | | | | | |

| LS Mean (SE) (mg/dL) | | | | | | |
|---|---|---|---|---|---|---|
| Baseline^{a} | 122.0 (1.5) | NA | NA | 122.8 (1.1) | NA | NA |
| Week 4 | 120.0 (1.0) | -2.5 (1.0) | 1.1 (0.9) | 44.2 (0.7) | -78.3 (0.7) | -64.8 (0.6) |
| Week 8 | 119.1 (1.0) | -3.4 (1.0) | 1.0 (0.9) | 42.9 (0.7) | -79.6 (0.7) | -65.8 (0.7) |
| Week 12 | 120.1 (1.1) | -2.4 (1.1) | 1.5 (1.0) | 45.7 (0.8) | -76.8 (0.8) | -63.3 (0.7) |
| Week 16 | 118.8 (1.1) | -3.7 (1.1) | 0.9 (1.0) | 44.1 (0.8) | -78.4 (0.8) | -64.5 (0.7) |
| Week 24 | 118.9 (1.2) | -3.6 (1.2) | 0.8 (1.0) | 48.3 (0.9) | -74.2 (0.9) | -61.0 (0.7) |
| Week 36 | 118.4 (1.2) | -4.1 (1.2) | 0.3 (1.1) | 49.3 (0.9) | -73.2 (0.9) | -59.5 (0.8) |
| Week 52 | 123.0 (1.4) | 0.5 (1.4) | 4.4 (1.2) | 53.1 (1.0) | -69.4 (1.0) | -56.8 (0.8) |

| | | | | | | |
|---|---|---|---|---|---|---|
| ^{a} Baseline is described using means and standard errors. Note: Least-squares (LS) means, standard errors (SE) and p-value taken from MMRM (mixed-effect model with repeated measures) analysis. The model includes the fixed categorical effects of treatment group, randomization strata as per IVRS, time point, treatment-by-time point interaction, strata-by-time point interaction, as well as the continuous fixed covariates of baseline LDL-C value and baseline LDL-C value-by-time point interaction MMRM model and baseline description run on patients with a baseline value and a post-baseline value in at least one of the analysis windows used in the model. | | | | | | |

Of note, as part of the model assumption checks, the homogeneity of treatment effect across lipids baseline levels has been assessed and shows a significant treatment-by baseline LDL-C level interaction (p<0.0001). To further explore this interaction, the treatment effect on the primary efficacy endpoint, according to 4 classes of baseline LDL-C level, is presented below. It shows a decreased treatment effect versus placebo for higher baseline LDL-C levels (from -75.0% for patients with baseline LDL-C < 100 mg/dL to -41.3% for patients with LDL-C ≥ 160 mg/dL). This observation appears to be due to changes in the placebo group, where the percent change in LDL-C from baseline to Week 24 decreases with increasing baseline LDL-C categories. Conversely, the percent change in LDL-C from baseline to Week 24 is consistent across the different sub-categories of LDL-C within the alirocumab group.

**Table 37 - Percent change from baseline in calculated LDL-C at Week 24: Subgroup analysis according to calculated LDL-C at baseline - ITT analysis**

| **Subgroup factor Percent change from baseline in calculated LDL-C at week 24 (%)** | **Placebo (N=780)** | **Alirocumab 150 Q2W (N=1530)** | **Interaction p-value** |
|---|---|---|---|
| Baseline calculated LDL-C | | | <0.0001 |
| <100 mg/dL / <2.59 mmol/L | | | |
| Number | 241 | 470 | |
| LS means (SE) | 13.6 (1.8) | -61.3 (1.3) | |
| LS mean difference (SE) vs | | | |
| placebo | | -75.0 (2.2) | |
| 95% CI | | (-79.3 to -70.6) | |
| LS mean absolute LDL-C | | | |
| reduction, mg/dL | 9.5 | -51.6 | |
| | | | |
| ≥ 100 to <130 mg/dL / ≥ 2.59 to | | | |
| <3.37 mmol/L | | | |
| Number | 285 | 562 | |
| LS means (SE) | 0.5 (1.7) | -62.0 (1.2) | |
| LS mean difference (SE) vs | | | |
| placebo | | -62.5 (2.0) | |
| 95% CI | | (-66.5 to -58.4) | |
| LS mean absolute LDL-C | | | |
| reduction, mg/dL | 0.0 | -71.2 | |
| ≥ 130 to <160 mg/dL / ≥ 3.37 to | | | |
| <4.14 mmol/L | | | |
| Number | 143 | 271 | |
| LS means (SE) | -5.2 (2.4) | -59.8 (1.7) | |
| LS mean difference (SE) vs | | | |
| placebo | | -54.6 (2.9) | |
| 95% CI | | (-60.3 to -48.8) | |
| LS mean absolute LDL-C | | | |
| reduction, mg/dL | -7.6 | -85.3 | |
| ≥ 160 mg/dL / ≥ 4.14 mmol/L | | | |
| Number | 111 | 227 | |
| LS means (SE) | -18.2 (2.8) | -59.5 (1.9) | |
| LS mean difference (SE) vs | | | |
| placebo | | -41.3 (3.3) | |
| 95% CI | | (-47.8 to -34.8) | |
| LS mean absolute LDL-C | | | |
| reduction, mg/dL | -33.7 | -115.8 | |
| | | | |
| Baseline HDL-C | | | 0.0989 |
| <40 mg/dL / <1.04 mmol/L | | | |
| Number | 151 | 306 | |
| LS means (SE) | 0.5 (2.3) | -65.5 (1.6) | |
| LS mean difference (SE) vs | | | |
| placebo | | -66.1 (2.8) | |
| 95% CI | | (-71.6 to -60.5) | |
| ≥ 40 mg/dL / ≥ 1.04 mmol/L | | | |
| Number | 629 | 1224 | |
| LS means (SE) | 0.9 (1.1) | -59.9 (0.8) | |
| LS mean difference (SE) vs | | | |
| placebo | | -60.8 (1.4) | |
| 95% CI | | (-63.6 to -58.1) | |

| | | | |
|---|---|---|---|
| Note: Least-squares (LS) means, standard errors (SE) and p-value taken from MMRM (mixed-effect model with repeated measures) analysis. The model includes the fixed categorical effects of treatment group, randomization strata as per IVRS, subgroup factor, time point, and the interactions treatment-by-time point, strata-by-time point, subgroup factor-by-time point, treatment group-by-subgroup factor, and treatment group-by-subgroup factor-by-time point for baseline LDL-C as subgroup factor. For other subgroup factors, the model also includes the continuous fixed covariates of baseline LDL-C value and baseline LDL-C value-by-time point interaction. Number corresponds to number of patients with a baseline value and a post-baseline value in at least one of the analysis windows used in the model The p-value is provided for descriptive purpose only | | | |

### Key secondary efficacy endpoints

**Table 38** summarizes analysis results on key secondary endpoints in the hierarchical order. All key secondary endpoints are statistically significant according to the hierarchical testing procedure.

**Table 38**

| **Endpoint** | **Analysis** | **Results** | **P-value** |
|---|---|---|---|
| Calculated LDL-C - Percent change from baseline to Week 24 | On-treatment | LS mean difference vs. placebo of -63.5% | <0.0001 |
| Calculated LDL-C - Percent change from baseline to Week 12 | ITT | LS mean difference vs. placebo of -64.8% | <0.0001 |
| Calculated LDL-C - Percent change from baseline to Week 12 | On-treatment | LS mean difference vs. placebo of -65.5% | <0.0001 |
| Measured LDL-C (by ultracentrifugation) - Percent change from baseline to Week 24 | ITT | LS mean difference vs. placebo of -61.3% | <0.0001 |
| Apo-B - Percent change from baseline to Week 24 | ITT | LS mean difference vs. placebo of -54% | <0.0001 |
| Apo-B - Percent change from baseline to Week 24 | On-treatment | LS mean difference vs. placebo of -55.5% | <0.0001 |
| Non-HDL-C - Percent change from baseline to Week 24 | ITT | LS mean difference vs. placebo of -52.3% | <0.0001 |
| Non-HDL-C - Percent change from baseline to Week 24 | On-treatment | LS mean difference vs. placebo of -53.7% | <0.0001 |
| Total-C - Percent change from baseline to Week 24 | ITT | LS mean difference vs. placebo of -37.5% | <0.0001 |
| Apo-B - Percent change from baseline to Week 12 | ITT | LS mean difference vs. placebo of -56% | <0.0001 |
| Non-HDL-C - Percent change from baseline to Week 12 | ITT | LS mean difference vs. placebo of -54.6% | <0.0001 |
| Total-C - Percent change from baseline to Week 12 | ITT | LS mean difference vs. placebo of -39% | <0.0001 |
| Proportion of very high CV risk patients reaching calculated LDL-C < 70 mg/dL (1.81 mmol/L) or high CV risk patients reaching calculated LDL-C < 100 mg/dL (2.59 mmol/L) at Week 24 | ITT | combined estimate for odds-ratio vs. placebo of 71.5 | <0.0001 |
| Proportion of very high CV risk patients reaching calculated LDL-C < 70 mg/dL (1.81 mmol/L) or high CV risk patients reaching calculated LDL-C < 100 mg/dL (2.59 mmol/L) at Week 24 | On-treatment | combined estimate for odds-ratio vs. placebo of 93.4 | <0.0001 |
| Proportion of patients reaching calculated LDL-C <70 mg/dL (1.81 mmol/L) at Week 24 | ITT | combined estimate for odds-ratio vs. placebo of 74.6 | <0.0001 |
| Proportion of patients reaching calculated LDL-C <70 mg/dL (1.81 mmol/L) at Week 24 | On-treatment | combined estimate for odds-ratio vs. placebo of 97.3 | <0.0001 |
| Lp(a) - Percent change from baseline to Week 24 | ITT | combined estimate for adjusted mean difference vs. placebo of -25.6% | <0.0001 |
| HDL-C - Percent change from baseline to Week 24 | ITT | LS mean difference vs. placebo of 4.6% | <0.0001 |
| Fasting TGs - Percent change from baseline to Week 24 | ITT | combined estimate for adjusted mean difference vs. placebo of -17.3% | <0.0001 |
| Apolipoprotein A1 - Percent change from baseline to Week 24 | ITT | LS mean difference vs. placebo of 2.9% | <0.0001 |
| Lp(a) - Percent change from baseline to Week 12 | ITT | combined estimate for adjusted mean difference vs. placebo of -25.1% | <0.0001 |
| HDL-C - Percent change from baseline to Week 12 | ITT | LS mean difference vs. placebo of 5.6% | <0.0001 |
| Fasting TGs - Percent change from baseline to Week 12 | ITT | combined estimate for adjusted mean difference vs. placebo of -17.9% | <0.0001 |
| Apolipoprotein A1 - Percent change from baseline to Week 12 | ITT | LS mean difference vs. placebo of 4% | <0.0001 |

**Table 39 - Percent change from baseline in lipid parameters at Week 24 (ITT population^{a)}**

| **Secondary endpoints** | **Placebo (N=780)** | **Alirocumab 150 Q2W (N=1530)** |
|---|---|---|
| Proportion (%) of patients reaching calculated LDL-C levels at Week 24 | | |
| <70 mg/dL (very high risk) or <100 mg/dL (high risk) | 8.5 | 80.7 |
| p-value vs placebo | | <0.0001^{*} |
| <70 mg/dL (regardless of risk) | 8.0 | 79.3 |
| p-value vs placebo | | <0.0001 |
| Percentage change from baseline to Week 78 in calculated LDL-C | 3.6 (1.3) | -52.4 (0.9) |
| LS mean difference (SE) vs placebo | | -56.0 (1.6) |
| 95% CI | | -59.1 to -52.8 |
| value vs placebo | | <0.001 |
| | | |
| LS mean (SE) change from baseline to Week 24 (%) in secondary lipid parameters | | |
| Non-HDL-C | 0.7 (0.9) | -51.6 (0.6) |
| LS mean difference (SE) vs placebo | | -52.3 (1.1) |
| 95% CI | | -54.4 to -50.2 |
| p-value vs placebo | | <0.0001 |
| Apo B | 1.2 (1.0) | -52.8 (0.7) |
| LS mean difference (SE) vs placebo | | -54.0 (1.2) |
| 95% CI | | -56.3 to -51.7 |
| p-value vs placebo | | <0.0001 |
| Total cholesterol | -0.3 (0.7) | -37.8 (0.5) |
| LS mean difference (SE) vs placebo | | -37.5 (0.8) |
| 95% CI | | -39.1 to -35.9 |
| p-value vs placebo | | <0.0001 |
| Lipoprotein (a)^{b} | -3.7 (1.0) | -29.3 (0.7) |
| LS mean difference (SE) vs placebo | | -25.6 (1.3) |
| 95% CI | | -28.1 to -23.1 |
| p-value vs placebo | | <0.0001 |
| Fasting triglycerides^{b} | 1.8 (1.2) | -15.6 (0.8) |
| LS mean difference (SE) vs placebo | | -17.3 (1.4) |
| 95% CI | | -20.1 to -14.6 |
| p-value vs placebo | | <0.0001 |
| HDL-C | -0.6 (0.5) | 4.0 (0.4) |
| LS mean difference (SE) vs placebo | | 4.6 (0.7) |
| 95% CI | | 3.3 to 5.9 |
| p-value vs placebo | | <0.0001 |
| Apo A1 | 1.2 (0.6) | 4.0 (0.4) |
| LS mean difference (SE) vs placebo | | 2.9 (0.7) |
| 95% CI | | 1.6 to 4.2 |
| p-value vs placebo | | <0.0001 |
| | | |
| Number of patients with measured LDL-C^{c} values available | 652 | 1278 |
| | | |
| LS mean (SE) change from baseline to Week | | |
| 24 in measured LDL-C, % | 3.5 (1.1) | -57.8 (0.8) |
| LS mean difference (SE) vs placebo | | -61.3 (1.4) |
| 95% CI | | -64.0 to -58.5 |
| p-value vs placebo | | <0.0001 |

| | | |
|---|---|---|
| Abbreviations: Cl, confidence intervals; HDL, high-density lipoprotein; ITT, intention-to-treat; LDL, low-density lipoprotein; LS, least squares; LLT, lipid-lowering therapy; SD, standard deviation; SE, standard error. ^{a} Primary and secondary efficacy analyses were conducted using an ITT approach including patients with baseline calculated LDL -cholesterol value and at least one calculated LDL-cholesterol value on or off-treatment within one of the analysis windows up to week 24. LS means, SE and p-value taken from mixed-effect model with repeated measures analysis. The p-values are significant according to the fixed hierarchical approach used to ensure control of the overall type-1 error rate at the 0.05 level. ^{b} Combined estimate for adjusted mean (SE) shown for lipoprotein (a) and triglycerides. ^{c} LDL-cholesterol measured by beta-quantification. | | |

**Table 40 - Percent change from baseline in lipid parameters at Week 24 - On-Treatment analysis^{a}**

| | **Placebo (N=777)** | **Alirocumab 150 Q2W (N=1523)** |
|---|---|---|
| Calculated LDL-C | | |
| aseline, mean (SD) mg/dL | 121.9 (41.5) | 122.8 (42.7) |
| Range | 19-404 | 39-424 |
| LS mean (SE) change from baseline to Week 24 (%) | 0.7 (1.0) | -62.8 (0.7) |
| LS mean difference (SE) vs placebo | | -63.5 (1.2) |
| 95% CI | | -65.9 to -61.2 |
| p-value vs placebo | | <0.0001 |
| LS mean (SE) change from baseline to Week 24 (%) in secondary lipid parameters | | |
| Non-HDL-C | 0.6 (0.9) | -53.1 (0.6) |
| LS mean difference (SE) vs placebo | | -53.7 (1.0) |
| 95% CI | | -55.7 to -51.6 |
| p-value vs placebo | | <0.0001 |
| Apo B | 1.2 (0.9) | -54.3 (0.7) |
| LS mean difference (SE) vs placebo | | -55.5 (1.2) |
| 95% CI | | -57.7 to -53.2 |
| p-value vs placebo | | <0.0001 |
| Total cholesterol | -0.4 (0.6) | -38.8 (0.5) |
| LS mean difference (SE) vs placebo | | -38.4 (0.8) |
| 95% CI | | -39.9 to -36.9 |
| p-value vs placebo | | <0.0001 |
| Lipoprotein (a)^{b} | -3.9 (1.0) | -30.2 (0.7) |
| LS mean difference (SE) vs placebo | | -26.3 (1.3) |
| 95% CI | | -28.8 to -23.8 |
| p-value vs placebo | | <0.0001 |
| Triglycerides^{b} | 1.4 (1.2) | -15.8 (0.8) |
| LS mean difference (SE) vs placebo | | -17.2 (1.4) |
| 95% CI | | -20.0 to -14.4 |
| p-value vs placebo | | <0.0001 |
| HDL-C | -0.7 (0.5) | 4.2 (0.4) |
| LS mean difference (SE) vs placebo | | 4.9 (0.7) |
| 95% CI | | 3.6 to 6.1 |
| p-value vs placebo | | <0.0001 |
| Apo A1 | 1.2 (0.6) | 4.2 (0.4) |
| LS mean difference (SE) vs placebo | | 2.9 (0.7) |
| 95% CI | | 1.6 to 4.3 |
| p-value vs placebo | | <0.0001 |

| | | |
|---|---|---|
| Abbreviations: Cl, confidence intervals; HDL, high-density lipoprotein; LDL, low-density lipoprotein; LS, least squares; LLT, lipid-lowering therapy; SE, standard error. ^{a} Only patients who were receiving study treatment were included in the on-treatment analysis. The on-treatment analysis excluded any patients without a calculated LDL-cholesterol value available between the first dose of study treatment up until 21 days after last injection. ^{b} Combined estimate for adjusted mean (SE) shown for lipoprotein (a) and triglycerides. P values taken from mixed-effect model with repeated measures analysis. | | |

**Table 41 - Percent change from baseline in calculated LDL-C at Week 24 (Pattern Mixture Model^{a})**

| | **Placebo (N=780)** | **Alirocumab 150 Q2W (N=1523)** |
|---|---|---|
| Calculated LDL-C | | |
| Baseline, mean (SD) mg/dL | 122.0 (41.6) | 122.7 (42.7) |
| Range | 19-404 | 39-424 |
| LS mean (SE) change from baseline to Week 24 (%) | 0.7 (1.1) | -57.8 (0.8) |
| LS mean difference (SE) vs placebo | | -58.5 (1.3) |
| 95% CI | | -61.1 to -55.8 |
| p-value vs placebo | | <0.0001 |

| | | |
|---|---|---|
| Abbreviations: Cl, confidence intervals; LDL, low-density lipoprotein; LS, least squares; LLT, lipid-lowering therapy; SE, standard error. ^{a} This sensitivity analysis has been conducted to further evaluate the impact of missing data on the primary endpoint: in this approach, missing calculated LDL-cholesterol values during the "on-treatment" period were multiply imputed using a model assuming Missing At Random and missing calculated LDL-cholesterol values during the post-treatment period were multiply imputed using random draws from a normal distribution, with mean equal to subject's own baseline value. | | |

Detailed analyses for each key secondary endpoint is described herein.

The on-treatment analysis of calculated LDL-C percent change from baseline to Week 24 shows consistent results with the ITT analysis with a LS mean difference vs. placebo of - 63.5% in the on-treatment analysis versus -61.9% in the ITT analysis. Indeed, few patients had LDL-C values collected post-treatment (i.e. more than 21 days after last injection at week 24: 37 patients (2.4%) in the alirocumab group versus 20 (2.6%) in the placebo group. Percent change from baseline in lipid parameters at Week 24 in the ITT population and the on-treatment population can be seen in **Table 39** and **Table 40**, respectively. The pattern mixture model analysis of percent change from baseline in calculated LDL-C at Week 24 can be seen in **Table 41.**

The ITT and on-treatment analyses of calculated LDL-C percent change from baseline to Week 12 also shows consistent results.

At Week 24, the treatment effect on measured LDL-C is comparable to the effect on calculated LDL-C. Results on measured LDL-C will be further investigated, including the correlation between calculated and measured LDL-C.

ITT analysis was performed on Apo B, non-HDL-C and total-C at Week 12 and 24. On-treatment analysis was also performed at Week 24 for Apo B and non-HDL-C. Results of ITT and on-treatment analyses are similar for these 2 lipid parameters. The alirocumab group exhibited a significant reduction in non-HDL-C, ApoB and Lp(a) levels relative to placebo at 24 weeks.

On the ITT analysis at Week 24, 80.7% of very high CV risk or high CV risk patients reached calculated LDL-C < 70 mg/dL or < 100 mg/dL, respectively, in the alirocumab group versus 8.5% in placebo group (p < 0.0001). Regarding the on-treatment analysis at Week 24, 82.8% of very high CV risk or high CV risk patients reached calculated LDL-C < 70 mg/dL or < 100 mg/dL, respectively, in the alirocumab group versus 8.5% in placebo group.

Proportion of patients reaching calculated LDL-C <70 mg/dL was studied on ITT and on-treatment analysis at Week 24. 79.3% of patients from the alirocumab group reached this level on the ITT analysis (81.2% on the on-treatment analysis) versus 8.0% in the placebo group on the ITT analysis (8.0% in the on-treatment analysis).

### Primary efficacy endpoint according to subgroup status

As described next, comparisons of the primary efficacy endpoint in various subgroups were analyzed, including HeFH status, demographic groups, medical history, and patients who reached to consecutive LDL-C <25 mg/dL.

A comparison in the percent change in LDL-C from baseline to week 24 between patients who have heterozygous familial hypercholesterolemia ("HeFH"), and those who do not, is provided in Table 43 and Figures 12A-C. **Figure 12B** shows a comparison between the population of HeFH patients that have baseline LDL-C levels of <160 mg/dL or ≥ 160 mg/dL; **Figure 12C** shows a comparison between the population of HeFH patients that have baseline LDL-C levels of <190 mg/dL or ≥ 190 mg/dL. All patients were on a background treatment including maximally tolerated statins and optional further lipid lowering therapies. The decrease in percent change in LDL-C from baseline to Week 24 was statistically significant in the alirocumab groups compared to the placebo groups regardless of whether the patients had HeFH (see Figure 12A). The decrease in percent change in LDL-C from baseline to Week 24 was also statistically significant in the alirocumab groups compared to the placebo groups regardless of and whether the patients with HeFH had LDL-C ≥ 160 mg/dL or <160 mg/dL (see **Figure 12B**) or LDL-C ≥ 190 mg/dL or <190 mg/dL (see **Figure 12C**).

The difference seen between LS mean (SE) percent change from baseline to Week 24 for LDL-C in alirocumab treated patients (compared to placebo patients) was also consistent in patients with PCSK9 levels that were less or more than the median total or free PCSK9 value. Median total PCSK9 levels were 637 ng/mL in the alirocumab group and 640 ng/mL in the placebo group. Total PCSK9 levels are defined as all circulating PCSK9, which includes PCSK9 that is free (unbound) as well as PCSK9 that is bound by anti-PCSK9 antibodies (and inactive). For patients with total PCSK9 levels below the median, the percent change from baseline in calculated LDL-C was significantly greater in the alirocumab group (LS mean versus baseline - 60.1%) compared to the placebo group (LS mean versus baseline -2.8%) (LS mean difference vs. placebo of -57.3%, p < 0.0001). For patients with total PCSK9 levels at or above the median, the percent change from baseline in calculated LDL-C was significantly greater in the alirocumab group (LS mean versus baseline -62.1%) compared to the placebo group (LS mean versus baseline 4.1%) (LS mean difference vs. placebo of -66.0%, p < 0.0001). The percent change in LDL-C from baseline to Week 24 showed a statistically greater reduction in subjects with total PCSK9 levels at or above the median compared to those with total PCSK9 levels below the median (interaction p-value 0.0003).

Similarly, for patients with free PCSK9 levels below the median, the percent change from baseline in calculated LDL-C was significantly greater in the alirocumab group (LS mean versus baseline -59.4%) compared to the placebo group (LS mean versus baseline -1.6%) (LS mean difference vs. placebo of -58.0%, p < 0.0001). For patients with free PCSK9 levels at or above the median, the percent change from baseline in calculated LDL-C was significantly greater in the alirocumab group (LS mean versus baseline -62.7%) compared to the placebo group (LS mean versus baseline 3.0%) (LS mean difference vs. placebo of -66.0%, p < 0.0001). The percent change in LDL-C from baseline to Week 24 showed a statistically greater reduction in subjects with free PCSK9 levels at or above the median compared to those with free PCSK9 levels below the median (interaction p-value 0.0015).

The decrease in percent change in LDL-C from baseline to Week 24 seen in this study was also statistically significant in the alirocumab groups compared to the placebo groups in both males and females. In men, the percent change from baseline in calculated LDL-C was significantly greater in the alirocumab group (LS mean versus baseline -65.5%) compared to the placebo group (LS mean versus baseline -0.7%) (LS mean difference vs. placebo of -64.7%, p < 0.0001). In women, the percent change from baseline in calculated LDL-C was significantly greater in the alirocumab group (LS mean versus baseline -53.4%) compared to the placebo group (LS mean versus baseline 3.2%) (LS mean difference vs. placebo of -56.6%, p < 0.0001). The percent change in LDL-C from baseline to Week 24 showed a statistically greater reduction in men than women (interaction p-value 0.0014).

The decrease in percent change in LDL-C from baseline to Week 24 seen in the ODDYSEY LONG TERM study was also statistically significant in the alirocumab groups compared to the placebo groups in different subgroups, including race, age, BMI, moderate chronic kidney disease, diabetes status, and baseline fasting TG levels (see **Figure 13**), different statin and lipid-lowering therapy (LLT) subgroups (see **Figure 14**). All patients were on a background statin (at the maximum tolerated level). A subset of patients also received a further lipid lowering therapy.

Two consecutive calculated LDL-C values <25 mg/dL (< 0.65 mmo/L) were observed in 562 (37.4%) patients. An analysis was made on the frequency of TEAEs (PT) vs. the overall alirocumab group assessing for marked imbalances. TEAEs that were AESI which occurred in patients with two consecutive calculated LDL-C values <25 mg/dL (< 0.65 mmo/L) were compared for frequency with the overall alirocumab group. Finally, theoretical AEs were reviewed to ensure that no such reported term existed, and if present were further investigated at the patient level data. No particular safety concern has been observed from such analysis in these patients.

**Table 42 - Number (%) of patients with 2 consecutive LDL-C < 25mg/dL (<0.65 mmol/L) during the treatment period- Safety population (at the time of the pre-specified analysis)**

| | **Placebo (N=788)** | **Alirocumab 150 Q2W (N=1550)** |
|---|---|---|
| Patients with 2 consecutive calculated LDL-C value <25 mg/dL¹ | 0/767 | 562/1503 (37.4%) |
| Time to the first calculated LDL-C value < 25 mg/dL (weeks) ² | | |
| Number | 0 | 562 |
| Mean (SD) | | 11.03 (11.51) |
| Median | | 4.86 |
| Min : Max | | 2.3 : 65.1 |
| | | |
| Patients with 2 consecutive calculated LDL-C value <15 mg/dL¹ | 0/767 | 223/1503 (14.8%) |
| | | |
| Time to the first calculated LDL-C value < 15 mg/dL (weeks) ² | | |
| Number | 0 | 223 |
| Mean (SD) | | 12.99 (12.43) |
| Median | | 8.14 |
| Min : Max | | 3.1 : 64.6 |

| | | |
|---|---|---|
| The number (n) represents the subset of the total number of patients who met the criteria The denominator (/N) within a treatment group is the number of patients for the treatment group who had at least two calculated LDL-C values assessed at least 21 days apart in the efficacy period ¹ 2 consecutive values are considered if spaced out by at least 21 days ² First calculated LDL-C value <25 or <15 mg/dL among the first 2 consecutive calculated LDL-C values <25 or <15 mg/dL per patient | | |

**Table 43 - Percent change from baseline in calculated LDL-C at Week 24: according to heFH status at randomization - ITT population**

| **Subgroup factor Percent change from baseline in calculated LDL-C at week 24 (%)** | **Placebo (N=780)** | **Alirocumab 150 Q2W (N=1530)** | **Interaction p-value** |
|---|---|---|---|
| HeFH | | | **0.6038** |
| HeFH population | | | |
| Number | 145 | 271 | |
| LS means (SE) | 7.0 (2.4) | -56.3 (1.9) | |
| LS mean difference (SE) vs | | | |
| placebo | | -63.2 (2.9) | |
| 95% CI | | (-69.0 to -57.5) | |
| Non-HeFH population | | | |
| Number | 635 | 1259 | |
| LS means (SE) | -0.5 (1.1) | -62.1 (0.8) | |
| LS mean difference (SE) vs | | | |
| placebo | | -61.5 (1.4) | |
| 95% CI | | (-64.3 to -58.8) | |

| | | | |
|---|---|---|---|
| Note: Least-squares (LS) means, standard errors (SE) and p-value taken from MMRM (mixed-effect model with repeated measures) analysis. The model includes the fixed categorical effects of treatment group, randomization strata as per IVRS, subgroup factor, time point, and the interactions treatment-by-time point, strata-by-time point, subgroup factor-by-time point, treatment group-by-subgroup factor, and treatment group-by-subgroup factor-by-time point, as well as the continuous fixed covariates of baseline LDL-C value and baseline LDL-C value-by-time point interaction. Number corresponds to number of patients with a baseline value and a post-baseline value in at least one of the analysis windows used in the model The p-value is provided for descriptive purpose only | | | |

### Summary efficacy results:

At Week 24, the percent change from baseline in calculated LDL-C in the ITT population was significantly greater in the alirocumab group (LS mean versus baseline -61.0%) compared to the placebo group (LS mean versus baseline 0.8%) (LS mean difference vs. placebo of -61.9%, p < 0.0001) (see **Table 35**). The on-treatment analysis of LDL-C percent change from baseline to Week 24 shows consistent results with the ITT analysis with a LS mean difference vs. placebo of -63.5% in the on-treatment analysis (p<0.0001). Indeed, at Week 24, few patients (2.5% of patients overall) had LDL-C values collected post-treatment (i.e. more than 21 days after last injection). At Week 24, the treatment effect on measured LDL-C (LS mean versus baseline -61.3%) is comparable to the effect on calculated LDL-C (ITT analysis).

In the alirocumab group, a consistent LDL-C reduction from baseline was observed from week 4 to Week 52.

The homogeneity of treatment effect across lipids baseline levels was assessed and it showed a significant treatment-by baseline LDL-C level interaction (p<0.0001). This observation appears to be due to changes in the placebo group. Indeed the change in LDL-C from baseline to Week 24 is consistent across the different sub-categories of LDL-C within the alirocumab group. The LS mean (SE) percent change from baseline to Week 24 for LDL-C in alirocumab group patients was statistically significant and consistent across a range of LDL-C baseline values (**Table 37**).

All key secondary endpoints were statistically significant according to the hierarchical testing procedure.

Two consecutive calculated LDL-C values <25 mg/dL (< 0.65 mmo/L) were observed in 562 (37.4%) patients. No particular safety concern has been observed in these patients.

### Summary safety results:

For the purposes of this study, TEAE are adverse events that developed, worsened, or became serious during the TEAE period (time from the first dose of the double-blind treatment to seventy days after the last dose). At the time of the pre-specified analysis, the percentages of patients who experienced TEAE, serious TEAE and TEAE leading to treatment discontinuation were similar between treatment groups (see **Table 44**).

The most frequently reported SOC in both treatment groups (≥ 10%) were "infections and infestations" (45.5% in the alirocumab group versus 46.1% in the placebo group), "musculoskeletal and connective tissue disorders" (27.2% in the alirocumab group vs. 28.6% in the placebo group), "gastrointestinal disorders" (18.6% in the alirocumab group vs.18.8% in the placebo group), "nervous system disorders" (17.0% in the alirocumab group vs.17.8% in the placebo group) "general disorders and administration site conditions" (15.4% in the alirocumab group vs. 17% in the placebo group), "injury, poisoning and procedural complications" (13.4% in the alirocumab group vs. 14.2% in the placebo group), and "respiratory, thoracic and mediastinal disorders" (11.0% vs. 10.9%). **Table 45** lists the number of TEAEs for selected system-organ-classes. **Table 46** lists the percentage of different TEAEs in alirocumab and placebo treated groups as well as specifically listing the TEAEs observed in alirocumab-treated patients (by percentage of patients) with two consecutive LDL-C measurements of less than 25 mg/dL. All patients were on a background statin (at the maximum tolerated level). A subset of patients also received a further lipid lowering therapy. Patients who have acheived LDL-C levels of less than 25 mg/dL in two or more consecutive periods make up 36.3% of the patient population treated with alirocumab (562 patients out of 1550). Certain laboratory values of interest (alanine aminotransferase > 3 x ULN, aspartate aminiotransferase > 3 x ULN, and creatine kinase >3 x ULN) are shown in **Table 47.** Few alirocumab patients (31 out of 1461, 2.1%) had decreased plasma vitamin E levels below the outer limit of normal during the TEAE period (compared to 1 patient out of 738 in the placebo group, 0.1%).

No marked imbalance was observed on the frequency of TEAE (Preferred Term). The following TEAEs were the most frequently reported in both treatment groups (≥ 5% in any group): nasopharyngitis (12.6% in alirocumab vs. 12.7% in placebo group), upper respiratory tract infection (7.0% vs. 8.0%), injection site reaction (5.7% vs. 4.3%), influenza (5.4% vs. 5.5%), diarrhea (5.3% vs 5.1%), urinary tract infection (5.2% vs. 6.2%), bronchitis (5.2% vs. 4.7%) and headache (4.8% vs. 5.6%). Most injection site reactions (treatment-emergent local injection site reactions) were of mild or moderate intensity, with the first reaction occurring in the first three months of therapy (57 days of alirocumab cersus 100 days of placebo) for the majority of patients.

TEAE of special interest included: treatment-emergent local injection site reactions; general allergic reaction events; neurological events; neurocognitive disorders; adjudicated cardiovascular events; neurocognitive disorders; and haemolytic anaemia. The prevalence of these events in the alirocumab and placebo groups is set forth in **Table 48.** The prevalence of specific neurocognitive adverse events in the alirocumab and placebo groups is set forth in **Table 49.**

Nineteen (19) deaths were reported during the study (11 (0.7%) in the alirocumab group versus 8 (1.0%) in the placebo group). Focusing on the TEAE leading to death, 7 (0.5%) deaths were reported in the alirocumab group versus 8 (1.0%) in the placebo group. Per adjudication, the primary cause of death was cardiovascular in both treatment groups (during TEAE period, 5 (0.3%) cardiovascular death in alirocumab group versus 5 (0.6%) in placebo group).

SAEs were reported by 394 patients (255 (16.5%) patients in the alirocumab group and 139 (17.6%) patients in the placebo group) during the TEAE period. No imbalance between groups is observed.

No specific pattern was noted among the TEAEs leading to permanent treatment discontinuation.

Among the events of interest, no particular signal was detected for TEAEs related to neurological events and ophthalmological events (including in the ophthalmological sub-study). For neurocognitive disorders, 18 (1.2%) were observed in the alirocumab group versus 4 (0.5%) in the placebo group including mainly amnesia, memory impairment and confusional state. No case of hemolytic anaemia was reported.

With regards to general allergic reaction, similar proportions of patients reported at least one such event in alirocumab (140 [9.0%]) vs. placebo (71 [9.0%]) groups.

124 (5.3%) patients (90 (5.8%) patients from alirocumab group and 34 (4.3%) patients from placebo group) experienced a treatment-emergent local injection site reaction. Most of these events were of mild or moderate intensity.

According to the "diabetes and impaired glucose control" exploratory analysis, a total of 36 (6.3%) patients in the alirocumab group and 10 (3.6%) patients in the placebo group classified as having "impaired glucose control" at baseline were defined as diabetic in the TEAE period. Two patients classified with normal status at baseline became diabetic (one in each treatment group).

There was no imbalance for PCSA of laboratory parameters, ECG and vital signs.

**Table 44 - Treatment Emergent Adverse Events (at the time of the pre-specified analysis)**

| % (n) of patients^{a} All patients on background of max tolerated statin ± other lipid-lowering therapy | Alirocumab (n=1550) | Alirocumab with two consecutive LDL-C <25 mg/dL (n = 562; 36%) | Placebo (n=788) |
|---|---|---|---|
| TEAEs^{b} | 78.6% (1218) | 71.9% (404) | 80.6% (635) |
| Treatment-emergent SAEs | 16.5% (255) | 14.6% (82) | 17.6% (139) |
| TEAE leading to death | 0.5% (7) | 0.5% (3) | 1.0% (8) |
| TEAEs leading to discontinuation | 6.2% (96) | 3.6% (20) | 5.5% (43) |

| | | | |
|---|---|---|---|
| ^{a} Safety results from a first-step analysis (52 weeks for all patients continuing treatment, and including -600 patients exposed for 78 weeks). ^{b} TEAEs are adverse events that developed, worsened, or became serious during the TEAE period (time from first to last injection of study treatment + 70 days). | | | |

**Table 45 - TEAEs by System-Organ-Class (≥2%) in any Group (at the time of the pre-specified analysis)**

| % (n) of patients All patients on background of max tolerated statin ± other LLT | Alirocumab (n=1550) | Alirocumab with two consecutive LDL-C <25 mg/dL (n = 562; 36%) | Placebo (n=788) |
|---|---|---|---|
| Infections and infestations | 45.5% (705) | 39.0% (219) | 46.1% (363) |
| Musculoskeletal and connective tissue disorders | 27.2% (422) | 22.6% (127) | 28.6% (225) |
| Gastrointestinal disorders | 18.6% (288) | 13.7% (77) | 18.8% (148) |
| Nervous system disorders | 17.0% (264) | 11.2% (63) | 17.8% (140) |
| General disorders and administration site conditions | 15.4% (238) | 11.0% (62) | 17.0% (134) |
| Injury, poisoning, and procedural complications | 13.4% (207) | 10.5% (59) | 14.2% (112) |
| Respiratory, thoracic, and mediastinal disorders | 11.0% (171) | 7.7% (43) | 10.9% (86) |
| Cardiac disorders | 9.1% (141) | 7.5% (42) | 11.8% (93) |
| Skin and subcutaneous tissue disorders | 9.1% (141) | 6.9% (39) | 8.5% (67) |
| Metabolism and nutrition disorders | 9.1% (141) | 8.0% (45) | 8.4% (66) |
| Vascular disorders | 7.9% (122) | 5.0% (28) | 8.9% (70) |
| Eye disorders | 6.5% (100) | 6.4% (36) | 6.1% (48) |
| Investigations (lab parameters) | 6.1% (95) | 3.7% (21) | 5.2% (41) |
| Psychiatric disorders | 5.9% (91) | 4.3% (24) | 8.0% (63) |
| Renal and urinary disorders | 4.6% (72) | 3.6% (20) | 6.0% (47) |
| Neoplasms, benign, malignant (incl cysts/polyps) | 2.5% (38) | 3.0% (17) | 3.4% (27) |
| Reproductive system and breast disorders | 2.5% (38) | 2.0% (11) | 3.2% (25) |
| Blood and lymphatic system disorders | 2.4% (37) | 1.6% (9) | 3.0% (24) |
| Ear and labyrinth disorders | 2.0% (31) | 1.2% (7) | 2.9% (23) |

**Table 46 - TEAEs listed by patient subgroup (at the time of the pre-specified analysis)**

| TEAE % (n) of patients | Alirocumab (n=1550) | Alirocumab with two consecutive LDL-C <25 mg/dL (n = 562; 36%) | Placebo (n=788) |
|---|---|---|---|
| Nasopharyngitis | 12.6% (196) | 10.0% (56) | 12.7% (100) |
| Upper respiratory tract infection (URTI) | 7.0% (109) | 5.7% (32) | 8.0% (63) |
| Injection site reaction | 5.7% (89) | 3.6% (20) | 3.4% (34) |
| Influenza | 5.4% (84) | 4.1% (23) | 5.5% (43) |
| Diarrhea | 5.3% (82) | 3.9% (22) | 5.1% (40) |
| Urinary tract infection | 5.2% (81) | 5.5% (31) | 6.2% (49) |
| Bronchitis | 5.2% (80) | 5.2% (29) | 4.7% (37) |
| Myalgia | 4.9% (76) | 3.0% (17) | 3.0% (24) |
| Headache | 4.8% (74) | 1.8% (10) | 5.6% (44) |
| Back pain | 4.7% (73) | 5.0% (28) | 6.0% (47) |
| Arthralgia | 4.5% (70) | 3.2% (18) | 6.0% (47) |
| Muscle spasms | 3.7% (58) | 2.8% (16) | 3.2% (25) |
| Fatigue | 3.0% (47) | 3.0% (17) | 3.8% (30) |
| Pain in extremity | 3.0% (46) | 2.1% (12) | 4.4% (35) |
| Hypertension | 3.5% (54) | 2.0% (11) | 3.4% (27) |
| Cough | 3.2% (49) | 2.0% (11) | 2.2% (17) |
| Lower respiratory tract infection (LRTI) | 3.0% (46) | 2.8% (16) | 2.9% (23) |
| Fall | 2.8% (43) | 1.6% (9) | 4.1% (32) |
| Sinusitis | 2.6% (40) | 3.0% (17) | 2.4% (19) |
| Dizziness | 2.5% (38) | 1.4% (8) | 3.7% (29) |
| Gastoenteritis | 2.4% (37) | 0.7% (4) | 2.8% (22) |
| Nausea | 2.4% (37) | 0.9% (5) | 2.5% (20) |
| Musculoskeletal pain | 2.3% (36) | 1.4% (8) | 1.9% (15) |
| Osteoarthritis | 2.3% (35) | 2.1% (12) | 3.0% (24) |
| Confusion | 2.3% (35) | 1.2% (7) | 0.8% (6) |
| Angina pectoris | 2.1% (32) | 1.8% (10) | 2.9% (23) |
| Constipation | 1.9% (30) | 1.6% (9) | 1.8% (14) |
| Depression | 1.8% (28) | 1.4% (8) | 3.2% (25) |
| Non-cardiac chest pain | 1.8% (28) | 2.0% (11) | 1.9% (15) |
| Diabetes in patients with no history of diabetes^{a} | 19 | | 9 |
| Diabetes in patients with history of diabetes^{a} | 48 | | 22 |
| Type-2 diabetes mellitus | 1.7% (27) | 2.5% (14) | 1.3% (10) |
| Rhinitis | 1.4% (22) | 1.2% (7) | 2.2% (17) |
| Atrial fibrillation | 1.4% (22) | 1.6% (9) | 2.2% (17) |
| Cellulitis | 1.4% (22) | 1.6% (9) | 1.0% (8) |
| ALT increase | 1.1% | 0.7% | 0.5% |
| CPK increase | 0.5% | 0.2% | 0.5% |
| AST increase | 0.2% | 0% | 0% |

| | | | |
|---|---|---|---|
| Abbreviations: CHD, coronary heart disease; CV, cardiovascular; MI, myocardial infarction; LDL, low-density lipoprotein; LLN, lower limit of normal; LLT, lipid-lowering therapy; SAEs, serious adverse events; TEAEs, treatment-emergent adverse events; ULN, upper limit of normal. ^{a} The selection of preferred terms is based on Company MedDRA Query "diabetes" and includes the preferred terms "diabetes mellitus", "diabetes mellitus inadequate control", "fulminant type 1 diabetes mellitus", "insulin resistant diabetes", "insulin requiring type 2 diabetes mellitus", "type 1 diabetes mellitus", "type 2 diabetes mellitus", "diabetes mellitus malnutrition-related", and "diabetes mellitus management" and excluding "blood glucose decreased". | | | |

**Table 47 - Laboratory values of interest (at the time of the pre-specified analysis)**

| TEAE % (n) of patients | Alirocumab | Placebo |
|---|---|---|
| Alanine aminotransferase > 3 x ULN | 25/1532 (1.6) | 13/779 (1.7) |
| Aspartate aminiotransferase > 3 x ULN | 19/1532 (1.2) | 15/779 (1.9) |
| Creatine kinase >3 x ULN | 50/1507 (3.3) | 32/771 (4.2) |

**Table 48 - Adverse Events of Special Interest (at the time of the pre-specified analysis)**

| % (n) of patients All patients on background of max tolerated statin ± other lipid-lowering therapy | Alirocumab (n=1550) | Alirocumab with two consecutive LDL-C <25 mg/dL (n = 562;36%) | Placebo (n=788) |
|---|---|---|---|
| Treatment-emergent local injection site reactions | 5.8% (90) | 6.2% | 4.3% (34) |
| General allergic reaction events | 9.0% (140) | 8.0% | 9.0% (71) |
| Neurological events ‡^{a} | 4.2% (65) | 3.2% | 3.9% (31) |
| All adjudicated cardiovascular events† | 4.0% (62) | 4.3% | 4.4% (35) |
| Neurocognitive disorders ‡ | 1.2% (18) | 0.7% | 0.5% (4) |
| Ophthalmological events ‡ | 2.5% (38) | 2.5% | 1.9% (15) |
| Haemolytic anaemia | 0 | 0 | 0 |

| | | | |
|---|---|---|---|
| † Adjudicated CV events. Adjudicated CV events include all CV AEs positively adjudicated. The adjudication categories are the following: CHD death, non-fatal MI, fatal and non-fatal ischemic stroke, unstable angina requiring hospitalisation, congestive heart failure requiring hospitalisation, ischemia driven coronary revascularisation procedure [PCI, CABG]. ‡Company MedDRA Queries (CMQ)^{a} The selection of preferred terms is based on Standardized MedDRA Query including 'demyelination' (broad + narrow), 'peripheral neuropathy' (broad + narrow) and 'Guillain-Barre syndrome' (broad + narrow) excluding the preferred terms 'acute respiratory distress syndrome', 'asthenia', 'respiratory arrest' and 'respiratory failure'. | | | |

**Table 49 - Neurocognitive Adverse Events (at the time of the pre-specified analysis)**

| % (n) of patients All patients on background of max tolerated statin ± other lipid-lowering therapy | Alirocumab (n=1550) | Alirocumab with two consecutive LDL-C <25 mg/dL (n = 562; 36%) | Placebo (n=788) |
|---|---|---|---|
| Any neurocognitive disorder^{a} | 1.2% (18) | 0.7% | 0.5% (4) |
| Amnesia | 0.3% (5) | 0 | 0% (0) |
| Memory impairment | 0.3% (5) | 0 | 0.1% (1) |
| Confusional state | 0.3% (4) | 0.2% (1) | 0.1% (1) |
| Confusion postoperative | <0.1% (1) | 0 | 0% (0) |
| Dementia | <0.1% (1) | 0.2% (1) | 0.1% (1) |
| Disorientation | <0.1% (1) | 0 | 0% (0) |
| Disturbance in attention | <0.1% (1) | 0 | 0.1% (1) |
| Frontotemporal dementia | <0.1% (1) | 0.2% (1) | 0% (0) |
| Transient global amnesia | <0.1% (1) | 0 | 0% (0) |

| | | | |
|---|---|---|---|
| ^{a} The selection of preferred terms is based on Company MedDRA Query. | | | |

### RESULTS II- FINAL ANALYSIS

Although all of the primary and secondary efficacy endpoints were completed at the time of the pre-specified interim analysis, the study continued until completion, allowing analysis of the complete safety population.

### Summary safety population characteristics:

Mean study-drug exposure was 70 weeks in the 2338 patients included in the safety analysis (1550 in alirocumab and 788 in placebo groups), providing 2061 patient-years of exposure to alirocumab 150 mg every two weeks. Overall mean adherence to study treatment (i.e. the percentage of days that a patient took their injections as per the planned dosing schedule) was 98.0% and 97.6% in alirocumab and placebo groups, respectively. Mean duration of follow-up (regardless of treatment adherence) in the safety population was 80.9 weeks for alirocumab and 80.1 weeks for placebo. Study treatment discontinuation rates were 28% for alirocumab and 25% for placebo.

### Efficacy

A consistent LDL cholesterol reduction from baseline was observed from week 4 to 78 in the alirocumab group (Figure 15). Measured LDL cholesterol results were consistent with those for calculated LDL cholesterol. The percent decrease in LDL cholesterol was slightly lower at Week 78 (52%) versus Week 24 (61%) in the intention-to-treat analysis; this observation was influenced by values collected after premature treatment discontinuation.

### Safety

Similar percentages of patients experienced treatment-emergent adverse events in both treatment groups (81% with alirocumab versus 83% with placebo) (Table 50). Treatment-emergent adverse events leading to study-drug discontinuation occurred in 7.2% of alirocumab patients and 5.8% of placebo patients. With regard to specific adverse events, there were differences between the alirocumab and placebo groups in rates of injection-site reactions (5.9% versus 4.2%, respectively), myalgia (5.4% versus 2.9%, respectively), neurocognitive events (1.2% versus 0.5%, respectively), and ophthalmological events (2.9% versus 1.9%, respectively; Table 50).

Among the alirocumab patients, 575 (38% of the total) had two consecutive calculated LDL cholesterol levels of less than 25 mg/dL. Rates of treatment-emergent adverse events among these patients were comparable with those among the overall alirocumab group.

**Table 50. AEs of Interest and Safety Laboratory Values (Safety Analysis)**

| **No. of patients** | **Alirocumab** | **Placebo** | **P-value** |
|---|---|---|---|
| Patients on maximally tolerated statin ± other LLT | (N=1550) | (N=788) | |

| **Summary of TEAEs*** | | | |
|---|---|---|---|
| TEAEs | 1255 (81.0) | 650 (82.5) | 0.3983 |
| Treatment-emergent SAEs | 290 (18.7) | 154 (19.5) | 0.6555 |
| TEAEs leading to death | 8 (0.5) | 10 (1.3) | 0.0760 |
| TEAEs leading to discontinuation | 111 (7.2) | 46 (5.8) | 0.2559 |

| **Cardiovascular AEs of interest** | | | |
|---|---|---|---|
| CHD death including unknown cause | 4 (0.3) | 7 (0.9) | 0.2559 |
| Non-fatal MI | 14 (0.9) | 18 (2.3) | 0.0129 |
| Fatal and non-fatal ischemic stroke | 9 (0.6) | 2 (0.3) | 0.3528 |
| Unstable angina requiring | 0 | 1 (0.1) | 0.337 |
| hospitalization | | | |
| Congestive heart failure requiring hospitalization | 9 (0.6) | 3 (0.4) | 0.761 |
| Ischemia-driven coronary | 48 (3.1) | 24 (3.0) | 1 |
| revascularization procedure | | | |
| Positively adjudicated cardiovascular events (including all cardiovascular AEs listed above) | 72 (4.6) | 40 (5.1) | 0.682 |
| *Post hoc* analysis of a subgroup of adjudicated MACE^{†} | 27 (1.7) | 26 (3.3) | 0.0116 |

| **Other AEs of interest** | | | |
|---|---|---|---|
| General allergic reaction events | 156 (10.1) | 75 (9.5) | 0.7142 |
| Local injection site reaction | 91 (5.9) | 33 (4.2) | 0.0968 |
| Myalgia | 84 (5.4) | 23 (2.9) | 0.0063 |
| Neurological events^{‡} | 65 (4.2) | 35 (4.4) | 0.8289 |
| Neurocognitive disorders^{§} | 18 (1.2) | 4 (0.5) | 0.1727 |
| Amnesia | 5 (0.3) | 0 | 0.1747 |
| Memory impairment | 4 (0.3) | 1 (0.1) | 0.6686 |
| Confusional state | 4 (0.3) | 1 (0.1) | 0.6686 |
| Ophthalmological events^{¶} | 35 (2.9) | 15 (1.9) | 0.6516 |
| Hemolytic anemia | 0 | 0 | NC |
| Diabetes in patients with no history of diabetes, n/N (%)" | 18/994 (1.8) | 10/509 (2.0) | 0.8419 |
| Worsening of diabetes in patients with history of diabetes, n/N (%)" | 72/556 (12.9) | 38/279 (13.6) | 0.8284 |

| **Laboratory values of interest** | | | |
|---|---|---|---|
| Alanine aminotransferase >3 times ULN, n/N (%) | 28/1533 (1.8) | 16/779 (2.1) | 0.748 |
| Aspartate aminotransferase >3 times ULN, n/N (%) | 22/1533 (1.4) | 18/779 (2.3) | 0.1316 |
| Creatine kinase >3 times ULN, n/N (%) | 56/1507 (3.7) | 38/771 (4.9) | 0.1819 |

| | | | |
|---|---|---|---|
| P-values calculated using Fisher exact test and not adjusted for multiplicity. Provided for descriptive purpose only. | | | |

### Overall Summary

This study is the largest and longest double-blind study of a PCSK9 inhibitor. The current analysis provides about 1900 patient-years of double-blind patient exposure to alirocumab 150 mg Q2W. In high CV risk patients on maximally-tolerated statin ± other lipid lowering therapy the following observations were made: 1) self-administered alirocumab treatment produced significantly greater LDL-C reductions than placebo at Week 24 (LS mean difference -61.9%); efficacy remained consistent throughout treatment, and at week 78 alirocumab reduced LDL-C from baseline by an additional 56 percent versus placebo; 2) 79% of alirocumab patients achieved LDL-C goal of <0.81 mmol/L (70 mg/dL) at Week 24; 3) reductions in LDL-C levels were observed in alirocumab patients by Week 4 and alirocumab patients achieved mean LDL-C levels of 1.4 mmol/L (53.1 mg/dL) at Week 52; and 4) TEAEs occurred in a similar frequency in the alirocumab and placebo arms as well as a subset of alirocumab-treated patients (n = 562) with two consecutive LDL-C levels of <25 mg/dL.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

The invention also pertains the following:
1. A method for treating a high cardiovascular risk patient with hypercholesterolemia and established coronary heart disease (CHD) or CHD risk equivalents that are not adequately controlled by maximum tolerated dose statin therapy with or without other lipid lowering therapy comprising administering one or more doses of a proprotein convertase subtilisin/kexin type 9 (PCSK9) inhibitor to the patient, wherein the patient exhibits inadequate control of the hypercholesterolemia despite treatment with the maximum tolerated dose statin therapy with or without other lipid lowering therapy in the absence of the PCSK9 inhibitor.
2. The method of item1, wherein the established CHD is defined by a history of CHD, including one or more of the following: acute myocardial infarction (MI), silent MI, unstable angina, coronary revascularization procedure, and clinically significant CHD diagnosed by invasive or non-invasive testing.
3. The method of item 1, wherein the CHD risk equivalents include one or more of the following four criteria: 1) peripheral arterial disease (PAD); 2) ischemic stroke; 3) chronic kidney disease; and/or 4) a history of diabetes mellitus and 2 or more additional risk factors, including: a) history of hypertension, b) history of ankle-brachial index ≤0.90, c) history of microalbuminuria or macroalbuminuria or dipstick urinalysis with >2+ protein, d) history of preproliferative or proliferative retinopathy or laser treatment for retinopathy, and e) known family history of premature CHD.
4. The method of any one of items1 to 3, wherein the PCSK9 inhibitor is an antibody or an antigen-binding fragment thereof that specifically binds PCSK9.
5. The method of item 4, wherein the antibody or antigen-binding fragment thereof comprises the heavy and light chain complementarity determining regions (CDRs) of a heavy chain variable region/light chain variable region (HCVR/LCVR) amino acid sequence pair selected from the group consisting of SEQ ID NOs: 1/6 and 11/15.
6. The method of item 5, wherein the antibody or antigen-binding fragment thereof comprises heavy and light chain CDR amino acid sequences having SEQ ID NOs:12, 13, 14, 16, 17, and 18.
7. The method of item 6, wherein the antibody or antigen-binding fragment thereof comprises an HCVR having the amino acid sequence of SEQ ID NO:11 and an LCVR having the amino acid sequence of SEQ ID NO:15.
8. The method of item 5, wherein the antibody or antigen-binding fragment thereof comprises heavy and light chain CDR amino acid sequences having SEQ ID NOs:2, 3, 4, 7, 8, and 10.
9. The method of item 8, wherein the antibody or antigen-binding fragment thereof comprises an HCVR having the amino acid sequence of SEQ ID NO:1 and an LCVR having the amino acid sequence of SEQ ID NO:6.
10. The method of item 4, wherein the antibody or antigen-binding fragment thereof binds to the same epitope on PCSK9 as an antibody comprising heavy and light chain CDR amino acid sequences having SEQ ID NOs: 12, 13, 14, 16, 17, and 18; or SEQ ID NOs: 2, 3, 4, 7, 8, and 10.
11. The method of item 4, wherein the antibody or antigen-binding fragment thereof competes for binding to PCSK9 with an antibody comprising heavy and light chain CDR amino acid sequences having SEQ ID NOs: 12, 13, 14, 16, 17, and 18; or SEQ ID NOs: 2, 3, 4, 7, 8 and 10.
12. The method of item 4, wherein the antibody or antigen-binding fragment thereof that specifically binds PCSK9 is administered to the patient at a dose of about 75 mg at a frequency of once every two weeks.
13. The method of item 12, wherein the about 75 mg dose is maintained if the patient's LDL-C measured after five or more doses is <70 mg/dL.
14. The method of item 12, wherein the about 75 mg dose is discontinued if the patient's LDL-C measured after five or more doses remains ≥70 mg/dL, and the antibody or antigen-binding fragment thereof that specifically binds PCSK9 is subsequently administered to the patient at a dose of about 150 mg at a frequency of once every two weeks.
15. The method of item 4, wherein the antibody or antigen-binding fragment thereof that specifically binds PCSK9 is administered to the patient at a dose of about 150 mg at a frequency of once every two weeks.
16. The method of any one of items 1 to 15, wherein the PCSK9 inhibitor is administered to the patient in combination with the maximum tolerated dose statin therapy.
17. The method of any one of items1 to 16, wherein the maximum tolerated dose statin therapy comprises a daily dose of about 40 mg to about 80 mg of atorvastatin.
18. The method of any one of items 1 to 16, wherein the maximum tolerated dose statin therapy comprises a daily dose of about 20 mg to about 40 mg of rosuvastatin.
19. The method of any one of items 1 to 16, wherein the maximum tolerated dose statin therapy comprises a daily dose of about 80 mg of simvastatin.
20. The method of any one of items1 to 19, wherein the PCSK9 inhibitor is administered to the patient in combination with the other lipid lowering therapy, wherein the other lipid lowering therapy comprises a therapeutic agent selected from the group consisting of ezetimibe, a fibrate, niacin, an omega-3 fatty acid, and a bile acid resin.
21. The method of any one of items 1-20, wherein the method improves at least one hypercholesterolemia-associated parameter selected from the group consisting of:
   (a) reduction of the patient's low density lipoprotein cholesterol (LDL-C) by at least 40%;
   (b) reduction of the patient's apolipoprotein B100 (ApoB) by at least 35%;
   (c) reduction of the patient's non-high density lipoproprotein cholesterol (non-HDL-C) by at least 35%;
   (d) reduction of the patient's total cholesterol by at least 20%;
   (e) increase of the patient's high density lipoprotein cholesterol (HDL-C) by at least 1%;
   (f) reduction of the patient's triglycerides by at least 1%;
   (g) reduction of the patient's lipoprotein a (Lp(a)) by at least 10%; and
   (h) increase of the patient's apolipoprotein A1 by at least 1%.
22. A method for reducing low-density lipoprotein cholesterol (LDL-C) in a high cardiovascular risk patient with hypercholesterolemia and established coronary heart disease (CHD) or CHD risk equivalents that is not adequately controlled by maximum tolerated dose statin therapy with or without other lipid lowering therapy comprising administering one or more doses of a proprotein convertase subtilisin/kexin type 9 (PCSK9) inhibitor to the patient, wherein the patient exhibits inadequate control of LDL-C despite treatment with a maximum tolerated dose statin therapy with or without other lipid lowering therapy in the absence of the PCSK9 inhibitor.
23. The method of item 22, wherein the established CHD is defined by a history of CHD, including one or more of the following: acute myocardial infarction (MI), silent MI, unstable angina, coronary revascularization procedure, and clinically significant CHD diagnosed by invasive or non-invasive testing.
24. The method of item 22, wherein the CHD risk equivalents include one or more of the following four criteria: 1) peripheral arterial disease (PAD); 2) ischemic stroke; 3) chronic kidney disease; and/or 4) a history of diabetes mellitus and 2 or more additional risk factors, including: a) history of hypertension, b) history of ankle-brachial index ≤0.90, c) history of microalbuminuria or macroalbuminuria or dipstick urinalysis at screening visit with >2+ protein, d) history of preproliferative or proliferative retinopathy or laser treatment for retinopathy, and e) known family history of premature CHD.
25. The method of any one of items 22 to 24, wherein the PCSK9 inhibitor is an antibody or an antigen-binding fragment thereof that specifically binds PCSK9.
26. The method of item 25, wherein the antibody or antigen-binding fragment thereof comprises the heavy and light chain complementarity determining regions (CDRs) of a heavy chain variable region/light chain variable region (HCVR/LCVR) amino acid sequence pair selected from the group consisting of SEQ ID NOs: 1/6 and 11/15.
27. The method of item 26, wherein the antibody or antigen-binding fragment thereof comprises heavy and light chain CDR amino acid sequences having SEQ ID NOs: 12, 13, 14, 16, 17, and 18.
28. The method of item 27, wherein the antibody or antigen-binding fragment thereof comprises an HCVR having the amino acid sequence of SEQ ID NO:11 and an LCVR having the amino acid sequence of SEQ ID NO:15.
29. The method of item 26, wherein the antibody or antigen-binding fragment thereof comprises heavy and light chain CDR amino acid sequences having SEQ ID NOs:2, 3, 4, 7, 8, and 10.
30. The method of item 29, wherein the antibody or antigen-binding fragment thereof comprises an HCVR having the amino acid sequence of SEQ ID NO:1 and an LCVR having the amino acid sequence of SEQ ID NO:6.
31. The method of item 25, wherein the antibody or antigen-binding fragment thereof binds to the same epitope on PCSK9 as an antibody comprising heavy and light chain CDR amino acid sequences having SEQ ID NOs: 12, 13, 14, 16, 17, and 18; or SEQ ID NOs: 2, 3, 4, 7, 8 and 10.
32. The method of item 25, wherein the antibody or antigen-binding fragment thereof competes for binding to PCSK9 with an antibody comprising heavy and light chain CDR amino acid sequences having SEQ ID NOs: 12, 13, 14, 16, 17, and 18; or SEQ ID NOs: 2, 3, 4, 7, 8 and 10.
33. The method of item 25, wherein the antibody or antigen-binding fragment thereof that specifically binds PCSK9 is administered to the patient at a dose of about 75 mg at a frequency of once every two weeks.
34. The method of item 33, wherein the about 75 mg dose is maintained if the patient's LDL-C measured after five or more doses is <70 mg/dL.
35. The method of item 33, wherein the about 75 mg dose is discontinued if the patient's LDL-C measured after five or more doses remains ≥70 mg/dL, and the antibody or antigen-binding fragment thereof that specifically binds PCSK9 is subsequently administered to the patient at a dose of about 150 mg at a frequency of once every two weeks.
36. The method of item 33, wherein the antibody or antigen-binding fragment thereof that specifically binds PCSK9 is administered to the patient at a dose of about 150 mg at a frequency of once every two weeks.
37. The method of any one of items 22 to 36, wherein the PCSK9 inhibitor is administered to the patient in combination with the maximum tolerated dose statin therapy.
38. The method of any one of items 22 to 37, wherein the maximum tolerated dose statin therapy comprises a daily dose of about 40 mg to about 80 mg of atorvastatin.
39. The method of any one of items 22 to 37, wherein the maximum tolerated dose statin therapy comprises a daily dose of about 20 mg to about 40 mg of rosuvastatin.
40. The method of any one of items 22 to 37, wherein the maximum tolerated dose statin therapy comprises a daily dose of about 80 mg of simvastatin.
41. The method of any one of items 22 to 39, wherein the PCSK9 inhibitor is administered to the patient in combination with the other lipid lowering therapy, wherein the other lipid lowering therapy comprises a therapeutic agent selected from the group consisting of ezetimibe, a fibrate, niacin, an omega-3 fatty acid, and a bile acid resin.
42. The method of any one of items 22-41, wherein the method improves at least one hypercholesterolemia-associated parameter selected from the group consisting of:
   (a) reduction of the patient's low density lipoprotein cholesterol (LDL-C) by at least 40%;
   (b) reduction of the patient's apolipoprotein B100 (ApoB) by at least 35%;
   (c) reduction of the patient's non-high density lipoproprotein cholesterol (non-HDL-C) by at least 35%;
   (d) reduction of the patient's total cholesterol by at least 20%;
   (e) increase of the patient's high density lipoprotein cholesterol (HDL-C) by at least 1%;
   (f) reduction of the patient's triglycerides by at least 1%;
   (g) reduction of the patient's lipoprotein a (Lp(a)) by at least 10%; and
   (h) increase of the patient's apolipoprotein A1 by at least 1%.
43. A method for treating hypercholesterolemia in a high cardiovascular risk patient with hypercholesterolemia and established coronary heart disease (CHD) or CHD risk equivalents that are not adequately controlled by maximum tolerated dose statin therapy with or without other lipid lowering therapy comprising administering one or more doses of a proprotein convertase subtilisin/kexin type 9 (PCSK9) inhibitor to the patient, wherein the patient exhibits inadequate control of the hypercholesterolemia despite treatment with the maximum tolerated dose statin therapy with or without other lipid lowering therapy in the absence of the PCSK9 inhibitor.
44. The method of item 43, wherein the established CHD is defined by a history of CHD, including one or more of the following: acute myocardial infarction (MI), silent MI, unstable angina, coronary revascularization procedure, and clinically significant CHD diagnosed by invasive or non-invasive testing.
45. The method of item 43, wherein the CHD risk equivalents include one or more of the following four criteria: 1) peripheral arterial disease (PAD); 2) ischemic stroke; 3) chronic kidney disease (CKD); and/or 4) history of diabetes mellitus and 2 or more additional risk factors, including: a) history of hypertension, b) history of ankle-brachial index ≤0.90, c) history of microalbuminuria or macroalbuminuria or dipstick urinalysis at screening visit with >2+ protein, d) history of preproliferative or proliferative retinopathy or laser treatment for retinopathy, and e) known family history of premature CHD.
46. The method of any one of items 43 to 45, wherein the PCSK9 inhibitor is an antibody or an antigen-binding fragment thereof that specifically binds PCSK9.
47. The method of item 46, wherein the antibody or antigen-binding fragment thereof comprises the heavy and light chain complementarity determining regions (CDRs) of a heavy chain variable region/light chain variable region (HCVR/LCVR) amino acid sequence pair selected from the group consisting of SEQ ID NOs: 1/6 and 11/15.
48. The method of item 47, wherein the antibody or antigen-binding fragment thereof comprises heavy and light chain CDR amino acid sequences having SEQ ID NOs: 12, 13, 14, 16, 17, and 18.
49. The method of item 48, wherein the antibody or antigen-binding fragment thereof comprises an HCVR having the amino acid sequence of SEQ ID NO:11 and an LCVR having the amino acid sequence of SEQ ID NO:15.
50. The method of item 47, wherein the antibody or antigen-binding fragment thereof comprises heavy and light chain CDR amino acid sequences having SEQ ID NOs:2, 3, 4, 7, 8 and 10.
51. The method of item 50, wherein the antibody or antigen-binding fragment thereof comprises an HCVR having the amino acid sequence of SEQ ID NO:1 and an LCVR having the amino acid sequence of SEQ ID NO:6.
52. The method of item 46, wherein the antibody or antigen-binding fragment thereof binds to the same epitope on PCSK9 as an antibody comprising heavy and light chain CDR amino acid sequences having SEQ ID NOs: 12, 13, 14, 16, 17, and 18; or SEQ ID NOs: 2, 3, 4, 7, 8, and 10.
53. The method of item 46, wherein the antibody or antigen-binding fragment thereof competes for binding to PCSK9 with an antibody comprising heavy and light chain CDR amino acid sequences having SEQ ID NOs: 12, 13, 14, 16, 17, and 18; or SEQ ID NOs: 2, 3, 4, 7, 8, and 10.
54. The method of item 46, wherein the antibody or antigen-binding fragment thereof that specifically binds PCSK9 is administered to the patient at a dose of about 75 mg at a frequency of once every two weeks.
55. The method of item 54, wherein the about 75 mg dose is maintained if the patient's LDL-C measured after five or more doses is <70 mg/dL.
56. The method of item 54, wherein the about 75 mg dose is discontinued if the patient's LDL-C measured after five or more doses remains ≥70 mg/dL, and the antibody or antigen-binding fragment thereof that specifically binds PCSK9 is subsequently administered to the patient at a dose of about 150 mg at a frequency of once every two weeks.
57. The method of item 46, wherein the antibody or antigen-binding fragment thereof that specifically binds PCSK9 is administered to the patient at a dose of about 150 mg at a frequency of once every two weeks.
58. The method of any one of items 43 to 57, wherein the PCSK9 inhibitor is administered to the patient in combination with the maximum tolerated dose statin therapy.
59. The method of any one of items 43 to 58, wherein the maximum tolerated dose statin therapy comprises a daily dose of about 40 mg to about 80 mg of atorvastatin.
60. The method of any one of items 43 to 58, wherein the maximum tolerated dose statin therapy comprises a daily dose of about 20 mg to about 40 mg of rosuvastatin.
61. The method of any one of items 43 to 58, wherein the maximum tolerated dose statin therapy comprises a daily dose of about 80 mg of simvastatin.
62. The method of any one of items 43 to 61, wherein the PCSK9 inhibitor is administered to the patient in combination with the other lipid lowering therapy, wherein the other lipid lowering therapy comprises a therapeutic agent selected from the group consisting of ezetimibe, a fibrate, niacin, an omega-3 fatty acid, and a bile acid resin.
63. The method of any one of items 43-62, wherein the method improves at least one hypercholesterolemia-associated parameter selected from the group consisting of:
   (a) reduction of the patient's low density lipoprotein cholesterol (LDL-C) by at least 40%;
   (b) reduction of the patient's apolipoprotein B100 (ApoB) by at least 35%;
   (c) reduction of the patient's non-high density lipoproprotein cholesterol (non-HDL-C) by at least 35%;
   (d) reduction of the patient's total cholesterol by at least 20%;
   (e) increase of the patient's high density lipoprotein cholesterol (HDL-C) by at least 1%;
   (f) reduction of the patient's triglycerides by at least 1%;
   (g) reduction of the patient's lipoprotein a (Lp(a)) by at least 10%; and
   (h) increase of the patient's apolipoprotein A1 by at least 1%.
64. A method for improving the serum level of one or more lipid components in a high cardiovascular risk patient with hypercholesterolemia and established coronary heart disease (CHD) or CHD risk equivalents that are not adequately controlled by maximum tolerated dose statin therapy with or without other lipid lowering therapy comprising administering one or more doses of a proprotein convertase subtilisin/kexin type 9 (PCSK9) inhibitor to the patient, wherein the patient exhibits inadequate control of the lipid component despite treatment with the maximum tolerated dose statin therapy with or without other lipid lowering therapy in the absence of the PCSK9 inhibitor, wherein the improvement in the serum level of one or more lipid components is selected from the group consisting of:
   (a) a reduction of the patient's low density lipoprotein cholesterol (LDL-C) by at least 40%;
   (b) a reduction of the patient's apolipoprotein B100 (ApoB) by at least 35%;
   (c) a reduction of the patient's non-high density lipoproprotein cholesterol (non-HDL-C) by at least 35%;
   (d) a reduction of the patient's total cholesterol by at least 20%;
   (e) an increase of the patient's high density lipoprotein cholesterol (HDL-C) by at least 1%;
   (f) a reduction of the patient's triglycerides by at least 1%;
   (g) a reduction of the patient's lipoprotein a (Lp(a)) by at least 10%; and
   (h) an increase of the patient's apolipoprotein A1 by at least 1%.
65. The method of item 64, wherein the established CHD is defined by a history of CHD, including one or more of the following: acute myocardial infarction (MI), silent MI, unstable angina, coronary revascularization procedure, and clinically significant CHD diagnosed by invasive or non-invasive testing.
66. The method of item 64, wherein the CHD risk equivalents include one or more of the following four criteria: 1) peripheral arterial disease (PAD); 2) ischemic stroke; 3) chronic kidney disease; and/or 4) known history of diabetes mellitus and 2 or more additional risk factors, including: a) history of hypertension, b) history of ankle-brachial index ≤0.90, c) history of microalbuminuria or macroalbuminuria or dipstick urinalysis at screening visit with >2+ protein, d) history of preproliferative or proliferative retinopathy or laser treatment for retinopathy, and e) known family history of premature CHD.
67. The method of any one of items 64 to 66, wherein the PCSK9 inhibitor is an antibody or an antigen-binding fragment thereof that specifically binds PCSK9.
68. The method of item 67, wherein the antibody or antigen-binding fragment thereof comprises the heavy and light chain complementarity determining regions (CDRs) of a heavy chain variable region/light chain variable region (HCVR/LCVR) amino acid sequence pair selected from the group consisting of SEQ ID NOs: 1/6 and 11/15.
69. The method of item 68, wherein the antibody or antigen-binding fragment thereof comprises heavy and light chain CDR amino acid sequences having SEQ ID NOs: 12, 13, 14, 16, 17, and 18.
70. The method of item 69, wherein the antibody or antigen-binding fragment thereof comprises an HCVR having the amino acid sequence of SEQ ID NO:11 and an LCVR having the amino acid sequence of SEQ ID NO:15.
71. The method of item 68, wherein the antibody or antigen-binding fragment thereof comprises heavy and light chain CDR amino acid sequences having SEQ ID NOs:2, 3, 4, 7, 8, and 10.
72. The method of item 71, wherein the antibody or antigen-binding fragment thereof comprises an HCVR having the amino acid sequence of SEQ ID NO:1 and an LCVR having the amino acid sequence of SEQ ID NO:6.
73. The method of item 67, wherein the antibody or antigen-binding fragment thereof binds to the same epitope on PCSK9 as an antibody comprising heavy and light chain CDR amino acid sequences having SEQ ID NOs: 12, 13, 14, 16, 17, and 18; or SEQ ID NOs: 2, 3, 4, 7, 8, and 10.
74. The method of item 67, wherein the antibody or antigen-binding fragment thereof competes for binding to PCSK9 with an antibody comprising heavy and light chain CDR amino acid sequences having SEQ ID NOs: 12, 13, 14, 16, 17, and 18; or SEQ ID NOs: 2, 3, 4, 7, 8, and 10.
75. The method of item 67, wherein the antibody or antigen-binding fragment thereof that specifically binds PCSK9 is administered to the patient at a dose of about 75 mg at a frequency of once every two weeks.
76. The method of item 75, wherein the about 75 mg dose is maintained if the patient's LDL-C measured after five or more doses is <70 mg/dL.
77. The method of item 75, wherein the about 75 mg dose is discontinued if the patient's LDL-C measured after five or more doses remains ≥70 mg/dL, and the antibody or antigen-binding fragment thereof that specifically binds PCSK9 is subsequently administered to the patient at a dose of about 150 mg at a frequency of once every two weeks.
78. The method of item 75, wherein the antibody or antigen-binding fragment thereof that specifically binds PCSK9 is administered to the patient at a dose of about 150 mg at a frequency of once every two weeks.
79. The method of any one of items 64 to 78, wherein the PCSK9 inhibitor is administered to the patient in combination with the maximum tolerated dose statin therapy.
80. The method of any one of items 64 to 79, wherein the maximum tolerated dose statin therapy comprises a daily dose of about 40 mg to about 80 mg of atorvastatin.
81. The method of any one of items 64 to 79, wherein the maximum tolerated dose statin therapy comprises a daily dose of about 20 mg to about 40 mg of rosuvastatin.
82. The method of any one of items 64 to 79, wherein the maximum tolerated dose statin therapy comprises a daily dose of about 80 mg of simvastatin.
83. The method of any one of items 64 to 82, wherein the PCSK9 inhibitor is administered to the patient in combination with the other lipid lowering therapy, wherein the other lipid lowering therapy comprises a therapeutic agent selected from the group consisting of ezetimibe, a fibrate, niacin, an omega-3 fatty acid, and a bile acid resin.
84. The method of item 64, wherein the lipid component is LDL-C and the reduction in serum level of LDL-C is compared to baseline.
85. The method of item 64, wherein the lipid component is Apo B, and the reduction in serum level of Apo B is compared to baseline.
86. The method of item 64, wherein the lipid component is non-HDL-C and the reduction in serum level of non-HDL-C is compared to baseline.
87. The method of item 64, wherein the lipid component is total cholesterol and the reduction in serum level of total cholesterol is compared to baseline.
88. The method of item 64, wherein the lipid component is Lp(a) and the reduction in serum level of Lp(a) is compared to baseline.
89. The method of item 64, wherein the lipid component is triglycerides and the reduction in serum level of triglycerides is compared to baseline.
90. The method of item 64, wherein the lipid component is Apo A1 and the reduction in serum level of Apo A1 is compared to baseline.
91. The method of item 64, wherein the lipid component is HDL-C and the reduction in serum level of HDL-C is compared to baseline.

## Claims

1. A human proprotein convertase subtilisin/kexin type 9 (PCSK9) inhibitor for use in the treatment of a high cardiovascular risk patient who has (a) hypercholesterolemia and (b) established coronary heart disease (CHD) or CHD risk equivalents.

2. The PCSK9 inhibitor for use according to claim 1, wherein the PCSK9 inhibitor is an antibody or antigen-binding fragment thereof which specifically binds PCSK9.

3. The PCSK9 inhibitor for use according to claim 2, wherein the antibody or antigen-binding fragment thereof comprises (i) the three heavy chain CDRs (HCDR1, HCDR2 and HCDR3) contained within a heavy chain variable region (HCVR) having an amino acid sequence according to SEQ ID NO: 1, or a variant thereof having at least 90% sequence identity to SEQ ID NO: 1, and (ii) the three light chain CDRs (LCVR1, LCVR2, LCVR3) contained within a light chain variable region (LCVR) having an amino acid sequence according to SEQ ID NOs: 6, or a variant thereof having at least 90% sequence identity to SEQ ID NO: 1.

4. The PCSK9 inhibitor for use according to claim 3, wherein the antibody or antigen-binding fragment thereof comprises the three heavy chain CDRs set forth in SEQ ID NOs: 2, 3, and 4 or variants thereof with one conservative amino acid substitution, and the three light chain CDRs set forth in SEQ ID NOs: 7, 8, and 10 or variants thereof with one conservative amino acid substitution.

5. The PCSK9 inhibitor for use according to any one of claims 2-4, wherein the antibody or antigen-binding fragment thereof has a K_{D} of less than about 1 nM or less than about 0.5 nM.

6. The PCSK9 inhibitor for use according to any one of claims 2-5, wherein the antibody or antigen-binding fragment thereof comprises the heavy chain variable region (HCVR) amino acid sequence set forth in SEQ ID NO: 1, and the light chain variable region (LCVR) amino acid sequence set forth in SEQ ID NO: 6.

7. The PCSK9 inhibitor for use according to any one of claims 1-6, wherein the patient has a serum low-density lipoprotein cholesterol (LDL-C) level above 70 mg/dL despite taking a stable daily maximally tolerated dose of stating therapy for at least 4 weeks.

8. The PCSK9 inhibitor for use according to any one of claims 1-7, wherein the CHD risk equivalents are defined by including one or more of the following four criteria: 1) peripheral arterial disease (PAD); 2) ischemic stroke; 3) chronic kidney disease; and/or 4) a history of diabetes mellitus and 2 or more additional risk factors, including: a) history of hypertension, b) history of ankle-brachial index ≤0.90, c) history of microalbuminuria or macroalbuminuria or dipstick urinalysis at screening visit with >2+ protein, d) history of preproliferative or proliferative retinopathy or laser treatment for retinopathy, and e) known family history of premature CHD

9. The PCSK9 inhibitor for use according to any one of claims 1-8, wherein the antibody or antigen-binding fragment thereof is administered at a dose of about 75 mg, about 150 mg or about 300 mg.

10. The PCSK9 inhibitor for use according to any one of claims 1-9, wherein the antibody or antigen-binding fragment thereof is administered at a dose of about 75 mg or about 150 mg every two weeks.

11. The PCSK9 inhibitor for use according to any one of claims 1-10, wherein the PCSK9 inhibitor is administered to the patient in combination with the maximum tolerated dose statin therapy.

12. The PCSK9 inhibitor for use according to any one of claims 1-10, wherein the antibody or antigen-binding fragment thereof is administered subcutaneously.

13. A pharmaceutical composition comprising the PCSK9 inhibitor for use according to any one of claims 1-12.

14. The pharmaceutical composition of claim 13, wherein the pharmaceutical composition is administered by bolus injection.

15. The pharmaceutical composition of claim 13 or 14, wherein administration of the pharmaceutical composition results in a mean percent reduction from baseline in serum low density lipoprotein cholesterol (LDL-C) of at least about 57%.


SEQUENCE LISTING

<110> Sanofi Biotechnology et.al

<120> METHODS FOR TREATING HIGH CARDIOVASCULAR RISK PATIENTS WITH
HYPERCHOLESTEROLEMIA

<130> 589-262 PCTEPT1

<150> US 62/025,371
<151> 2014-07-16

<150> US 62/043,167
<151> 2014-08-28

<150> US 62/080,725
<151> 2014-11-17

<150> US 62/132,709
<151> 2015-03-13

<150> EP15305830.0
<151> 2015-05-29

<150> PCT/US2015/040765
<151> 2015-07-16

<151> EP 15 760 317.6
<151> 2015-07-16

<160> 198

<170> PatentIn version 3.5

<210> 1
<211> 118
<212> PRT
<213> Artificial Sequence

<220>
<223> Description of Artificial Sequence: Synthetic
      polypeptide

<400> 1

<210> 2
<211> 8
<212> PRT
<213> Artificial Sequence

<220>
<223> Description of Artificial Sequence: Synthetic
      peptide

<400> 2
Gly Phe Thr Phe Asn Asn Tyr Ala
1               5

<210> 3
<211> 8
<212> PRT
<213> Artificial Sequence

<220>
<223> Description of Artificial Sequence: Synthetic
      peptide

<400> 3
Ile Ser Gly Ser Gly Gly Thr Thr
1               5

<210> 4
<211> 11
<212> PRT
<213> Artificial Sequence

<220>
<223> Description of Artificial Sequence: Synthetic
      peptide

<400> 4
Ala Lys Asp Ser Asn Trp Gly Asn Phe Asp Leu
1               5                   10

<210> 5
<211> 447
<212> PRT
<213> Artificial Sequence

<220>
<223> Description of Artificial Sequence: Synthetic
      REGN727 heavy chain polypeptide

<400> 5
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Asn Tyr
            20                  25                  30

Ala Met Asn Trp Val Arg Gln Ala Pro Gly Lys Gly Leu Asp Trp Val
        35                  40                  45

Ser Thr Ile Ser Gly Ser Gly Gly Thr Thr Asn Tyr Ala Asp Ser Val
    50                  55                  60

Lys Gly Arg Phe Ile Ile Ser Arg Asp Ser Ser Lys His Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Ser Asn Trp Gly Asn Phe Asp Leu Trp Gly Arg Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg

245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly
        435                 440                 445

<210> 6
<211> 113
<212> PRT
<213> Artificial Sequence

<220>
<223> Description of Artificial Sequence: Synthetic
      polypeptide

<400> 6

<210> 7
<211> 12
<212> PRT
<213> Artificial Sequence

<220>
<223> Description of Artificial Sequence: Synthetic
      peptide

<400> 7
Gln Ser Val Leu Tyr Arg Ser Asn Asn Arg Asn Phe
1               5                   10

<210> 8
<211> 3
<212> PRT
<213> Artificial Sequence

<220>
<223> Description of Artificial Sequence: Synthetic
      peptide

<400> 8
Trp Ala Ser
1

<210> 9
<211> 220
<212> PRT
<213> Artificial Sequence

<220>
<223> Description of Artificial Sequence: Synthetic
      REGN727 light chain polypeptide

<400> 9
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Leu Tyr Arg
            20                  25                  30

Ser Asn Asn Arg Asn Phe Leu Gly Trp Tyr Gln Gln Lys Pro Gly Gln
        35                  40                  45

Pro Pro Asn Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Thr Thr Pro Tyr Thr Phe Gly Gln Gly Thr Lys Leu Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210> 10
<211> 9

<212> PRT
<213> Artificial Sequence

<220>
<223> Description of Artificial Sequence: Synthetic
      peptide

<400> 10
Gln Gln Tyr Tyr Thr Thr Pro Tyr Thr
1               5


<210> 11
<211> 127
<212> PRT
<213> Artificial Sequence

<220>
<223> Description of Artificial Sequence: Synthetic
      polypeptide

<400> 11
Glu Met Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Ser His
            20                  25                  30


Trp Met Lys Trp Val Arg Gln Ala Pro Gly Lys Gly Leu Glu Trp Val
        35                  40                  45


Ala Asn Ile Asn Gln Asp Gly Ser Glu Lys Tyr Tyr Val Asp Ser Val
    50                  55                  60


Lys Gly Arg Phe Thr Ile Ser Arg Asp Asn Ala Lys Asn Ser Leu Phe
65                  70                  75                  80


Leu Gln Met Asn Ser Leu Arg Ala Glu Asp Thr Ala Val Tyr Tyr Cys
                85                  90                  95


Ala Arg Asp Ile Val Leu Met Val Tyr Asp Met Asp Tyr Tyr Tyr Tyr
            100                 105                 110


Gly Met Asp Val Trp Gly Gln Gly Thr Thr Val Thr Val Ser Ser
        115                 120                 125


<210> 12
<211> 8
<212> PRT
<213> Artificial Sequence

<220>
<223> Description of Artificial Sequence: Synthetic
      peptide

<400> 12
Gly Phe Thr Phe Ser Ser His Trp
1               5

<210> 13
<211> 8
<212> PRT
<213> Artificial Sequence

<220>
<223> Description of Artificial Sequence: Synthetic
      peptide

<400> 13
Ile Asn Gln Asp Gly Ser Glu Lys
1               5

<210> 14
<211> 20
<212> PRT
<213> Artificial Sequence

<220>
<223> Description of Artificial Sequence: Synthetic
      peptide

<400> 14
Ala Arg Asp Ile Val Leu Met Val Tyr Asp Met Asp Tyr Tyr Tyr Tyr
1               5                   10                  15

Gly Met Asp Val
            20

<210> 15
<211> 112
<212> PRT
<213> Artificial Sequence

<220>
<223> Description of Artificial Sequence: Synthetic
      polypeptide

<400> 15
Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Asn Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Thr
                85                  90                  95

Leu Gln Thr Pro Leu Thr Phe Gly Gly Gly Thr Lys Val Glu Ile Lys
            100                 105                 110

<210> 16
<211> 11
<212> PRT
<213> Artificial Sequence

<220>
<223> Description of Artificial Sequence: Synthetic
      peptide

<400> 16
Gln Ser Leu Leu His Ser Asn Gly Asn Asn Tyr
1               5                   10

<210> 17
<211> 3
<212> PRT
<213> Artificial Sequence

<220>
<223> Description of Artificial Sequence: Synthetic
      peptide

<400> 17
Leu Gly Ser
1

<210> 18
<211> 9
<212> PRT
<213> Artificial Sequence

<220>
<223> Description of Artificial Sequence: Synthetic
      peptide

<400> 18
Met Gln Thr Leu Gln Thr Pro Leu Thr
1               5

<210> 19
<211> 8
<212> PRT
<213> Artificial Sequence

<220>
<223> Description of Artificial Sequence: Synthetic
      peptide

<400> 19
Gly Phe Thr Phe Ser Ser His Trp
1               5

<210> 20
<211> 8
<212> PRT
<213> Artificial Sequence

<220>
<223> Description of Artificial Sequence: Synthetic
      peptide

<400> 20
Ile Asn Gln Asp Gly Ser Glu Lys
1               5

<210> 21
<211> 20
<212> PRT
<213> Artificial Sequence

<220>
<223> Description of Artificial Sequence: Synthetic
      peptide

<400> 21
Ala Arg Asp Ile Val Leu Met Val Tyr Asp Met Asp Tyr Tyr Tyr Tyr
1               5                   10                  15

Gly Met Asp Val
            20

<210> 22
<211> 11
<212> PRT
<213> Artificial Sequence

<220>
<223> Description of Artificial Sequence: Synthetic
      peptide

<400> 22
Gln Ser Leu His His Ser Asn Gly Asn Asn Tyr
1               5                   10

<210> 23
<211> 3
<212> PRT
<213> Artificial Sequence

<220>
<223> Description of Artificial Sequence: Synthetic
      peptide

<400> 23
Leu Gly Ser
1

<210> 24
<211> 9
<212> PRT
<213> Artificial Sequence

<220>
<223> Description of Artificial Sequence: Synthetic
      peptide

<400> 24
Met Gln Thr Leu Gln Thr Pro Leu Thr
1               5


<210> 25
<211> 8
<212> PRT
<213> Artificial Sequence

<220>
<223> Description of Artificial Sequence: Synthetic
      peptide

<400> 25
Gly Phe Thr Phe Ser Ser His Trp
1               5


<210> 26
<211> 8
<212> PRT
<213> Artificial Sequence

<220>
<223> Description of Artificial Sequence: Synthetic
      peptide

<400> 26
Ile Asn Gln Asp Gly Ser Glu Lys
1               5


<210> 27
<211> 20
<212> PRT
<213> Artificial Sequence

<220>
<223> Description of Artificial Sequence: Synthetic
      peptide

<400> 27
Ala Arg Asp Ile Val Leu Met Val Tyr His Met Asp Tyr Tyr Tyr Tyr
1               5                   10                  15


Gly Met Asp Val
            20


<210> 28

<211> 11
<212> PRT
<213> Artificial Sequence

<220>
<223> Description of Artificial Sequence: Synthetic
      peptide

<400> 28
Gln Ser Leu Leu His Ser Asn Gly Asn Asn Tyr
1               5                   10


<210> 29
<211> 3
<212> PRT
<213> Artificial Sequence

<220>
<223> Description of Artificial Sequence: Synthetic
      peptide

<400> 29
Leu Gly Ser
1


<210> 30
<211> 9
<212> PRT
<213> Artificial Sequence

<220>
<223> Description of Artificial Sequence: Synthetic
      peptide

<400> 30
Met Gln Thr Leu Gln Thr Pro Leu Thr
1               5


<210> 31
<211> 8
<212> PRT
<213> Artificial Sequence

<220>
<223> Description of Artificial Sequence: Synthetic
      peptide

<400> 31
Gly Phe Thr Phe Ser Ser His Trp
1               5


<210> 32
<211> 8
<212> PRT
<213> Artificial Sequence

<220>
<223> Description of Artificial Sequence: Synthetic
      peptide

<400> 32
Ile Asn Gln Asp Gly Ser Glu Lys
1               5


<210> 33
<211> 20
<212> PRT
<213> Artificial Sequence

<220>
<223> Description of Artificial Sequence: Synthetic
      peptide

<400> 33
Ala Arg Asp Ile Val Leu Met Val Tyr His Met Asp Tyr Tyr Tyr Tyr
1               5                   10                  15


Gly Met Asp Val
            20


<210> 34
<211> 11
<212> PRT
<213> Artificial Sequence

<220>
<223> Description of Artificial Sequence: Synthetic
      peptide

<400> 34
Gln Ser Leu His His Ser Asn Gly Asn Asn Tyr
1               5                   10


<210> 35
<211> 3
<212> PRT
<213> Artificial Sequence

<220>
<223> Description of Artificial Sequence: Synthetic
      peptide

<400> 35
Leu Gly Ser
1


<210> 36
<211> 9
<212> PRT
<213> Artificial Sequence

<220>
<223> Description of Artificial Sequence: Synthetic
      peptide

<400> 36
Met Gln Thr Leu Gln Thr Pro Leu Thr

1               5


<210> 37
<211> 131
<212> PRT
<213> Artificial Sequence

<220>
<223> Description of Artificial Sequence: Synthetic
      VH; m2CX1D05 polypeptide

<400> 37
Gln Val Gln Leu Val Gln Ser Gly Ala Glu Val Lys Lys Pro Gly Ser
1               5                   10                  15


Ser Val Lys Val Ser Cys Lys Ala Ser Gly Gly Thr Phe Asn Ser His
            20                  25                  30


Ala Ile Ser Trp Val Arg Gln Ala Pro Gly Gln Gly Leu Glu Trp Met
        35                  40                  45


Gly Gly Ile Asn Pro Ile Leu Gly Ile Ala Asn Tyr Ala Gln Lys Phe
    50                  55                  60


Gln Gly Arg Val Thr Ile Thr Ala Asp Glu Ser Thr Ser Thr Ala Tyr
65                  70                  75                  80


Met Glu Leu Ser Ser Leu Arg Ser Glu Asp Thr Ala Val Tyr Tyr Cys
                85                  90                  95


Ala Arg His Tyr Glu Ile Gln Ile Gly Arg Tyr Gly Met Asn Val Tyr
            100                 105                 110


Tyr Leu Met Tyr Arg Phe Ala Ser Trp Gly Gln Gly Thr Leu Val Thr
        115                 120                 125


Val Ser Ser
    130


<210> 38
<211> 10
<212> PRT
<213> Artificial Sequence

<220>
<223> Description of Artificial Sequence: Synthetic
      VH CDR1; m2CX1D05 peptide

<400> 38
Gly Gly Thr Phe Asn Ser His Ala Ile Ser
1               5                   10

<210> 39
<211> 20
<212> PRT
<213> Artificial Sequence

<220>
<223> Description of Artificial Sequence: Synthetic
      VH CDR2; m2CX1D05 peptide

<400> 39
Trp Met Gly Gly Ile Asn Pro Ile Leu Gly Ile Ala Asn Tyr Ala Gln
1               5                   10                  15

Lys Phe Gln Gly
            20

<210> 40
<211> 22
<212> PRT
<213> Artificial Sequence

<220>
<223> Description of Artificial Sequence: Synthetic
      VH CDR3; m2CX1D05 peptide

<400> 40
His Tyr Glu Ile Gln Ile Gly Arg Tyr Gly Met Asn Val Tyr Tyr Leu
1               5                   10                  15

Met Tyr Arg Phe Ala Ser
            20

<210> 41
<211> 213
<212> PRT
<213> Artificial Sequence

<220>
<223> Description of Artificial Sequence: Synthetic
      LC; m2CX1D05 polypeptide

<400> 41
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Arg Ser Ala
            20                  25                  30

Leu Asn Trp Tyr Gln Gln Lys Pro Gly Lys Ala Pro Lys Leu Leu Ile
        35                  40                  45

Tyr Asn Gly Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro

65                  70                  75                  80

Glu Asp Phe Ala Val Tyr Tyr Cys Gln Gln Phe Asp Gly Asp Pro Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Ala
    210

<210> 42
<211> 11
<212> PRT
<213> Artificial Sequence

<220>
<223> Description of Artificial Sequence: Synthetic
      VL CDR 1; m2CX1D05 peptide

<400> 42
Arg Ala Ser Gln Gly Ile Arg Ser Ala Leu Asn
1               5                   10

<210> 43
<211> 11
<212> PRT
<213> Artificial Sequence

<220>
<223> Description of Artificial Sequence: Synthetic
      VL CDR2; m2CX1D05 peptide

<400> 43

Leu Leu Ile Tyr Asn Gly Ser Thr Leu Gln Ser
1               5                   10

<210> 44
<211> 7
<212> PRT
<213> Artificial Sequence

<220>
<223> Description of Artificial Sequence: Synthetic
      VL CDR3; m2CX1D05 peptide

<400> 44
Gln Gln Phe Asp Gly Asp Pro
1               5

<210> 45
<211> 119
<212> PRT
<213> Artificial Sequence

<220>
<223> Description of Artificial Sequence: Synthetic
      VH; 1B20 polypeptide

<400> 45
Gln Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Asn Tyr
            20                  25                  30

Trp Ile Ser Trp Val Arg Gln Met Pro Gly Lys Gly Leu Glu Trp Met
        35                  40                  45

Gly Ile Ile Tyr Pro Gly Asp Ser Tyr Thr Asn Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Asp Tyr Trp Tyr Lys Pro Leu Phe Asp Ile Trp Gly Gln Gly
            100                 105                 110

Thr Leu Val Thr Val Ser Ser
        115

<210> 46
<211> 10
<212> PRT

<213> Artificial Sequence

<220>
<223> Description of Artificial Sequence: Synthetic
      VH CDR1; 1B20 peptide

<400> 46
Gly Tyr Ser Phe Thr Asn Tyr Trp Ile Ser
1               5                   10


<210> 47
<211> 20
<212> PRT
<213> Artificial Sequence

<220>
<223> Description of Artificial Sequence: Synthetic
      VH CDR2; 1B20 peptide

<400> 47
Trp Met Gly Ile Ile Tyr Pro Gly Asp Ser Tyr Thr Asn Tyr Ser Pro
1               5                   10                  15


Ser Phe Gln Gly
            20


<210> 48
<211> 10
<212> PRT
<213> Artificial Sequence

<220>
<223> Description of Artificial Sequence: Synthetic
      VH CDR3; 1B20 peptide

<400> 48
Asp Tyr Trp Tyr Lys Pro Leu Phe Asp Ile
1               5                   10


<210> 49
<211> 220
<212> PRT
<213> Artificial Sequence

<220>
<223> Description of Artificial Sequence: Synthetic
      LC; 1B20 polypeptide

<400> 49
Asp Ile Val Met Thr Gln Ser Pro Asp Ser Leu Ala Val Ser Leu Gly
1               5                   10                  15


Glu Arg Ala Thr Ile Asn Cys Arg Ser Ser Gln Ser Val Leu Tyr Ser
            20                  25                  30


Ser Asn Asn Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Ser Ser Phe Pro Ile Thr Phe Gly Gln Gly Thr Lys Val Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Ala
    210                 215                 220

<210> 50
<211> 17
<212> PRT
<213> Artificial Sequence

<220>
<223> Description of Artificial Sequence: Synthetic
      VL CDR1; 1B20 peptide

<400> 50
Arg Ser Ser Gln Ser Val Leu Tyr Ser Ser Asn Asn Lys Asn Tyr Leu
1               5                   10                  15

Ala

<210> 51
<211> 11
<212> PRT
<213> Artificial Sequence

<220>
<223> Description of Artificial Sequence: Synthetic
      VL CDR2; 1B20 peptide

<400> 51
Leu Leu Ile Tyr Trp Ala Ser Thr Arg Glu Ser
1               5                   10


<210> 52
<211> 8
<212> PRT
<213> Artificial Sequence

<220>
<223> Description of Artificial Sequence: Synthetic
      VL CDR3; 1B20 peptide

<400> 52
Gln Gln Tyr Ser Ser Phe Pro Ile
1               5


<210> 53
<211> 120
<212> PRT
<213> Artificial Sequence

<220>
<223> Description of Artificial Sequence: Synthetic
      variable heavy antibody region polypeptide

<400> 53
Glu Val Gln Leu Leu Glu Ser Gly Gly Gly Leu Val Gln Pro Gly Gly
1               5                   10                  15

Ser Leu Arg Leu Ser Cys Lys Ala Ser Gly Tyr Thr Phe Ser Ser Tyr
            20                  25                  30

Gly Met Tyr Trp Val Arg Gln Ala Pro Gly Lys Gly Leu Glu Trp Ile
        35                  40                  45

Gly Trp Ile Asp Pro Gly Ser Gly Gly Thr Lys Tyr Asn Glu Lys Phe
    50                  55                  60

Lys Gly Lys Ala Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Arg Tyr Gly Tyr Tyr Phe Asp Tyr Trp Gly Gln Gly Thr
            100                 105                 110

Leu Val Thr Val Ser Ser Ala Ser
        115                 120

<210> 54
<211> 16
<212> PRT
<213> Artificial Sequence

<220>
<223> Description of Artificial Sequence: Synthetic
      AX132 heavy chain CDR1 antibody region peptide

<400> 54
Lys Ala Ser Gly Tyr Thr Phe Ser Ser Tyr Gly Met Tyr Trp Val Arg
1               5                   10                  15

<210> 55
<211> 23
<212> PRT
<213> Artificial Sequence

<220>
<223> Description of Artificial Sequence: Synthetic
      AX132 heavy chain CDR2 antibody region peptide

<400> 55
Trp Ile Gly Trp Ile Asp Pro Gly Ser Gly Gly Thr Lys Tyr Asn Glu
1               5                   10                  15

Lys Phe Lys Gly Lys Ala Thr
            20

<210> 56
<211> 15
<212> PRT
<213> Artificial Sequence

<220>
<223> Description of Artificial Sequence: Synthetic
      AX132 heavy chain CDR3 antibody region peptide

<400> 56
Cys Ala Arg Glu Arg Tyr Gly Tyr Tyr Phe Asp Tyr Trp Gly Gln
1               5                   10                  15

<210> 57
<211> 108
<212> PRT
<213> Artificial Sequence

<220>
<223> Description of Artificial Sequence: Synthetic
      variable light antibody region polypeptide

<400> 57
Glu Ile Val Leu Thr Gln Ser Pro Ala Thr Leu Ser Leu Ser Pro Gly
1               5                   10                  15

Glu Arg Ala Thr Ile Thr Cys Arg Ala Ser Gln Tyr Val Gly Ser Tyr
            20                  25                  30

Leu Asn Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Val Trp Asp Ser Ser Pro Pro
                85                  90                  95

Val Val Phe Gly Gly Gly Thr Lys Val Glu Ile Lys
            100                 105

<210> 58
<211> 17
<212> PRT
<213> Artificial Sequence

<220>
<223> Description of Artificial Sequence: Synthetic
      AX213 and AX132 light chain CDR1 antibody region peptide

<400> 58
Ile Thr Cys Arg Ala Ser Gln Tyr Val Gly Ser Tyr Leu Asn Trp Tyr
1               5                   10                  15

Gln

<210> 59
<211> 13
<212> PRT
<213> Artificial Sequence

<220>
<223> Description of Artificial Sequence: Synthetic
      AX213 and AX132 light chain CDR2 antibody region peptide

<400> 59
Leu Ile Tyr Asp Ala Ser Asn Arg Ala Thr Gly Ile Pro
1               5                   10

<210> 60
<211> 16

<212> PRT
<213> Artificial Sequence

<220>
<223> Description of Artificial Sequence: Synthetic
      AX132 & AX213 light chain CDR3 antibody region peptide

<400> 60
Tyr Tyr Cys Gln Val Trp Asp Ser Ser Pro Pro Val Val Phe Gly Gly
1               5                   10                  15


<210> 61
<211> 120
<212> PRT
<213> Artificial Sequence

<220>
<223> Description of Artificial Sequence: Synthetic
      variable heavy antibody region polypeptide

<400> 61
Glu Val Gln Leu Leu Glu Ser Gly Gly Gly Leu Val Gln Pro Gly Gly
1               5                   10                  15


Ser Leu Arg Leu Ser Cys Lys Ala Ser Gly Tyr Thr Phe Ser Arg Tyr
            20                  25                  30


Gly Ile Asn Trp Val Arg Gln Ala Pro Gly Lys Gly Leu Glu Trp Ile
        35                  40                  45


Gly Arg Ile Asp Pro Gly Asn Gly Gly Thr Arg Tyr Asn Glu Lys Phe
    50                  55                  60


Lys Gly Lys Ala Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Ala Asn Asp Gly Tyr Ser Phe Asp Tyr Trp Gly Gln Gly Thr
            100                 105                 110


Leu Val Thr Val Ser Ser Ala Ser
        115                 120


<210> 62
<211> 16
<212> PRT
<213> Artificial Sequence

<220>
<223> Description of Artificial Sequence: Synthetic
      AX213 heavy chain CDR1 antibody region peptide

<400> 62
Lys Ala Ser Gly Tyr Thr Phe Ser Arg Tyr Gly Ile Asn Trp Val Arg
1               5                   10                  15

<210> 63
<211> 23
<212> PRT
<213> Artificial Sequence

<220>
<223> Description of Artificial Sequence: Synthetic
      AX213 heavy chain CDR2 antibody region peptide

<400> 63
Trp Ile Gly Arg Ile Asp Pro Gly Asn Gly Gly Thr Arg Tyr Asn Glu
1               5                   10                  15

Lys Phe Lys Gly Lys Ala Thr
            20

<210> 64
<211> 15
<212> PRT
<213> Artificial Sequence

<220>
<223> Description of Artificial Sequence: Synthetic
      AX213 heavy chain CDR3 antibody region peptide

<400> 64
Cys Ala Arg Ala Asn Asp Gly Tyr Ser Phe Asp Tyr Trp Gly Gln
1               5                   10                  15

<210> 65
<211> 108
<212> PRT
<213> Artificial Sequence

<220>
<223> Description of Artificial Sequence: Synthetic
      variable light antibody region polypeptide

<400> 65
Glu Ile Val Leu Thr Gln Ser Pro Ala Thr Leu Ser Leu Ser Pro Gly
1               5                   10                  15

Glu Arg Ala Thr Ile Thr Cys Arg Ala Ser Gln Tyr Val Gly Ser Tyr
            20                  25                  30

Leu Asn Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Val Trp Asp Ser Ser Pro Pro
                85                  90                  95

Val Val Phe Gly Gly Gly Thr Lys Val Glu Ile Lys
            100                 105

<210> 66
<211> 17
<212> PRT
<213> Artificial Sequence

<220>
<223> Description of Artificial Sequence: Synthetic
      AX213 and AX132 light chain CDR1 antibody region peptide

<400> 66
Ile Thr Cys Arg Ala Ser Gln Tyr Val Gly Ser Tyr Leu Asn Trp Tyr
1               5                   10                  15

Gln

<210> 67
<211> 13
<212> PRT
<213> Artificial Sequence

<220>
<223> Description of Artificial Sequence: Synthetic
      AX213 and AX132 light chain CDR2 antibody region peptide

<400> 67
Leu Ile Tyr Asp Ala Ser Asn Arg Ala Thr Gly Ile Pro
1               5                   10

<210> 68
<211> 16
<212> PRT
<213> Artificial Sequence

<220>
<223> Description of Artificial Sequence: Synthetic
      AX132 & AX213 light chain CDR3 antibody region peptide

<400> 68
Tyr Tyr Cys Gln Val Trp Asp Ser Ser Pro Pro Val Val Phe Gly Gly
1               5                   10                  15

<210> 69
<211> 119
<212> PRT
<213> Artificial Sequence

<220>
<223> Description of Artificial Sequence: Synthetic
      AX1 VH antibody sequence polypeptide

<400> 69
Glu Val Gln Leu Leu Glu Ser Gly Gly Gly Leu Val Gln Pro Gly Gly
1               5                   10                  15

Ser Leu Arg Leu Ser Cys Lys Ala Ser Gly Phe Thr Phe Thr Ser Tyr
            20                  25                  30

Tyr Met His Trp Val Arg Gln Ala Pro Gly Lys Gly Leu Glu Trp Ile
        35                  40                  45

Gly Arg Ile Asn Pro Asp Ser Gly Ser Thr Lys Tyr Asn Glu Lys Phe
    50                  55                  60

Lys Gly Arg Ala Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Gly Arg Leu Ser Trp Asp Phe Asp Val Trp Gly Gln Gly
            100                 105                 110

Thr Leu Val Thr Val Ser Ser
        115


<210> 70
<211> 16
<212> PRT
<213> Artificial Sequence

<220>
<223> Description of Artificial Sequence: Synthetic
      AX1 VH CDR1 antibody sequence peptide

<400> 70
Lys Ala Ser Gly Phe Thr Phe Thr Ser Tyr Tyr Met His Trp Val Arg
1               5                   10                  15


<210> 71
<211> 23
<212> PRT
<213> Artificial Sequence

<220>
<223> Description of Artificial Sequence: Synthetic
      AX1 VH CDR2 antibody sequence peptide

<400> 71
Trp Ile Gly Arg Ile Asn Pro Asp Ser Gly Ser Thr Lys Tyr Asn Glu
1               5                   10                  15

Lys Phe Lys Gly Arg Ala Thr
            20

<210> 72
<211> 16
<212> PRT
<213> Artificial Sequence

<220>
<223> Description of Artificial Sequence: Synthetic
      AX1 VH CDR3 antibody sequence peptide

<400> 72
Cys Ala Arg Gly Gly Arg Leu Ser Trp Asp Phe Asp Val Trp Gly Gln
1               5                   10                  15

<210> 73
<211> 109
<212> PRT
<213> Artificial Sequence

<220>
<223> Description of Artificial Sequence: Synthetic
      AX1 VL antibody sequence polypeptide

<400> 73
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Arg Tyr
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Ala Ala Tyr Asp Tyr Ser Leu Gly
                85                  90                  95

Gly Tyr Val Phe Gly Asp Gly Thr Lys Val Glu Ile Lys
            100                 105

<210> 74
<211> 11
<212> PRT
<213> Artificial Sequence

<220>
<223> Description of Artificial Sequence: Synthetic
      AX1 VL CDR1 antibody sequence peptide

<400> 74
Arg Ala Ser Gln Asp Ile Ser Arg Tyr Leu Ala
1               5                   10


<210> 75
<211> 7
<212> PRT
<213> Artificial Sequence

<220>
<223> Description of Artificial Sequence: Synthetic
      AX1 AX9 AX189 VL CDR2 antibody sequence peptide

<400> 75
Ala Ala Ser Ser Leu Gln Ser
1               5


<210> 76
<211> 11
<212> PRT
<213> Artificial Sequence

<220>
<223> Description of Artificial Sequence: Synthetic
      AX1 VL CDR3 antibody sequence peptide

<400> 76
Ala Ala Tyr Asp Tyr Ser Leu Gly Gly Tyr Val
1               5                   10


<210> 77
<211> 121
<212> PRT
<213> Artificial Sequence

<220>
<223> Description of Artificial Sequence: Synthetic
      AX9 AX189 VH antibody sequence polypeptide

<400> 77
Glu Val Gln Leu Leu Glu Ser Gly Gly Gly Leu Val Gln Pro Gly Gly
1               5                   10                  15


Ser Leu Arg Leu Ser Cys Lys Ala Ser Gly Tyr Thr Phe Ser Ser Tyr
            20                  25                  30


Trp Met His Trp Val Arg Gln Ala Pro Gly Lys Gly Leu Glu Trp Ile
        35                  40                  45


Gly Arg Ile Asp Pro Tyr Asn Gly Gly Thr Lys Tyr Asn Glu Lys Phe
    50                  55                  60

Lys Gly Lys Ala Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Gly Tyr Tyr Leu Gly Ser Tyr Ala Met Asp Tyr Trp Gly
            100                 105                 110

Gln Gly Thr Leu Val Thr Val Ser Ser
        115                 120

<210> 78
<211> 16
<212> PRT
<213> Artificial Sequence

<220>
<223> Description of Artificial Sequence: Synthetic
      AX9 AX189 VH CDR1 antibody sequence peptide

<400> 78
Lys Ala Ser Gly Tyr Thr Phe Ser Ser Tyr Trp Met His Trp Val Arg
1               5                   10                  15

<210> 79
<211> 23
<212> PRT
<213> Artificial Sequence

<220>
<223> Description of Artificial Sequence: Synthetic
      AX9 AX189 VH CDR2 antibody sequence peptide

<400> 79
Trp Ile Gly Arg Ile Asp Pro Tyr Asn Gly Gly Thr Lys Tyr Asn Glu
1               5                   10                  15

Lys Phe Lys Gly Lys Ala Thr
            20

<210> 80
<211> 18
<212> PRT
<213> Artificial Sequence

<220>
<223> Description of Artificial Sequence: Synthetic
      AX9 AX189 VH CDR3 antibody sequence peptide

<400> 80
Cys Ala Arg Tyr Gly Tyr Tyr Leu Gly Ser Tyr Ala Met Asp Tyr Trp
1               5                   10                  15

Gly Gln

<210> 81
<211> 109
<212> PRT
<213> Artificial Sequence

<220>
<223> Description of Artificial Sequence: Synthetic
      AX189 VL antibody sequence polypeptide

<400> 81
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Arg Tyr
            20                  25                  30

Leu Thr Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ala Tyr Asp Tyr Ser Leu Ser
                85                  90                  95

Gly Tyr Val Phe Gly Gly Gly Thr Lys Val Glu Ile Lys
            100                 105


<210> 82
<211> 11
<212> PRT
<213> Artificial Sequence

<220>
<223> Description of Artificial Sequence: Synthetic
      AX189 VL CDR1 antibody sequence peptide

<400> 82
Arg Ala Ser Gln Asp Val Ser Arg Tyr Leu Thr
1               5                   10


<210> 83
<211> 7
<212> PRT
<213> Artificial Sequence

<220>
<223> Description of Artificial Sequence: Synthetic
      AX1 AX9 AX189 VL CDR2 antibody sequence peptide

<400> 83
Ala Ala Ser Ser Leu Gln Ser
1               5

<210> 84
<211> 11
<212> PRT
<213> Artificial Sequence

<220>
<223> Description of Artificial Sequence: Synthetic
      AX189 VL CDR3 antibody sequence peptide

<400> 84
Gln Ala Tyr Asp Tyr Ser Leu Ser Gly Tyr Val
1               5                   10

<210> 85
<211> 115
<212> PRT
<213> Homo sapiens

<400> 85
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Leu Thr Ser Tyr
            20                  25                  30

Gly Ile Ser Trp Val Arg Gln Ala Pro Gly Gln Gly Leu Glu Trp Met
        35                  40                  45

Gly Trp Val Ser Phe Tyr Asn Gly Asn Thr Asn Tyr Ala Gln Lys Leu
    50                  55                  60

Gln Gly Arg Gly Thr Met Thr Thr Asp Pro Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Gly Tyr Gly Met Asp Val Trp Gly Gln Gly Thr Thr Val Thr
            100                 105                 110

Val Ser Ser
        115

<210> 86
<211> 5
<212> PRT
<213> Homo sapiens

<400> 86
Ser Tyr Gly Ile Ser
1               5

<210> 87
<211> 17
<212> PRT
<213> Homo sapiens

<400> 87
Trp Val Ser Phe Tyr Asn Gly Asn Thr Asn Tyr Ala Gln Lys Leu Gln
1               5                   10                  15

Gly

<210> 88
<211> 6
<212> PRT
<213> Homo sapiens

<400> 88
Gly Tyr Gly Met Asp Val
1               5

<210> 89
<211> 109
<212> PRT
<213> Homo sapiens

<400> 89
Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Ser Val Ser Trp Tyr Gln Gln His Pro Gly Lys Ala Pro Lys Leu
        35                  40                  45

Met Ile Tyr Glu Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Asn Ser Tyr Thr Ser Thr
                85                  90                  95

Ser Met Val Phe Gly Gly Gly Thr Lys Leu Thr Val Leu
            100                 105

<210> 90
<211> 14
<212> PRT
<213> Homo sapiens

<400> 90
Thr Gly Thr Ser Ser Asp Val Gly Gly Tyr Asn Ser Val Ser
1               5                   10


<210> 91
<211> 7
<212> PRT
<213> Homo sapiens

<400> 91
Glu Val Ser Asn Arg Pro Ser
1               5


<210> 92
<211> 9
<212> PRT
<213> Homo sapiens

<400> 92
Asn Ser Tyr Thr Ser Thr Ser Met Val
1               5


<210> 93
<211> 123
<212> PRT
<213> Homo sapiens

<400> 93
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ser Ser Ser Ser Tyr Ile Ser Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Phe Cys
                85                  90                  95

Ala Arg Asp Tyr Asp Phe Trp Ser Ala Tyr Tyr Asp Ala Phe Asp Val
            100                 105                 110

Trp Gly Gln Gly Thr Met Val Thr Val Ser Ser
        115                 120

<210> 94
<211> 10
<212> PRT
<213> Homo sapiens

<400> 94
Gly Phe Thr Phe Ser Ser Tyr Ser Met Asn
1               5                   10

<210> 95
<211> 17
<212> PRT
<213> Homo sapiens

<400> 95
Ser Ile Ser Ser Ser Ser Ser Tyr Ile Ser Tyr Ala Asp Ser Val Lys
1               5                   10                  15

Gly

<210> 96
<211> 14
<212> PRT
<213> Homo sapiens

<400> 96
Asp Tyr Asp Phe Trp Ser Ala Tyr Tyr Asp Ala Phe Asp Val
1               5                   10

<210> 97
<211> 111
<212> PRT
<213> Homo sapiens

<400> 97
Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Asp Val His Trp Tyr Gln Gln Leu Pro Gly Thr Ala Pro Lys Leu
        35                  40                  45

Leu Ile Ser Gly Asn Ser Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Ser Ser
                85                  90                  95

Leu Ser Gly Ser Val Phe Gly Gly Gly Thr Lys Leu Thr Val Leu
            100                 105                 110

<210> 98
<211> 14
<212> PRT
<213> Homo sapiens

<400> 98
Thr Gly Ser Ser Ser Asn Ile Gly Ala Gly Tyr Asp Val His
1               5                   10

<210> 99
<211> 7
<212> PRT
<213> Homo sapiens

<400> 99
Gly Asn Ser Asn Arg Pro Ser
1               5

<210> 100
<211> 11
<212> PRT
<213> Homo sapiens

<400> 100
Gln Ser Tyr Asp Ser Ser Leu Ser Gly Ser Val
1               5                   10

<210> 101
<211> 114
<212> PRT
<213> Homo sapiens

<400> 101
Gln Val Gln Leu Val Glu Ser Gly Gly Gly Val Ala Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Tyr Tyr Asp Gly Ile Asn Lys His Tyr Ala Asp Ser Val
    50                  55                  60

Lys Gly Arg Phe Thr Ile Ser Arg Asp Asn Ser Lys Asn Thr Leu Tyr

65                  70                  75                  80

Leu Gln Met Asn Ser Leu Arg Ala Glu Asp Thr Ala Val Tyr Tyr Cys
                85                  90                  95

Ala Arg Asp Arg Gly Leu Asp Trp Gly Gln Gly Thr Leu Val Thr Val
            100                 105                 110

Ser Ser

<210> 102
<211> 10
<212> PRT
<213> Homo sapiens

<400> 102
Gly Phe Thr Phe Ser Ser Tyr Gly Met His
1               5                   10

<210> 103
<211> 17
<212> PRT
<213> Homo sapiens

<400> 103
Val Ile Tyr Tyr Asp Gly Ile Asn Lys His Tyr Ala Asp Ser Val Lys
1               5                   10                  15

Gly

<210> 104
<211> 5
<212> PRT
<213> Homo sapiens

<400> 104
Asp Arg Gly Leu Asp
1               5

<210> 105
<211> 113
<212> PRT
<213> Homo sapiens

<400> 105
Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Leu Tyr Ser
            20                  25                  30

Ser Asn Ser Lys Asn Tyr Leu Val Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Thr Pro Trp Thr Phe Gly Gln Gly Thr Lys Val Glu Ile
            100                 105                 110

Lys

<210> 106
<211> 17
<212> PRT
<213> Homo sapiens

<400> 106
Lys Ser Ser Gln Ser Val Leu Tyr Ser Ser Asn Ser Lys Asn Tyr Leu
1               5                   10                  15

Val

<210> 107
<211> 7
<212> PRT
<213> Homo sapiens

<400> 107
Trp Ala Ser Thr Arg Glu Ser
1               5

<210> 108
<211> 9
<212> PRT
<213> Homo sapiens

<400> 108
Gln Gln Tyr Tyr Ser Thr Pro Trp Thr
1               5

<210> 109
<211> 118
<212> PRT
<213> Artificial Sequence

<220>
<223> Description of Artificial Sequence: Synthetic
      anti-PCSK9 monoclonal antibody pJG04 (clones LGT-209 and
      LGT-210) Vh heavy chain variable region (FR1-FR4)
      polypeptide

<400> 109
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Thr Met
            20                  25                  30

Tyr Met Ser Trp Val Arg Gln Ala Pro Gly Gln Gly Leu Glu Trp Met
        35                  40                  45

Gly Arg Ile Asp Pro Ala Asn Glu His Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Thr Ser Asp Asp Thr Ala Val Tyr Tyr Cys
                85                  90                  95

Ala Arg Ser Tyr Tyr Tyr Tyr Asn Met Asp Tyr Trp Gly Gln Gly Thr
            100                 105                 110

Leu Val Thr Val Ser Ser
        115


<210> 110
<211> 5
<212> PRT
<213> Artificial Sequence

<220>
<223> Description of Artificial Sequence: Synthetic
      anti-PCSK9 monoclonal antibody clones LGT-209,
      LGT-210 and LGT-211 heavy chain CDR1 peptide

<400> 110
Thr Met Tyr Met Ser
1               5


<210> 111
<211> 17
<212> PRT
<213> Artificial Sequence

<220>
<223> Description of Artificial Sequence: Synthetic
      anti-PCSK9 monoclonal antibody clones LGT-209,
      LGT-210 and LGT-211 heavy chain CDR2 peptide

<400> 111
Arg Ile Asp Pro Ala Asn Glu His Thr Asn Tyr Ala Gln Lys Phe Gln
1               5                   10                  15

Gly

<210> 112
<211> 9
<212> PRT
<213> Artificial Sequence

<220>
<223> Description of Artificial Sequence: Synthetic
      anti-PCSK9 monoclonal antibody pJG04(clones
      LGT-209 and LGT-210) Vh heavy chain complementarity
      determining region 3 (CDR3) peptide

<400> 112
Ser Tyr Tyr Tyr Tyr Asn Met Asp Tyr
1               5

<210> 113
<211> 106
<212> PRT
<213> Artificial Sequence

<220>
<223> Description of Artificial Sequence: Synthetic
      anti-PCSK9 monoclonal antibody pJG10(clones
      LGT-209 and LGT-211) Vk light chain variable
      region (FR1-FR4) polypeptide

<400> 113
Gln Ile Val Leu Thr Gln Ser Pro Ala Thr Leu Ser Val Ser Pro Gly
1               5                   10                  15

Glu Arg Ala Thr Leu Ser Cys Arg Ala Ser Gln Ser Val Ser Tyr Met
            20                  25                  30

His Trp Tyr Gln Gln Lys Pro Gly Gln Ala Pro Arg Leu Leu Ile Tyr
        35                  40                  45

Gly Val Phe Arg Arg Ala Thr Gly Ile Pro Asp Arg Phe Ser Gly Ser
    50                  55                  60

Gly Ser Gly Thr Asp Phe Thr Leu Thr Ile Gly Arg Leu Glu Pro Glu
65                  70                  75                  80

Asp Phe Ala Val Tyr Tyr Cys Leu Gln Trp Ser Ser Asp Pro Pro Thr
                85                  90                  95

Phe Gly Gln Gly Thr Lys Leu Glu Ile Lys

            100                 105

<210> 114
<211> 10
<212> PRT
<213> Artificial Sequence

<220>
<223> Description of Artificial Sequence: Synthetic
      anti-PCSK9 monoclonal antibody clones LGT-209,
      LGT-210 and LGT-211 light chain CDR1 peptide

<400> 114
Arg Ala Ser Gln Ser Val Ser Tyr Met His
1               5                   10

<210> 115
<211> 7
<212> PRT
<213> Artificial Sequence

<220>
<223> Description of Artificial Sequence: Synthetic
      anti-PCSK9 monoclonal antibody clones LGT-209,
      LGT-210 and LGT-211 light chain CDR1 peptide

<400> 115
Gly Val Phe Arg Arg Ala Thr
1               5

<210> 116
<211> 9
<212> PRT
<213> Artificial Sequence

<220>
<223> Description of Artificial Sequence: Synthetic
      mouse anti-PCSK9 monoclonal antibody LFU720 and
      anti-PCSK9 monoclonal antibody clones LGT-209,
      LGT-210 and LGT-211 light chain CDR3 peptide

<400> 116
Leu Gln Trp Ser Ser Asp Pro Pro Thr
1               5

<210> 117
<211> 118
<212> PRT
<213> Homo sapiens

<400> 117
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Glu Ile Ser Pro Phe Gly Gly Arg Thr Asn Tyr Asn Glu Lys Phe
    50                  55                  60

Lys Ser Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Glu Arg Pro Leu Tyr Ala Ser Asp Leu Trp Gly Gln Gly Thr
            100                 105                 110

Thr Val Thr Val Ser Ser
        115

<210> 118
<211> 7
<212> PRT
<213> Artificial Sequence

<220>
<223> Description of Artificial Sequence: Synthetic
      peptide

<400> 118
Gly Tyr Thr Phe Thr Ser Tyr
1               5

<210> 119
<211> 6
<212> PRT
<213> Artificial Sequence

<220>
<223> Description of Artificial Sequence: Synthetic
      peptide

<400> 119
Ser Pro Phe Gly Gly Arg
1               5

<210> 120
<211> 9
<212> PRT
<213> Artificial Sequence

<220>
<223> Description of Artificial Sequence: Synthetic
      variable heavy chain CDR peptide

<400> 120
Glu Arg Pro Leu Tyr Ala Ser Asp Leu

1               5


<210> 121
<211> 107
<212> PRT
<213> Homo sapiens

<400> 121
Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Gly Ile Ser Ser Ala
            20                  25                  30


Leu Ala Trp Tyr Gln Gln Lys Pro Gly Lys Ala Pro Lys Leu Leu Ile
        35                  40                  45


Tyr Ser Ala Ser Tyr Arg Tyr Thr Gly Val Pro Ser Arg Phe Ser Gly
    50                  55                  60


Ser Gly Ser Gly Thr Asp Phe Thr Phe Thr Ile Ser Ser Leu Gln Pro
65                  70                  75                  80


Glu Asp Ile Ala Thr Tyr Tyr Cys Gln Gln Arg Tyr Ser Leu Trp Arg
                85                  90                  95


Thr Phe Gly Gln Gly Thr Lys Leu Glu Ile Lys
            100                 105


<210> 122
<211> 11
<212> PRT
<213> Artificial Sequence

<220>
<223> Description of Artificial Sequence: Synthetic
      variable light chain CDR peptide

<400> 122
Arg Ala Ser Gln Gly Ile Ser Ser Ala Leu Ala
1               5                   10


<210> 123
<211> 7
<212> PRT
<213> Artificial Sequence

<220>
<223> Description of Artificial Sequence: Synthetic
      variable light chain CDR peptide

<400> 123
Ser Ala Ser Tyr Arg Tyr Thr
1               5

<210> 124
<211> 9
<212> PRT
<213> Artificial Sequence

<220>
<223> Description of Artificial Sequence: Synthetic
      variable light chain CDR peptide

<400> 124
Gln Gln Arg Tyr Ser Leu Trp Arg Thr
1               5


<210> 125
<211> 118
<212> PRT
<213> Homo sapiens

<400> 125
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Glu Ile His Pro Ser Gly Gly Arg Thr Asn Tyr Asn Glu Lys Phe
    50                  55                  60

Lys Ser Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Glu Arg Pro Leu Tyr Ala Met Asp Tyr Trp Gly Gln Gly Thr
            100                 105                 110

Thr Val Thr Val Ser Ser
        115


<210> 126
<211> 10
<212> PRT
<213> Homo sapiens

<400> 126
Gly Tyr Thr Phe Thr Ser Tyr Tyr Met His
1               5                   10

<210> 127
<211> 17
<212> PRT
<213> Homo sapiens

<400> 127
Glu Ile His Pro Ser Gly Gly Arg Thr Asn Tyr Asn Glu Lys Phe Lys
1               5                   10                  15

Ser

<210> 128
<211> 9
<212> PRT
<213> Homo sapiens

<400> 128
Glu Arg Pro Leu Tyr Ala Met Asp Tyr
1               5

<210> 129
<211> 107
<212> PRT
<213> Homo sapiens

<400> 129

<210> 130
<211> 11
<212> PRT
<213> Homo sapiens

<400> 130
Lys Ala Ser Gln Asp Val His Thr Ala Val Ala
1               5                   10

<210> 131
<211> 7
<212> PRT
<213> Homo sapiens

<400> 131
His Ala Ser Tyr Arg Tyr Thr
1               5

<210> 132
<211> 9
<212> PRT
<213> Homo sapiens

<400> 132
Gln Gln Arg Tyr Ser Leu Trp Arg Thr
1               5

<210> 133
<211> 118
<212> PRT
<213> Homo sapiens

<400> 133

<210> 134
<211> 10
<212> PRT
<213> Homo sapiens

<400> 134
Gly Tyr Thr Phe Thr Ser Tyr Tyr Met His
1               5                   10


<210> 135
<211> 17
<212> PRT
<213> Homo sapiens

<400> 135
Glu Ile His Pro Ser Gly Gly Arg Thr Asn Tyr Asn Glu Lys Phe Lys
1               5                   10                  15

Ser


<210> 136
<211> 9
<212> PRT
<213> Homo sapiens

<400> 136
Glu Arg Pro Leu Tyr Ala Ser Asp Leu
1               5


<210> 137
<211> 107
<212> PRT
<213> Homo sapiens

<400> 137

<210> 138
<211> 11
<212> PRT
<213> Homo sapiens

<400> 138
Lys Ala Ser Gln Asp Val His Thr Ala Val Ala
1               5                   10

<210> 139
<211> 7
<212> PRT
<213> Homo sapiens

<400> 139
His Ala Ser Tyr Arg Tyr Thr
1               5

<210> 140
<211> 9
<212> PRT
<213> Homo sapiens

<400> 140
Gln Gln Arg Tyr Ser Leu Trp Arg Thr
1               5

<210> 141
<211> 118
<212> PRT
<213> Mus musculus

<400> 141
Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Glu Ile Asn Pro Ser Asn Gly Arg Thr Asn Tyr Asn Glu Lys Phe
    50                  55                  60

Lys Ser Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Tyr Cys

85                                      90                                      95

Ala Arg Glu Arg Pro Leu Tyr Ala Met Asp Tyr Trp Gly Gln Gly Thr
                100                                     105                                     110

Ser Val Thr Val Ser Ser
        115

<210> 142
<211> 7
<212> PRT
<213> Mus musculus

<400> 142
Gly Tyr Thr Phe Thr Ser Tyr
1               5

<210> 143
<211> 6
<212> PRT
<213> Mus musculus

<400> 143
Asn Pro Ser Asn Gly Arg
1               5

<210> 144
<211> 9
<212> PRT
<213> Mus musculus

<400> 144
Glu Arg Pro Leu Tyr Ala Met Asp Tyr
1               5

<210> 145
<211> 108
<212> PRT
<213> Mus musculus

<400> 145
Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Ser Thr Ala
                20                  25                  30

Val Ala Trp Tyr Gln Gln Lys Pro Gly Gln Ser Pro Lys Leu Leu Ile
        35                  40                  45

Tyr Ser Ala Ser Tyr Arg Tyr Thr Gly Val Pro Asp Arg Phe Thr Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln Arg Tyr Ser Thr Pro Arg
                85                  90                  95

Thr Phe Gly Gly Gly Thr Lys Leu Glu Ile Lys Arg
            100                 105

<210> 146
<211> 11
<212> PRT
<213> Mus musculus

<400> 146
Lys Ala Ser Gln Asp Val Ser Thr Ala Val Ala
1               5                   10

<210> 147
<211> 7
<212> PRT
<213> Artificial Sequence

<220>
<223> Description of Artificial Sequence: Synthetic
      variable light chain CDR peptide

<400> 147
Ser Ala Ser Tyr Arg Tyr Thr
1               5

<210> 148
<211> 9
<212> PRT
<213> Mus musculus

<400> 148
Gln Gln Arg Tyr Ser Thr Pro Arg Thr
1               5

<210> 149
<211> 115
<212> PRT
<213> Mus musculus

<400> 149
Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Thr Phe Thr Asp Tyr
            20                  25                  30

Tyr Met Asn Trp Val Lys Gln Ser His Gly Lys Ser Leu Glu Trp Ile
        35                  40                  45

Gly Asp Ile Asn Pro Asn Asn Gly Gly Thr Thr Tyr Asn Gln Lys Phe
    50                  55                  60

Lys Gly Lys Ala Thr Leu Thr Val Asp Lys Ser Tyr Ser Thr Ala Tyr
65                  70                  75                  80

Met Glu Leu Arg Ser Leu Thr Ser Glu Asp Ser Ala Val Tyr Tyr Cys
                85                  90                  95

Ala Arg Trp Leu Leu Phe Ala Tyr Trp Gly Gln Gly Thr Leu Val Thr
            100                 105                 110

Val Ser Ala
        115

<210> 150
<211> 7
<212> PRT
<213> Mus musculus

<400> 150
Gly Tyr Thr Phe Thr Asp Tyr
1               5

<210> 151
<211> 6
<212> PRT
<213> Mus musculus

<400> 151
Asn Pro Asn Asn Gly Gly
1               5

<210> 152
<211> 6
<212> PRT
<213> Mus musculus

<400> 152
Trp Leu Leu Phe Ala Tyr
1               5

<210> 153
<211> 108
<212> PRT
<213> Mus musculus

<400> 153
Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Thr Asn
            20                  25                  30

Val Ala Trp Tyr Gln Gln Lys Pro Gly Gln Ser Pro Lys Ala Leu Ile
        35                  40                  45

Tyr Ser Ala Ser Tyr Arg Tyr Ser Gly Val Pro Asp Arg Phe Thr Gly
    50                  55                  60

Ser Gly Ser Gly Thr Asp Phe Thr Leu Thr Ile Ser Asn Val Leu Ser
65                  70                  75                  80

Glu Asp Leu Ala Glu Tyr Phe Cys Gln Gln Phe Tyr Ser Tyr Pro Tyr
                85                  90                  95

Thr Phe Gly Gly Gly Thr Lys Leu Glu Ile Lys Arg
            100                 105

<210> 154
<211> 11
<212> PRT
<213> Mus musculus

<400> 154
Lys Ala Ser Gln Asn Val Gly Thr Asn Val Ala
1               5                   10

<210> 155
<211> 7
<212> PRT
<213> Mus musculus

<400> 155
Ser Ala Ser Tyr Arg Tyr Ser
1               5

<210> 156
<211> 9
<212> PRT
<213> Mus musculus

<400> 156
Gln Gln Phe Tyr Ser Tyr Pro Tyr Thr
1               5

<210> 157
<211> 123
<212> PRT
<213> Mus musculus

<400> 157
Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Thr Phe Thr Asp Tyr

20                  25                  30

Tyr Met Asn Trp Val Lys Gln Ser His Gly Lys Ser Leu Glu Trp Ile
        35                  40                  45

Gly Asp Ile Asn Pro Asn Asn Gly Gly Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Ala Gly Gly Gly Ile Tyr Tyr Arg Tyr Asp Arg Asn Tyr Phe Asp Tyr
            100                 105                 110

Trp Gly Gln Gly Thr Thr Leu Thr Val Ser Ser
        115                 120

<210> 158
<211> 7
<212> PRT
<213> Mus musculus

<400> 158
Gly Tyr Thr Phe Thr Asp Tyr
1               5

<210> 159
<211> 6
<212> PRT
<213> Mus musculus

<400> 159
Asn Pro Asn Asn Gly Gly
1               5

<210> 160
<211> 14
<212> PRT
<213> Mus musculus

<400> 160
Gly Gly Ile Tyr Tyr Arg Tyr Asp Arg Asn Tyr Phe Asp Tyr
1               5                   10

<210> 161
<211> 107
<212> PRT
<213> Mus musculus

<400> 161

Asp Ile Gln Met Thr Gln Thr Thr Ser Ser Leu Ser Ala Ser Leu Gly
1               5                   10                  15

Asp Arg Val Thr Ile Ser Cys Ser Ala Ser Gln Gly Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Lys Leu Pro Phe
                85                  90                  95

Thr Phe Gly Ser Gly Thr Lys Leu Glu Ile Lys
            100                 105

<210> 162
<211> 11
<212> PRT
<213> Mus musculus

<400> 162
Ser Ala Ser Gln Gly Ile Ser Asn Tyr Leu Asn
1               5                   10

<210> 163
<211> 7
<212> PRT
<213> Mus musculus

<400> 163
Tyr Thr Ser Ser Leu His Ser
1               5

<210> 164
<211> 9
<212> PRT
<213> Artificial Sequence

<220>
<223> Description of Artificial Sequence: Synthetic
      peptide

<400> 164
Gln Gln Tyr Ser Lys Leu Pro Phe Thr
1               5

<210> 165

<211> 117
<212> PRT
<213> Mus musculus

<400> 165
Glu Val Lys Leu Val Glu Ser Glu Gly Gly Leu Val Gln Pro Gly Ser
1               5                   10                  15

Ser Met Lys Leu Ser Cys Thr Ala Ser Gly Phe Thr Phe Ser Asp Tyr
            20                  25                  30

Tyr Met Ala Trp Val Arg Gln Val Pro Glu Lys Gly Leu Glu Trp Val
        35                  40                  45

Ala Asn Ile Asn Tyr Asp Gly Ser Asn Thr Ser Tyr Leu Asp Ser Leu
    50                  55                  60

Lys Ser Arg Phe Ile Ile Ser Arg Asp Asn Ala Lys Asn Ile Leu Tyr
65                  70                  75                  80

Leu Gln Met Ser Ser Leu Lys Ser Glu Asp Thr Ala Thr Tyr Tyr Cys
                85                  90                  95

Ala Arg Glu Lys Phe Ala Ala Met Asp Tyr Trp Gly Gln Gly Thr Ser
            100                 105                 110

Val Thr Val Ser Ser
        115


<210> 166
<211> 7
<212> PRT
<213> Mus musculus

<400> 166
Gly Phe Thr Phe Ser Asp Tyr
1               5


<210> 167
<211> 6
<212> PRT
<213> Mus musculus

<400> 167
Asn Tyr Asp Gly Ser Asn
1               5


<210> 168
<211> 8
<212> PRT
<213> Mus musculus

<400> 168

Glu Lys Phe Ala Ala Met Asp Tyr
1               5

<210> 169
<211> 108
<212> PRT
<213> Mus musculus

<400> 169
Asp Ile Val Met Thr Gln Ser His Lys Phe Met Ser Thr Ser Phe Gly
1               5                   10                  15

Asp Arg Val Ser Ile Thr Cys Lys Ala Ser Gln Asp Val Ser Asn Ala
            20                  25                  30

Leu Ala Trp Tyr Gln Gln Lys Pro Gly His Ser Pro Lys Leu Leu Ile
        35                  40                  45

Phe Ser Ala Ser Tyr Arg Tyr Thr Gly Val Pro Asp Arg Phe Thr Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln His Tyr Ser Thr Pro Trp
                85                  90                  95

Thr Phe Gly Gly Gly Thr Lys Leu Glu Ile Lys Arg
            100                 105

<210> 170
<211> 11
<212> PRT
<213> Mus musculus

<400> 170
Lys Ala Ser Gln Asp Val Ser Asn Ala Leu Ala
1               5                   10

<210> 171
<211> 7
<212> PRT
<213> Artificial Sequence

<220>
<223> Description of Artificial Sequence: Synthetic
      variable light chain CDR peptide

<400> 171
Ser Ala Ser Tyr Arg Tyr Thr
1               5

<210> 172

<211> 9
<212> PRT
<213> Mus musculus

<400> 172
Gln Gln His Tyr Ser Thr Pro Trp Thr
1               5


<210> 173
<211> 121
<212> PRT
<213> Artificial Sequence

<220>
<223> Description of Artificial Sequence: Synthetic
      polypeptide

<400> 173
Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Thr Arg His
            20                  25                  30


Thr Ile His Trp Val Arg Gln Ala Pro Gly Lys Gly Leu Glu Trp Val
        35                  40                  45


Ala Arg Ile Ser Pro Ala Asn Gly Asn Thr Asn Tyr Ala Asp Ser Val
    50                  55                  60


Lys Gly Arg Phe Thr Ile Ser Ala Asp Thr Ser Lys Asn Thr Ala Tyr
65                  70                  75                  80


Leu Gln Met Asn Ser Leu Arg Ala Glu Asp Thr Ala Val Tyr Tyr Cys
                85                  90                  95


Ala Arg Trp Ile Gly Ser Arg Glu Leu Tyr Ile Met Asp Tyr Trp Gly
            100                 105                 110


Gln Gly Thr Leu Val Thr Val Ser Ser
        115                 120


<210> 174
<211> 10
<212> PRT
<213> Artificial Sequence

<220>
<223> Description of Artificial Sequence: Synthetic
      peptide

<400> 174
Gly Phe Thr Phe Thr Arg His Thr Ile His
1               5                   10

<210> 175
<211> 17
<212> PRT
<213> Artificial Sequence

<220>
<223> Description of Artificial Sequence: Synthetic
      peptide

<400> 175
Arg Ile Ser Pro Ala Asn Gly Asn Thr Asn Tyr Ala Asp Ser Val Lys
1               5                   10                  15

Gly

<210> 176
<211> 12
<212> PRT
<213> Artificial Sequence

<220>
<223> Description of Artificial Sequence: Synthetic
      peptide

<400> 176
Trp Ile Gly Ser Arg Glu Leu Tyr Ile Met Asp Tyr
1               5                   10

<210> 177
<211> 108
<212> PRT
<213> Artificial Sequence

<220>
<223> Description of Artificial Sequence: Synthetic
      polypeptide

<400> 177
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Arg Ile Gln Pro
                85                  90                  95

Thr Phe Gly Gln Gly Thr Lys Val Glu Ile Lys Arg
            100                 105

<210> 178
<211> 11
<212> PRT
<213> Artificial Sequence

<220>
<223> Description of Artificial Sequence: Synthetic
      peptide

<400> 178
Arg Ala Ser Gln Asp Val Ser Thr Ala Val Ala
1               5                   10

<210> 179
<211> 7
<212> PRT
<213> Artificial Sequence

<220>
<223> Description of Artificial Sequence: Synthetic
      peptide

<400> 179
Ser Ala Ser Phe Leu Tyr Ser
1               5

<210> 180
<211> 9
<212> PRT
<213> Artificial Sequence

<220>
<223> Description of Artificial Sequence: Synthetic
      peptide

<400> 180
Gln Gln Ser Tyr Arg Ile Gln Pro Thr
1               5

<210> 181
<211> 121
<212> PRT
<213> Artificial Sequence

<220>
<223> Description of Artificial Sequence: Synthetic
      polypeptide

<400> 181
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Thr
            20                  25                  30

Ala Ile His Trp Val Arg Gln Ala Pro Gly Lys Gly Leu Glu Trp Val
        35                  40                  45

Ala Arg Ile Ser Pro Ala Asn Gly Asn Thr Asn Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Trp Ile Gly Ser Arg Glu Leu Tyr Ile Met Asp Tyr Trp Gly
            100                 105                 110

Gln Gly Thr Leu Val Thr Val Ser Ser
        115                 120

<210> 182
<211> 10
<212> PRT
<213> Artificial Sequence

<220>
<223> Description of Artificial Sequence: Synthetic
      peptide

<400> 182
Gly Phe Thr Phe Ser Ser Thr Ala Ile His
1               5                   10

<210> 183
<211> 17
<212> PRT
<213> Artificial Sequence

<220>
<223> Description of Artificial Sequence: Synthetic
      peptide

<400> 183
Arg Ile Ser Pro Ala Asn Gly Asn Thr Asn Tyr Ala Asp Ser Val Lys
1               5                   10                  15

Gly

<210> 184

<211> 12
<212> PRT
<213> Artificial Sequence

<220>
<223> Description of Artificial Sequence: Synthetic
      peptide

<400> 184
Trp Ile Gly Ser Arg Glu Leu Tyr Ile Met Asp Tyr
1               5                   10


<210> 185
<211> 108
<212> PRT
<213> Artificial Sequence

<220>
<223> Description of Artificial Sequence: Synthetic
      polypeptide

<400> 185
Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Asp Val Ser Thr Ala
            20                  25                  30


Val Ala Trp Tyr Gln Gln Lys Pro Gly Lys Ala Pro Lys Leu Leu Ile
        35                  40                  45


Tyr Ser Ala Ser Phe Leu Tyr Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Ser Tyr Pro Ala Leu His
                85                  90                  95


Thr Phe Gly Gln Gly Thr Lys Val Glu Ile Lys Arg
            100                 105


<210> 186
<211> 11
<212> PRT
<213> Artificial Sequence

<220>
<223> Description of Artificial Sequence: Synthetic
      peptide

<400> 186
Arg Ala Ser Gln Asp Val Ser Thr Ala Val Ala
1               5                   10

<210> 187
<211> 7
<212> PRT
<213> Artificial Sequence

<220>
<223> Description of Artificial Sequence: Synthetic
      peptide

<400> 187
Ser Ala Ser Phe Leu Tyr Ser
1               5


<210> 188
<211> 9
<212> PRT
<213> Artificial Sequence

<220>
<223> Description of Artificial Sequence: Synthetic
      peptide

<400> 188
Gln Gln Ser Tyr Pro Ala Leu His Thr
1               5


<210> 189
<211> 125
<212> PRT
<213> Artificial Sequence

<220>
<223> Description of Artificial Sequence: Synthetic
      polypeptide

<400> 189
Glu Val Gln Leu Val Glu Ser Gly Gly Gly Leu Val Lys Pro Gly Gly
1               5                   10                  15


Ser Leu Arg Leu Ser Cys Ala Ala Ser Gly Phe Pro Phe Ser Lys Leu
            20                  25                  30


Gly Met Val Trp Val Arg Gln Ala Pro Gly Lys Gly Leu Glu Trp Val
        35                  40                  45


Ser Thr Ile Ser Ser Gly Gly Gly Tyr Thr Tyr Tyr Pro Asp Ser Val
    50                  55                  60


Lys Gly Arg Phe Thr Ile Ser Arg Asp Asn Ala Lys Asn Ser Leu Tyr
65                  70                  75                  80


Leu Gln Met Asn Ser Leu Arg Ala Glu Asp Thr Ala Val Tyr Tyr Cys
                85                  90                  95

Ala Arg Glu Gly Ile Ser Phe Gln Gly Gly Thr Tyr Thr Tyr Val Met
            100                 105                 110

Asp Tyr Trp Gly Gln Gly Thr Leu Val Thr Val Ser Ser
        115                 120                 125

<210> 190
<211> 10
<212> PRT
<213> Artificial Sequence

<220>
<223> Description of Artificial Sequence: Synthetic
      peptide

<400> 190
Gly Phe Pro Phe Ser Lys Leu Gly Met Val
1               5                   10

<210> 191
<211> 17
<212> PRT
<213> Artificial Sequence

<220>
<223> Description of Artificial Sequence: Synthetic
      peptide

<400> 191
Thr Ile Ser Ser Gly Gly Gly Tyr Thr Tyr Tyr Pro Asp Ser Val Lys
1               5                   10                  15

Gly

<210> 192
<211> 16
<212> PRT
<213> Artificial Sequence

<220>
<223> Description of Artificial Sequence: Synthetic
      peptide

<400> 192
Glu Gly Ile Ser Phe Gln Gly Gly Thr Tyr Thr Tyr Val Met Asp Tyr
1               5                   10                  15

<210> 193
<211> 112
<212> PRT
<213> Artificial Sequence

<220>
<223> Description of Artificial Sequence: Synthetic
      polypeptide

<400> 193
Asp Ile Val Met Thr Gln Ser Pro Leu Ser Leu Pro Val Thr Pro Gly
1               5                   10                  15

Glu Pro Ala Ser Ile Ser Cys Arg Ser Ser Lys Ser Leu Leu His Arg
            20                  25                  30

Asn Gly Ile Thr Tyr Ser Tyr Trp Tyr Leu Gln Lys Pro Gly Gln Ser
        35                  40                  45

Pro Gln Leu Leu Ile Tyr Gln Leu Ser Asn Leu Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Tyr Gln Asn
                85                  90                  95

Leu Glu Leu Pro Leu Thr Phe Gly Gln Gly Thr Lys Val Glu Ile Lys
            100                 105                 110

<210> 194
<211> 16
<212> PRT
<213> Artificial Sequence

<220>
<223> Description of Artificial Sequence: Synthetic
      peptide

<400> 194
Arg Ser Ser Lys Ser Leu Leu His Arg Asn Gly Ile Thr Tyr Ser Tyr
1               5                   10                  15

<210> 195
<211> 7
<212> PRT
<213> Artificial Sequence

<220>
<223> Description of Artificial Sequence: Synthetic
      peptide

<400> 195
Gln Leu Ser Asn Leu Ala Ser
1               5

<210> 196
<211> 9
<212> PRT
<213> Artificial Sequence

<220>
<223> Description of Artificial Sequence: Synthetic
      peptide

<400> 196
Tyr Gln Asn Leu Glu Leu Pro Leu Thr
1               5


<210> 197
<211> 2076
<212> DNA
<213> Homo sapiens

<400> 197
atgggcaccg tcagctccag gcggtcctgg tggccgctgc cactgctgct gctgctgctg      60

ctgctcctgg gtcccgcggg cgcccgtgcg caggaggacg aggacggcga ctacgaggag     120

ctggtgctag ccttgcgttc cgaggaggac ggcctggccg aagcacccga gcacggaacc     180

acagccacct tccaccgctg cgccaaggat ccgtggaggt tgcctggcac ctacgtggtg     240

gtgctgaagg aggagaccca cctctcgcag tcagagcgca ctgcccgccg cctgcaggcc     300

caggctgccc gccggggata cctcaccaag atcctgcatg tcttccatgg ccttcttcct     360

ggcttcctgg tgaagatgag tggcgacctg ctggagctgg ccttgaagtt gccccatgtc     420

gactacatcg aggaggactc ctctgtcttt gcccagagca tcccgtggaa cctggagcgg     480

attacccctc cacggtaccg ggcggatgaa taccagcccc ccgacggagg cagcctggtg     540

gaggtgtatc tcctagacac cagcatacag agtgaccacc gggaaatcga gggcagggtc     600

atggtcaccg acttcgagaa tgtgcccgag gaggacggga cccgcttcca cagacaggcc     660

agcaagtgtg acagtcatgg cacccacctg gcaggggtgg tcagcggccg ggatgccggc     720

gtggccaagg gtgccagcat gcgcagcctg cgcgtgctca actgccaagg gaagggcacg     780

gttagcggca ccctcatagg cctggagttt attcggaaaa gccagctggt ccagcctgtg     840

gggccactgg tggtgctgct gcccctggcg ggtgggtaca gccgcgtcct caacgccgcc     900

tgccagcgcc tggcgagggc tggggtcgtg ctggtcaccg ctgccggcaa cttccgggac     960

gatgcctgcc tctactcccc agcctcagct cccgaggtca tcacagttgg ggccaccaat    1020

gcccaagacc agccggtgac cctggggact ttggggacca actttggccg ctgtgtggac    1080

ctctttgccc caggggagga catcattggt gcctccagcg actgcagcac ctgctttgtg    1140

tcacagagtg ggacatcaca ggctgctgcc cacgtggctg gcattgcagc catgatgctg    1200

tctgccgagc cggagctcac cctggccgag ttgaggcaga gactgatcca cttctctgcc    1260

aaagatgtca tcaatgaggc ctggttccct gaggaccagc gggtactgac ccccaacctg    1320

gtggccgccc tgccccccag cacccatggg gcaggttggc agctgttttg caggactgta    1380

tggtcagcac actcggggcc tacacggatg gccacagccg tcgcccgctg cgccccagat    1440

gaggagctgc tgagctgctc cagtttctcc aggagtggga agcggcgggg cgagcgcatg 1500

gaggcccaag ggggcaagct ggtctgccgg gcccacaacg cttttggggg tgagggtgtc 1560

tacgccattg ccaggtgctg cctgctaccc caggccaact gcagcgtcca cacagctcca 1620

ccagctgagg ccagcatggg gacccgtgtc cactgccacc aacagggcca cgtcctcaca 1680

ggctgcagct cccactggga ggtggaggac cttggcaccc acaagccgcc tgtgctgagg 1740

ccacgaggtc agcccaacca gtgcgtgggc cacagggagg ccagcatcca cgcttcctgc 1800

tgccatgccc caggtctgga atgcaaagtc aaggagcatg gaatcccggc ccctcaggag 1860

caggtgaccg tggcctgcga ggagggctgg accctgactg gctgcagtgc cctccctggg 1920

acctcccacg tcctgggggc ctacgccgta gacaacacgt gtgtagtcag gagccgggac 1980

gtcagcacta caggcagcac cagcgaaggg gccgtgacag ccgttgccat ctgctgccgg 2040

agccggcacc tggcgcaggc ctcccaggag ctccag 2076

<210> 198
<211> 692
<212> PRT
<213> Homo sapiens

<400> 198